# EUROPEAN PATENT SPECIFICATION

(11) **EP 3 317 411 B1**
(45) Date of publication and mention of the grant of the patent: **30.12.2020**
(21) Application number: 16742172.6
(22) Date of filing: 30.06.2016
(51) Int. Cl.: C12N 15/11, A61P 27/00, A61P 9/00, A61P 3/10, A61P 1/16, A61P 11/00, A61P 25/00, A61K 38/16

(54) **ATP-BINDING CASSETTE FAMILY CODING POLYRIBONUCLEOTIDES AND FORMULATIONS THEREOF**
FÜR ATP-BINDUNGSKASSETTENFAMILIE CODIERENDE POLYRIBONUKLEOTIDE UND FORMULIERUNGEN DAVON
POLYRIBONUCLÉOTIDES CODANT POUR UNE FAMILLE DE CASSETTES DE LIAISON À L'ATP ET FORMULATIONS ASSOCIÉES

(30) Priority: 30.06.2015 EP 15174677
(43) Date of publication of application: 09.05.2018
(73) Proprietor: ethris GmbH, 82152 Planegg (DE)
(72) Inventor: PLANK, Christian, 82234 Wessling (DE); RUDOLPH, Carsten, 82152 Krailing (DE); ANEJA, Manish Kumar, 81373 Munich (DE); WEISS, Ludwig, 86438 Kissing (DE); FERIZI, Mehrije, 81539 Munich (DE); GEIGER, Johannes, 81249 Munich (DE)
(74) Representative: Vossius & Partner Patentanwälte Rechtsanwälte mbB
(86) International application number: PCT/EP2016/065321
(87) International publication number: WO 2017/001570

(56) References cited:
- EP-A1- 3 112 469
- WO-A1-2009/055760
- WO-A1-2013/182683
- WO-A1-2015/024667
- MIGNONE F ET AL: "Untranslated regions of mRNAs", GENOME BIOLOGY, BIOMED CENTRAL LTD., LONDON, GB, vol. 3, no. 3, 1 January 2002 (2002-01-01) , pages 4.1-4.10, XP002428984, ISSN: 1465-6906, DOI: 10.1186/GB-2002-3-3-REVIEWS0004
- MICHAEL S D KORMANN ET AL: "Expression of therapeutic proteins after delivery of chemically modified mRNA in mice", NATURE BIOTECHNOLOGY, vol. 29, no. 2, 1 February 2011 (2011-02-01), pages 154-157, XP55040839, ISSN: 1087-0156, DOI: 10.1038/nbt.1733
- YOSHIDA ICHIRO ET AL: "Expression of ABCA3, a causative gene for fatal surfactant deficiency, is up-regulated by glucocorticoids in lung alveolar type II cells", BIOCHEMICAL AND BIOPHYSICAL RESEARCH COMMUNICATIONS, vol. 323, no. 2, 2004, pages 547-555, XP028808629, ISSN: 0006-291X, DOI: 10.1016/J.BBRC.2004.08.133
- J. WINTER ET AL: "Neonatal Respiratory Insufficiency Caused by an (Homozygous) ABCA3-Stop Mutation: a Systematic Evaluation of Therapeutic Options", KLINISCHE PAEDIATRIE., vol. 226, no. 02, 31 March 2014 (2014-03-31), pages 53-58, XP055313269, DE ISSN: 0300-8630, DOI: 10.1055/s-0033-1363687

## Description

### CROSS-REFERENCE

This application claims the benefit of EP15174677.3 filed on June 30, 2015 under 35 U.S.C. § 119.

### BACKGROUND

Messenger RNAs (mRNA) are polymers containing a number of linked nucleotides, each composed of a sugar, a phosphate, and a base. Each mRNA polymer stores genetic information along the nucleotide chain. Messenger RNA polymers carry the genetic information from the DNA in the nucleus of the cell to the cytoplasm where proteins are made. Each triplet of nucleotides in the mRNA is called a codon, and each codon specifies the identity of an amino acid in the translated protein.

A cell can also take up and translate an exogenous RNA, but many factors influence efficient uptake and translation. For instance, the immune system recognizes many exogenous RNAs as foreign and triggers a response that is aimed at inactivating the RNAs. In addition, many exogenous RNAs are not sufficiently stable to be adequately expressed within a host cell.

Attempts for overcoming these shortcomings have been described, such as in the patent application WO 2013/182683 A1.

### SUMMARY

The present disclosure provides a composition comprising a modified polyribonucleotide for treating a subject having or suspected of having a disease as herein defined associated with a gene of the ATP-binding cassette (ABC) family, such as ABCA3 that may be associated with a respiratory distress syndrome. The modified polyribonucleotide encodes an ABC-binding cassette (ABC) family member and comprises an untranslated region derived from a cytochrome b-245 alpha polypeptide gene, wherein upon translation the modified polyribonucleotide yields an ATP-binding cassette polypeptide that ameliorates the symptom of the disease. The modified polyribonucleotide can include a codon sequence that is optimized for translation within cells of the subject exposed to the modified polyribonucleotide, wherein upon translation the modified polyribonucleotide yields a polypeptide that ameliorates a symptom of the disease. The gene in the ATP-binding cassette family can be selected from the group consisting of ABCA1, ABCA3, ABCA4, ABCA12, ABCB4, ABCB7, ABCB11, ABCC2, ABCC6, ABCC8, ABCC9, ABCD1, ABCG5, ABCG8, and CFTR. In some cases, the gene in the ATP-binding cassette family is ABCA3 or at least 70% homologous to the human ABCA3. In some cases, the composition comprises a ratio of moles of amine groups of cationic polymers to moles of phosphate groups of the modified polyribonucleotide of at least about 8. In some cases, the composition is selected from TABLE 8. In some cases, the optimized codon sequence is translated at least 20% more effectively within a cell of a subject than a non-optimized codon sequence. According to the invention, the modified polyribonucleotide comprises a combination of unmodified and modified nucleotides, or comprises analogues of uridine or analogues of cytidine. A composition of the disclosure can be used to treat a disease. According to the invention, the disease is selected from the group consisting of age-related macular degeneration, benign recurrent intrahepatic cholestasis, Cantu syndrome, congenital bilateral absence of the vas deferens, congenital hyperinsulinism, cystic fibrosis, Dubin-Johnson syndrome, familial dilated cardiomyopathy, familial HDL deficiency, generalized arterial calcification of infancy, harlequin ichthyosis, hereditary pancreatitis, intrahepatic cholestasis of pregnancy, lamellar ichthyosis, permanent neonatal diabetes mellitus, progressive familial intrahepatic cholestasis, pseudoxanthoma elasticum, retinitis pigmentosa, sitosterolemia, Stargardt macular degeneration, surfactant dysfunction, Tangier disease, X-linked adrenoleukodystrophy, X-linked sideroblastic anemia and ataxia. In some cases, the modified polyribonucleotide provides expression of the polypeptide for a time period in the cell of the subject having the modified polyribonucleotide, wherein the time period is up to 4 weeks, wherein the expression is enhanced as compared to expression in a control cell that has been exposed to an unmodified polyribonucleotide encoding the polypeptide. The time period can be of at least about 30 seconds and up to 5 days. In some cases, the modified polyribonucleotide comprises a 3' or 5' noncoding region flanking the codon sequence which encodes the polypeptide, wherein the noncoding region aids in enhanced expression of the polypeptide in the cells. In some cases, the modified polyribonucleotide is formulated in a nanoparticle, nanocapsule, cationic lipid, cationic polymer, nanoemulsion. In some cases the modified polyribonucleotide comprises analogues of uridine or analogues of cytidine. In some cases, the modified polyribonucleotide comprises 5% to 50% analogues of uridine or 5% to 50% analogues of cytidine. In some cases, the modified polyribonucleotide comprises 15% to 30% analogues of uridine or 15% to 30% analogues of cytidine. In some cases the analogues of uridine are selected from the group consisting of pseudouridine, 2-thiouridine, 5-iodouridine, and 5-methyluridine. In some cases the analogues of cytidine are selected from the group consisting of 5-methylcytidine, 2'-amino-2'-deoxycytidine, 2'-fluoro-2'-deoxycytidine, and 5-iodocytidine. In some cases, the modified polyribonucleotide comprises 5-methylcytidine or pseudouridine. In some cases, the modified polyribonucleotide comprises (i) uridine and cytidine; and (ii) analogues of the uridine and cytidine. In some cases the modified polyribonucleotide comprises analogues of adenosine or analogues of guanosine. In some cases, the modified polyribonucleotide comprises (i) adenosine or guanosine; and (ii) analogues of the adenosine or guanosine. In some cases, the modified polyribonucleotide comprises less than 50% analogues of adenosine or guanosine. In some cases, the modified polyribonucleotide comprises (i) adenosine and guanosine; and (ii) analogues of the adenosine and guanosine. In some cases, the modified polyribonucleotide has a transfection efficiency greater than 80% among cells exposed to the modified polyribonucleotide. In some cases, the modified polyribonucleotide induces substantially no change in a level of at least one inflammatory marker expressed by peripheral blood mononuclear cells exposed to the modified polyribonucleotide. In some cases (i) the codon sequence is a gene or fragment whose defect or deficiency is associated with a presence of the disease, or (ii) a lack or deficiency of the polypeptide is associated with the presence of the disease. For example, a deficiency in the ABCA3 gene can be a cause of respiratory distress syndrome. In some cases, the modified polyribonucleotide lowers an immune response of the subject as compared to an unmodified polyribonucleotide encoding the polypeptide, which immune response is as determined by a level of at least one inflammatory marker selected from TNF-α, IL-2 and IL-8 expressed by peripheral blood mononuclear cells exposed to the modified polyribonucleotide as compared to a level of the at least one inflammatory marker in peripheral blood mononuclear cells in a control that has been exposed to the unmodified polyribonucleotide.

### BRIEF DESCRIPTION OF THE DRAWINGS

The novel features of the invention are set forth in the appended claims. A better understanding of the features and advantages of the present invention will be obtained by reference to the following detailed description that sets forth illustrative embodiments, in which the principles of the invention are utilized, and the accompanying drawings of which:
**FIGURE 1** illustrates an example of aerosol administration of engineered polyribonucleotides of the disclosure to mice.
**FIGURE 2** is a western blot illustrating the translation of ABCA3 mRNAs with different UTRs in A549 cells 6 hours post-transfection.
**FIGURE 3** is a western blot illustrating ABCA3 protein expression from mRNAs comprising various nucleotide modifications in HEK-293 cells.
**FIGURE 4** is a western blot illustrating ABCA3 protein expression from mRNAs comprising various nucleotide modifications in A549 cells.
**FIGURE 5** is a western blot illustrating ABCA3 protein expression from mRNAs comprising various nucleotide modifications in MLE-15 cells.
**FIGURE 6** illustrates a comparison of the ABCA3 protein expression levels measured by Western Blot 6 hours post mRNA transfection in HEK-293 cells, A549 cells, and MLE-15 cells.
**FIGURE 7** illustrates the induction of IL-6 in A549 cells translating the ABCA3 protein from ABCA3-FLAG mRNAs comprising various nucleotide modifications.
**FIGURE 8** illustrates induction of IP-10 in HepG2 cells translating the ABCA3 protein from ABCA3-FLAG mRNAs comprising various nucleotide modifications.
**FIGURE 9** illustrates the cell viability of A549 cells after transfection with mRNAs comprising various chemical modifications.
**FIGURE 10** illustrates the cell viability of HEP G2 cells after transfection with mRNAs comprising various chemical modifications.
**FIGURE 11** illustrate the results of an ELISA assay measuring the levels of IL-6 from A549 cells after transfected with mRNAs comprising various chemical modifications.
**FIGURE 12** illustrate the results of an ELISA assay measuring the levels of IP-10 from A549 cells after transfected with mRNAs comprising various chemical modifications.
**FIGURE 13** is a western blot illustrating the time-course of translation of the ABCA3 polypeptide expressed from SEQ ID NO: 17 in lung cells in vitro.

### DETAILED DESCRIPTION

The term "subject," as used herein generally refers to a human. In some instances, a subject can also be an animal, such as a mouse, a rat, a guinea pig, a dog, a cat, a horse, a rabbit, and various other animals. A subject can be of any age, for example, a subject can be an infant, a toddler, a child, a pre-adolescent, an adolescent, an adult, or an elderly individual.

The term "disease," as used herein, generally refers to an abnormal physiological condition that affects part or all of a subject, such as an illness (e.g., asthma) or a cancer.

The term "cancer," as used herein, generally refers to any growth resulting from the abnormal division of cells. A cancer can be a malignant or benign growth or tumor resulting from the division of abnormal cells. A cancer can be a hematologic cancer or a cancer can be a solid cancer. A cancer can be disease can be a benign or a malignant uncontrolled division of abnormal cells in any part of the body of a subject. Non-limiting examples of cancers encompassed include breast cancer and lung cancer. Examples of lung cancers are non-small cell lung cancer, small cell lung cancer, and lung carcinoid tumor. Examples of breast cancers include metastatic breast cancer, inflammatory breast cancer, triple negative breast cancer (negative for progesterone, estrogen, and HER2/neu receptors), invasive ductal carcinoma, and ductal carcinoma in situ.

The term "polynucleotide" or "nucleic acid" as used herein refers to a polymeric form of nucleotides of any length, either ribonucleotides or deoxyribonucleotides, that comprise purine and pyrimidine bases, chemically or biochemically modified, natural or non-natural, or derivatized nucleotide bases. In some embodiments, a polynucleotide comprises purine and/or pyrimidine analogues. Polynucleotides include sequences of deoxyribonucleic acid (DNA), ribonucleic acid (RNA), or DNA copies of ribonucleic acid (cDNA), all of which can be recombinantly produced, artificially synthesized, or isolated and purified from natural sources. The polynucleotides and nucleic acids may exist as single-stranded or double-stranded. The backbone of the polynucleotide can comprise sugars and phosphate groups, as may typically be found in RNA or DNA, or modified or substituted sugar or phosphate groups. A polynucleotide may comprise modified nucleotides, including naturally occurring or non-naturally occurring nucleotides, such as methylated nucleotides and nucleotide analogues (or analogs). The sequence of nucleotides may be interrupted by non-nucleotide components.

The term "polyribonucleotide," as used herein, generally refers to polynucleotide polymers that contain greater than 50% of ribose bases, including unmodified and/or modified ribonucleotides. The term also refers to polynucleotide polymers that comprise ribonucleic acids, such as that contain greater than 50% of ribose bases, including unmodified and/or modified ribonucleotides. The term also refers to polynucleotide polymers that comprise chemically modified ribonucleotides, such as analogues. A polyribonucleotide can be formed of D-ribose , which can be found in nature, and L-ribose , which are not found in nature.

The term "polypeptides," as used herein, generally refers to polymer chains comprised of amino acid residue monomers which are joined together through amide bonds (peptide bonds). The amino acids may be the L-optical isomer or the D-optical isomer. A polypeptide can be a chain of at least three amino acids, peptide-mimetics, a protein, a recombinant protein, an antibody (monoclonal or polyclonal), an antibody fragment, a single-chain variable fragment (scFv), an antigen, an epitope, an enzyme, a receptor, a vitamin, or a structure analogue or combinations thereof. As used herein, the abbreviations for the L-enantiomeric and D-enantiomeric amino acids that form a polypeptide are as follows: alanine (A, Ala); arginine (R, Arg); asparagine (N, Asn); aspartic acid (D, Asp); cysteine (C, Cys); glutamic acid (E, Glu); glutamine (Q, Gln); glycine (G, Gly); histidine (H, His); isoleucine (I, Ile); leucine (L, Leu); lysine (K, Lys); methionine (M, Met); phenylalanine (F, Phe); proline (P, Pro); serine (S, Ser); threonine (T, Thr); tryptophan (W, Trp); tyrosine (Y, Tyr); valine (V, Val). X or Xaa can indicate any amino acid.

The term "engineered," as used herein, generally refers to non-naturally occurring, genetically modified polynucleotides, vectors, and nucleic acid constructs that have been genetically designed and manipulated to provide a polynucleotide intracellularly. In some embodiments, engineered refers to polynucleotides, vectors, and nucleic acid constructs that have been genetically designed and manipulated to provide a polynucleotide intracellularly. An engineered polynucleotide can be partially or fully synthesized in vitro. An engineered polynucleotide can also be cloned. An engineered polyribonucleotide can contain one or more modified bases or base or sugar analogues, such as ribonucleotides not naturally-found in messenger RNAs. An engineered polyribonucleotide can contain modified nucleotides or nucleotide analogues that exist in transfer RNAs (tRNAs), ribosomal RNAs (rRNAs), guide RNAs (gRNAs), small nuclear RNA (snRNA), small nucleolar RNA (snoRNA), SmY RNA, spliced leader RNA (SL RNA), CRISPR RNA, long noncoding RNA (lncRNA), microRNA (miRNA), or another suitable RNA.

### Overview

The present disclosure provides compositions and methods for the treatment of conditions with stable nucleic acids encoding a protein or protein fragment(s). The present disclosure also provides methods for delivering a polyribonucleotide that can be translated within a cell involved in surfactant production to a subject. For instance, the *ABCA3* gene provides instructions for making a protein involved in surfactant production. Surfactant can be a mixture of certain fats (called phospholipids) and proteins that lines the lung tissue and facilitates expansion and contraction of the lungs and breathing of a subject. Without normal surfactant, the tissue surrounding the air sacs in the lungs (the alveoli) sticks together after exhalation (because of a force called surface tension), causing the alveoli to collapse. As a result, filling the lungs with air on each breath can be difficult, and delivery of oxygen to the body can be impaired. This can lead to respiratory distress syndrome in subjects, such as infants.

The ABCA3 protein is typically found in the membrane that surrounds lamellar bodies, which are the cellular structures in which the phospholipids and proteins that make up surfactant are packaged. The ABCA3 protein can transport phospholipids into the lamellar bodies where they interact with surfactant proteins to form surfactant. The ABCA3 protein also appears to be involved in the formation of normal lamellar bodies. In addition to packaging, lamellar bodies can be important for the correct processing of surfactant proteins, which is necessary for the proteins to mature and become functional. An engineered polyribonucleotide of the disclosure can be delivered and translated within a cell of a subject to yield an ABCA3 protein, a functional fragment thereof, or a functional homolog to treat a condition that is associated with, for instance, a defect in surfactant production.

In some instances, the engineered polyribonucleotide comprises the genetic code of 5' untranslated regions (UTRs) and 3' UTRS shown in Table 1 (thereby, the CYBA 5' and CYBA 3' constructs are according to the invention and the other constructs are cited for reference purpose):

| **Table 1-UTRs** | |
|---|---|
| **UTR** | **DNA sequence (from 5' to 3')** |
| **CYBA 5'** | CGCGCCTAGCAGTGTCCCAGCCGGGTTCGTGTCGCC (SEQ ID NO: 1) |
| **CYBA 3'** | CCTCGCCCCGGACCTGCCCTCCCGCCAGGTGCACCCACCTGCAATAAATG CAGCGAAGCCGGGA (SEQ ID NO: 2) |
| **α-globin 5' UTR (HBA1)** | CATAAACCCTGGCGCGCTCGCGGCCCGGCACTCTTCTGGTCCCCACAGAC TCAGAGAGAACCCACC (SEQ ID NO: 3) |
| **α-globin 5' UTR (HBA2)** | CATAAACCCTGGCGCGCTCGCGGGCCGGCACTCTTCTGGTCCCCACAGAC TCAGAGAGAACCCACC (SEQ ID NO: 4) |
| α-**globin 5' UTR ETH** | TCTTCTGGTCCCCACAGACTCAGAGAGAAC (SEQ ID NO: 5) |
| **ABCA3 5'** | GCGGCCGCTGCGTCCGCCAGTAGCGGGTTGCAGGCGCACCCTCCCCTCCA GGGCGGCCACGCAGCTGTCAGTGCCGCCGCCACTGCGAGGCTGGAGCGGA GCCCGGGTGGCCGAGGGAGGGGACCCCGCGAGAGGGCCGCGCGCCGGCC GCCGCCGCCCCGGCGCCCAGGCTCGGTGCTGGAGAGTCATGCCTGTGAGC CCTGGGCACCTCCTGATGTCCTGCGAGGTCACGGTGTTCCCAAACCTCAGG GTTGCCCTGCCCCACTCCAGAGGCTCTCAGGCCCCACCCCGGAGCCCTCTG TGCGGAGCCGCCTCCTCCTGGCCAGTTCCCCAGTAGTCCTGAAGGGAGAC CTGCTGTGTGGAGCCTCTTCTGGGACCCAGCCATGAGTGTGGAGCTGAGC AACTGAACCTGAAACTCTTCCACTGTGAGTCAAGGAGGCTTTTCCGCACAT GAAGGACGCTGAGCGGGAAGGACTCCTCTCTGCCTGCAGTTGTAGCGAGT GGACCAGCACCAGGGGCTCTCTAGACTGCCCCTCCTCCATCGCCTTCCCTG CCTCTCCAGGACAGAGCAGCCACGTCTGCACACCTCGCCCTCTTTACACTC AGTTTTCAGAGCACGTTTCTCCTATTTCCTGCGGGTTGCAGCGCCTACTTG AACTTACTCAGACCACCTACTTCTCTAGCAGCACTGGGCGTCCCTTTCAGC AAGACG (SEQ ID NO: 6) |
| **ABCA3 3'** | GGGGTGGCGGCTGTCTCGCCATCAGGCAGGGACAGGACGGCAAGCAGG GCCCATCTTACATCCTCTCTCTCCAAGTTTATCTCATCCTTTATTTTTAATC ACTTTTTTCTATGATGGATATGAAAAATTCAAGGCAGTATGCACAGAATGG ACGAGTGCAGCCCAGCCCTCATGCCCAGGATCAGCATGCGCATCTCCATG TCTGCATACTCTGGAGTTCACTTTCCCAGAGCTGGGGCAGGCCGGGCAGTC TGCGGGCAAGCTCCGGGGTCTCTGGGTGGAGAGCTGACCCAGGAAGGGCT GCAGCTGAGCTGGGGGTTGAATTTCTCCAGGCACTCCCTGGAGAGAGGAC CCAGTGACTTGTCCAAGTTTACACACGACACTAATCTCCCCTGGGGAGGA AGCGGGAAGCCAGCCAGGTTGAACTGTAGCGAGGCCCCCAGGCCGCCAG GAATGGACCATGCAGATCACTGTCAGTGGAGGGAAGCTGCTGACTGTGAT TAGGTGCTGGGGTCTTAGCGTCCAGCGCAGCCCGGGGGCATCCTGGAGGC TCTGCTCCTTAGGGCATGGTAGTCACCGCGAAGCCGGGCACCGTCCCACA GCATCTCCTAGAAGCAGCCGGCACAGGAGGGAAGGTGGCCAGGCTCGAA GCAGTCTCTGTTTCCAGCACTGCACCCTCAGGAAGTCGCCCGCCCCAGGAC ACGCAGGGACCACCCTAAGGGCTGGGTGGCTGTCTCAAGGACACATTGAA TACGTTGTGACCATCCAGAAAATAAATGCTGAGGGGACACAGTC (SEQ ID NO: 7) |

The engineered polynucleotide can comprise the mRNA sequence of ABCA3 gene (SEQ ID NO: 8), the DNA sequence of ABCA3 (SEQ ID NO: 9), a native ABCA3 DNA sequence with a 5' CYBA UTR and a 3' CYBA UTR (SEQ ID NO: 10), a native ABCA3 mRNA sequence with a 5' CYBA UTR and a 3' CYBA UTR (SEQ ID NO: 11), a codon optimized ABCA3 DNA sequence with a 5' CYBA UTR and a 3' CYBA UTR (SEQ ID NO: 12), or a codon optimized ABCA3 mRNA sequence with a 5' CYBA UTR and a 3' CYBA UTR (SEQ ID NO: 13).

In some cases the engineered polyribonucleotide comprises at least a portion of the sequence of a gene in the ATP-binding cassette (ABC) family. The gene in the ATP-binding cassette family can be *ABCA1, ABCA3, ABCA4, ABCA12, ABCB4, ABCB7, ABCB11, ABCC2, ABCC6, ABCC8, ABCC9, ABCD1, ABCG5, ABCG8,* or *CFTR.* The engineered polyribonucleotide can comprise a sequence that is at least 10%, at least 20%, at least 30%, at least 40%, at least 50%, at least 60%, at least 70%, at least 80%, at least 90%, or at least 95% homologous to a sequence of a gene in the ATP-binding cassette (ABC) family.

### Engineered Polynucleotides

The present disclosure provides nucleic acid molecules, such as polynucleotides, which encode one or more polypeptides of interest. The term nucleic acid includes any compound and/or substance that comprise a polymer of nucleotides. Nucleotide polymers that contain greater than 50% of ribose bases or modified ribonucleotides or ribonucleotide analogues are referred to as polyribonucleotides. The sequence of the engineered polynucleotides can be derived from, for example, DNA, RNA, mRNA transcripts, genomic DNA, mitochondrial DNA, mitochondrial RNA, or another suitable nucleic acid that comprises the genetic information of a gene of interest. The nucleic acid constructs, vectors, engineered polynucleotides or polyribonucleotides, or compositions can be derived from nucleic acids carrying mutated genes and polymorphisms.

In addition to the four classical/canonical ribonucleotides, namely, adenosine, guanosine, cytidine and uridine, several cellular RNAs also contain a number of structurally diverse ribonucleotides. About a hundred structurally different nucleotides, modified nucleotides, or nucleotide analogues have been identified in transfer RNAs (tRNAs), ribosomal RNAs (rRNAs), messenger RNAs (mRNAs) and small nuclear RNAs (snRNAs). In tRNAs, some nucleotides or modified nucleotides can be important determinants of the specificity and efficiency of aminoacylation and codon recognition. Such structurally diverse ribonucleotides can be a modified ribonucleotide or a nucleotide analogue. In some cases a polynucleotide of the disclosure is engineered to comprise a modified ribonucleotide or ribonucleotide analogue.

Exemplary nucleic acids that can form a polynucleotide of the disclosure include, but are not limited to, ribonucleic acids (RNAs), deoxyribonucleic acids (DNAs), or hybrids thereof. Exemplary modified nucleotides that can form at least a fraction of a polynucleotide of the disclosure include, but are not limited to, pseudouridine (Ψ), 5-iodouridine (I⁵U), 5-iodocytidine (I⁵C), 2-thiouridine (s²U), 5-methylcytidine (m⁵C).

A modification, such as a chemical modification, can be located on one or more nucleoside(s) or the backbone of the nucleic acid molecule. They can be located on both a nucleoside and a backbone linkage. A modification can be engineered into a polynucleotide *in vitro.* Modified ribonucleotides and nucleic acid analogues can also be synthesized post-transcriptionally by covalent modification of the classical ribonucleotides.

An engineered polyribonucletide of the disclosure can comprise modified purines and pyrimidines or purine and pyrimidine analogues. In some cases, a polyribonucleotide of the disclosure comprises a modified pyrimidine, such as a modified uridine and/or a modified cytidine. In some cases a modified uridine or uridine analogue is selected from pseudouridine (Ψ), 2-thiouridine (s²U), 5-methyluridine (m⁵U), 5-methyluridine (m⁵U), 5-iodouridine (I⁵U), 4-thiouridine (s⁴U), 5-bromouridine (Br⁵U), 2'O-methyluridine (U2'm), 2'-amino-2'-deoxyuridine (U2'NH₂), 2'-azido-2'-deoxyuridine (U2'N₃), and 2'-fluoro-2'-deoxyuridine (U2'F). In some cases, a modified cytidine is selected from 5-methylcytidine (m⁵C), 3-methylcytidine (m³C), 2-thiocytidine (s²C), 2'-O-methylcytidine +(C2'm), 2'-amino-2'-deoxycytidine (C2'NH₂), 2'-fluoro-2'-deoxycytidine (C2'F), 5-iodocytidine (I⁵C), 5-bromocytidine 5'-triphosphate (Br⁵C) and 2'-azido-2'-deoxycytidine 5'-triphosphate (C2'N₃). Note that when referring to analogs, the foregoing (or the analogs listed in the tables below) also refers to analogs in their 5' triphosphate form.

In some instances the engineered polyribonucleotide comprises at least one modified ribonucleotide. In some cases, the modified ribonucleotide is at least 25% more stable in the subject as compared to a non-modified (or unmodified) ribonucleotide. In some cases, the modified nucleotide can be at least 30% more stable, at least 35% more stable, at least 40% more stable, at least 45% more stable, at least 50% more stable, at least 55% more stable, at least 60% more stable, at least 65% more stable, at least 70% more stable, at least 75% more stable, at least 80% more stable, at least 85% more stable, at least 90% more stable, or at least 95% more stable in the subject as compared to a non-modified ribonucleotide.

A polyribonucleotide can have nucleotides that have been modified in the same form or else a mixture of different modified nucleotides. The modified nucleotides can have modifications that are naturally or not naturally occurring in messenger RNA. A mixture of various modified nucleotides can be used. For example one or more modified nucleotides within a polynucleotide can have natural modifications, while another part has modifications that are not naturally found in mRNA. In some cases, the stability of the RNA can be selectively optimized by changing the nature of modified bases within the modified polyribonucleotide.

Non-limiting examples of uridine modifications that have an effect on the stability or immunogenicity of the polynucleotide are shown in **TABLE 2.**

| | **TABLE 2** | | |
|---|---|---|---|
| Name | Base modification | Sugar modification (2' position) | Naturally occurring in mRNA |
| Pseudouridine | - | | Yes |
| 5-methyluridine (m⁵U) | CH₃ | | No |
| 5-iodouridine (I⁵U) | I | | No |
| 5-bromouridine (Br⁵U) | Br | | No |
| 2-thiouridine (s²U) | S (in 2 position) | | No |
| 4-thiouridine (s⁴U) | S (in 4 position) | | No |
| 2'-O-methyluridine (U2'm) | - | CH₃ | Yes |
| 2 '-amino-2 '-deoxyuridine (U2'NH₂) | - | NH₂ | No |
| 2 '-azido-2 '-deoxyuridine (U2'N₃) | - | N₃ | No |
| 2'-fluoro-2'-deoxyuridine (U2'F) | - | F | No |

Non-limiting examples of cytidine modifications that have an effect on the stability or immunogenicity of the polynucleotide are shown in **TABLE 3**.

| | **TABLE 3** | | |
|---|---|---|---|
| Name | Base modification | Sugar modification (2' position) | Naturally occurring in mRNA |
| 5-methylcytidine (m⁵C) | CH₃ | | yes |
| 5-iodocytidine (I⁵C) | I | | no |
| 5-bromocytidine (Br⁵C) | Br | | no |
| 2-thiocytidine (s²C) | S (in 2 position) | | no |
| 2'-O-methylcytidine (C2'm) | - | CH₃ | yes |
| 2'-amino-2'-deoxycytidine (C2'NH₂) | - | NH₂ | no |
| 2'-azido-2'-deoxycytidine (C2'N₃) | - | N₃ | no |
| 2'-fluoro-2'-deoxycytidine (C2'F) | - | F | no |

Non-limiting examples of adenosine modifications that have an effect on the stability or immunogenicity of the polynucleotide are shown in **TABLE 4**.

| | **TABLE 4** | | |
|---|---|---|---|
| Name | Base modification | Sugar modification (2' position) | Naturally occurring in mRNA |
| N6-methyladenosine (m⁶A) | CH₃ (in 6 position) | | Yes |
| N1-methyladenosine (m¹A) | CH₃ (in 1 position) | | No |
| 2'-O-methyladenosine (A2'm) | - | CH₃ | Yes |
| 2'-amino-2'-deoxyadenosine (A2'NH₂) | - | NH₂ | No |
| 2'-azido-2'-deoxyadenosine (A2'N₃) | - | N₃ | No |
| 2'-fluoro-2'-deoxyadenosine (A2'F) | - | F | No |

Non-limiting examples of guanosine modifications that have an effect on the stability or immunogenicity of the polynucleotide are shown in **TABLE 5**.

| | **TABLE 5** | | |
|---|---|---|---|
| Name | Base modification (5'-position) | Sugar modification (2' position) | Naturally occurring in mRNA |
| N1-methylguanosine (m¹G) | CH₃ (in position 1) | | No |
| 2'-O-methylguanosine (G2'm) | - | CH₃ | Yes |
| 2'-amino-2'-deoxyguanosine (G2'NH₂) | - | NH₂ | No |
| 2'-azido-2'-deoxyguanosine (G2'N₃) | - | N₃ | No |
| 2'-fluoro-2'-deoxyguanosine (G2'F) | - | F | No |

A modified nucleotide can be selected from the group comprising pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl- uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thiopseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxypseudoisocytidine, 4-methoxy-1-methyl-pseudoisocytidine, 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6- diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, 2-methoxy-adenine, inosine, 1-methylinosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

In some cases, at least about 5 % of the engineered polyribonucleotide includes non-naturally occurring (e.g., modified or engineered) uracil, adenine, guanine, or cytosine, such as the modified nucleotides described herein. In some cases, at least about 10 %, 15 %, 20 %, 25 %, 30 %, 40 %, 45 %, 50 %, 55 %, 60 %, 65 %, 70 %, 75 %, 80 %, 85 %, 90 %, 95 % of the engineered polyribonucleotide includes non-naturally occurring uracil, adenine, guanine, or cytosine. In some cases, at most about 99 %, 95 %, 90 %, 85 %, 80 %, 75 %, 70 %, 65 %, 60 %, 55 %, 50 %, 45 %, 40 %, 35 %, 30 %, 25 %, 20 %, 15 %, 10 %, 5 %, 1 %, of the engineered polyribonucleotide includes non-naturally occurring uracil, adenine, guanine, or cytosine.

An engineered polyribonucleotide of the disclosure can comprise one or more promoter sequences and any associated regulatory sequences. A promoter sequence and/or an associated regulatory sequence can comprise any number of modified or unmodified nucleotides. Promoter sequences and/or any associated regulatory sequences can comprise, for example, at least 150 bases or base pairs, 200 bases or base pairs, 300 bases or base pairs, 400 bases or base pairs, 500 bases or base pairs, 600 bases or base pairs, 700 bases or base pairs, 800 bases or base pairs, 900 bases or base pairs, 1000 bases or base pairs, 2000 bases or base pairs, 3000 bases or base pairs, 4000 bases or base pairs, 5000 bases or base pairs, or at least 10000 bases or base pairs. A promoter sequence and/or an associated regulatory sequence can comprise any number of modified or unmodified nucleotides, for example, at most 10000 bases or base pairs, 5000 bases or base pairs, 4000 bases or base pairs, 3000 bases or base pairs, 2000 bases or base pairs, 1000 bases or base pairs, 900 bases or base pairs, 800 bases or base pairs, 700 bases or base pairs, 600 bases or base pairs, 500 bases or base pairs, 400 bases or base pairs, 300 bases or base pairs, 200 bases or base pairs, or 100 bases or base pairs.

In some cases, less than all of the nucleotides in the promoter sequence or associated regulatory region are modified. For instance, in some cases, less than or equal to 99%, 95%, 90%, 85%, 80%, 75%, 70%, 65%, 60%, 55%, 50%, 45%, 40%, 35%, 30%, 25%, 20%, 15%, 10%, or 5% of the nucleotides in a promoter or associated regulatory region. In some cases, all of the nucleotides in a promoter or associated regulatory region are modified.

An engineered polyribonucleotide of the disclosure can comprise an engineered 5' cap, or a 5' Cap can be added to a polyribonucleotide intracellularly. The 5'cap structure of an mRNA can be involved in binding to the mRNA Cap Binding Protein (CBP), which is responsible for mRNA stability in the cell and translation competency through the association of CBP with poly(A) binding protein to form the mature cyclic mRNA species. The 5'cap structure can also be involved in nuclear export, increases in mRNA stability, and in assisting the removal of 5' proximal introns during mRNA splicing.

An engineered polyribonucleotide can be 5-end capped generating a 5 -ppp-5'-triphosphate linkage between a terminal guanosine cap residue and the 5-terminal transcribed sense nucleotide of the mRNA molecule. The cap-structure can comprise a modified or unmodified 7-methylguanosine linked to the first nucleotide via a 5'-5' triphosphate bridge. This 5-guanylate cap can then be methylated to generate an N7-methyl-guanylate residue. The ribose sugars of the terminal and/or anteterminal transcribed nucleotides of the 5'end of the mRNA may optionally also be 2'-O-methylated. 5'-decapping through hydrolysis and cleavage of the guanylate cap structure may target a nucleic acid molecule, such as an mRNA molecule, for degradation.

In some cases, a cap can comprise further modifications, including the methylation of the 2' hydroxy-groups of the first 2 ribose sugars of the 5' end of the mRNA. For instance, an eukaryotic cap-1 has a methylated 2'-hydroxy group on the first ribose sugar, while a cap-2 has methylated 2'-hydroxy groups on the first two ribose sugars. Such double modification can provide significant resistance to 5' exonucleases. Non-limiting examples of 5' cap structures that can be used with an engineered polyribonucleotide include, but are not limited to, m⁷G(5')ppp(5')N (Cap-0), m⁷G(5')ppp(5')N1mpNp (Cap-1), and 7mG(5')-ppp(5')NlmpN2mp (Cap-2).

Modifications to the modified mRNA of the present disclosure may generate a non-hydrolyzable cap structure preventing decapping and thus increasing mRNA half-life. Because cap structure hydrolysis requires cleavage of 5'-ppp-5'phosphorodiester linkages, modified nucleotides may be used during the capping reaction. For example, a Vaccinia Capping Enzyme from New England Biolabs (Ipswich, MA) may be used with a-thio-guanosine nucleotides according to the manufacturer's instructions to create a phosphorothioate linkage in the 5'-ppp-5' cap. Additional modified guanosine nucleotides may be used such as a-methyl-phosphonate and seleno-phosphate nucleotides. Additional modifications include, but are not limited to, 2'-O-methylation of the ribose sugars of 5'-terminal and/or 5'-anteterminal nucleotides of the mRNA on the 2'-hydroxyl group of the sugar ring. Multiple distinct 5'-cap structures can be used to generate the 5'-cap of a polyribonucleotide.

The modified mRNA may be capped post-transcriptionally, According to the present disclosure, 5' terminal caps may include endogenous caps or cap analogues. According to the present disclosure, a 5' terminal cap may comprise a guanine analogue. Useful guanine analogues include, but are not limited to, inosine, Nl-methyl-guanosine, 2'fluoro-guanosine, 7-deaza-guanosine, 8-oxo-guanosine, 2-amino-guanosine, LNA-guanosine, and 2-azido-guanosine.

Further, an engineered polyribonucleotide can contain one or more internal ribosome entry site(s) (IRES). IRES sequences can initiate protein synthesis in absence of the 5' cap structure. An IRES sequence can also be the sole ribosome binding site, or it can serve as one of multiple ribosome binding sites of an mRNA. Engineered polyribonucleotides containing more than one functional ribosome binding site can encode several peptides or polypeptides that are translated by the ribosomes ("polycistronic or multicistronic polynucleotides"). An engineered polynucleotide described here can comprise at least 1 IRES sequence, two IRES sequences, three IRES sequences, four IRES sequences, five IRES sequences, six IRES sequences, seven IRES sequences, eight IRES sequences, nine IRES sequences, ten IRES sequences, or another suitable number are present in an engineered polyribonucleotide. Examples of IRES sequences that can be used according to the present disclosure include without limitation, those from picornaviruses (e.g., FMDV), pest viruses (CFFV), polio viruses (PV), encephalomyocarditis viruses (ECMV), foot-and-mouth disease viruses (FMDV), hepatitis C viruses (HCV), classical swine fever viruses (CSFV), murine leukemia virus (MLV), simian immune deficiency viruses (SIV) or cricket paralysis viruses (CrPV). An IRES sequence can be derived, for example, from commercially available vectors such as the IRES sequences available from Clontech™, GeneCopoeia™, Sigma-Aldrich™. IRES sequences can be, for example, at least 150 bases or base pairs, 200 bases or base pairs, 300 bases or base pairs, 400 bases or base pairs, 500 bases or base pairs, 600 bases or base pairs, 700 bases or base pairs, 800 bases or base pairs, 900 bases or base pairs, 1000 bases or base pairs, 2000 bases or base pairs, 3000 bases or base pairs, 4000 bases or base pairs, 5000 bases or base pairs, or 10000 bases or base pairs. IRES sequences can at most 10000 bases or base pairs, 5000 bases or base pairs, 4000 bases or base pairs, 3000 bases or base pairs, 2000 bases or base pairs, 1000 bases or base pairs, 900 bases or base pairs, 800 bases or base pairs, 700 bases or base pairs, 600 bases or base pairs, 500 bases or base pairs, 400 bases or base pairs, 300 bases or base pairs, 200 bases or base pairs, 100 bases or base pairs, 50 bases or base pairs, or 10 bases or base pairs.

An engineered polyribonucleotide of the disclosure comprise one or more untranslated regions. These are derived from the cytochrome b-245 alpha polypeptide gene. An untranslated can comprise any number of modified or unmodified nucleotides. Untranslated regions (UTRs) of a gene are transcribed but not translated into a polypeptide. In some cases, an untranslated sequence can increase the stability of the nucleic acid molecule and the efficiency of translation. The regulatory features of a UTR can be incorporated into the modified mRNA molecules of the present disclosure, for instance, to increase the stability of the molecule. The specific features can also be incorporated to ensure controlled down-regulation of the transcript in case they are misdirected to undesired organs sites. Some 5' UTRs play roles in translation initiation. A 5' UTR can comprise a Kozak sequence which is involved in the process by which the ribosome initiates translation of many genes. Kozak sequences can have the consensus CCR(A/G)CCAUGG, where R is a purine (adenine or guanine) that is located three bases upstream of the start codon (AUG). 5' UTRs may form secondary structures which are involved in binding of translation elongation factor. In some cases, one can increase the stability and protein production of the engineered polynucleotide molecules of the disclosure, by engineering the features typically found in abundantly expressed genes of specific target organs. For example, introduction of 5 'UTR of liver-expressed mRNA, such as albumin, serum amyloid A, Apolipoprotein A/B/E, transferrin, alpha fetoprotein, erythropoietin, or Factor VIII, can be used to increase expression of an engineered polynucleotide in a liver. Likewise, use of 5' UTR from muscle proteins (MyoD, Myosin, Myoglobin, Myogenin, Herculin), for endothelial cells (Tie-1, CD36), for myeloid cells (C/EBP, AML1, G-CSF, GM-CSF, CD1 lb, MSR, Fr-1, i-NOS), for leukocytes (CD45, CD18), for adipose tissue (CD36, GLUT4, ACRP30, adiponectin) and for lung epithelial cells (SP-A/B/C/D) can be used to increase expression of an engineered polynucleotide in a desired cell or tissue. In some cases a UTR of the disclosure can be derived from the sequence of a cytochrome b-245 alpha polypeptide (CYBA), an α-globin gene, or a gene of the ATP-binding cassette (ABC) family, such as the ABCA3 gene.

According to the invention, the composition comprising a modified polyribonucleotide comprises one or more untranslated regions (UTR) derived from the sequence of a cytochrome b-245 alpha polypeptide (CYBA). Also according to the invention, the composition comprising a modified polyribonucleotide comprising one or more untranslated region (UTR) derived from the sequence of a cytochrome b-245 alpha polypeptide (CYBA) is a composition wherein the modified polyribonucleotide encodes a gene of the ABC-binding cassette (ABC) family as described herein above and below.

In the following, preferred embodiments of the untranslated region(s) (UTR) derived from the sequence of a cytochrome b-245 alpha polypeptide (CYBA) are described:
In the above **Table 1**, the DNA sequences displaying the human CYBA gene 5'- and 3' UTRs are shown as SEQ ID NO:1 and SEQ ID NO:2, respectively.

In view of the fact that the present invention predominantly relates to an RNA molecule (i.e., a modified polyribonucleotide molecule in terms of the present invention) reference is made in the following to the corresponding RNA sequences of said UTRs.

Derived from the above DNA sequence, SEQ ID NO:1 corresponds to the following UTR sequence on the RNA level:
5'-CGCGCCUAGCAGUGUCCCAGCCGGGUUCGUGUCGCC-3' (SEQ ID NO:14).

This 5'UTR sequence immediately precedes the start codon of the human CYBA gene.

Derived from the above DNA sequence, SEQ ID NO:2 corresponds to the following UTR sequence on the RNA level:

5'-CCUCGCCCCGGACCUGCCCUCCCGCCAGGUGCACCC
ACCUGCAAUAAAUGCAGCGAAGCCGGGA-3' (SEQ ID NO:15)).

SEQ ID NO: 5 corresponds, on the RNA level to the sequence set forth in SEQ ID NO: 16.

The term "untranslated region" or "UTR" as used in accordance with the present invention relates sections of the mRNA upstream the start codon and downstream the stop codon that are not translated, and are, therefore, termed the five prime untranslated region (5' UTR) and three prime untranslated region (3' UTR), respectively. These regions are transcribed with the coding region and thus are exonic as they are present in the mature mRNA.

As used in the present invention, the 3' untranslated region (3'-UTR) relates to the section of messenger RNA (mRNA) that immediately follows the translation termination codon. An mRNA molecule is transcribed from the DNA sequence and is later translated into protein. Several regions of the mRNA molecule are not translated into protein including the 5' cap, 5' UTR, 3' UTR, and the poly-A tail.

As used in the present invention, the 5' untranslated region (5' UTR) (also known as a Leader Sequence or Leader RNA) is the region of an mRNA that is directly upstream from the start codon. The 5' UTR begins at the transcription start site and ends one nucleotide (nt) before the start codon (usually AUG) of the coding region. In prokaryotes, the length of the 5' UTR tends to be 3-10 nucleotides long while in eukaryotes it tends to be, longer, generally from 100 to several thousand nucleotides long but sometimes also shorter UTRs occur in eukaryotes.

As used in the present invention, the 3' UTR may comprise regulatory regions within the 3'-untranslated region which are known to influence polyadenylation and stability of the mRNA. Many 3'-UTRs also contain AU-rich elements (AREs). Furthermore, the 3'-UTR contains the sequence AAUAAA that directs addition of several hundred adenine residues called the poly(A) tail to the end of the mRNA transcript.

Thus, an RNA molecule as used in accordance with the present invention may also contain a poly-A tail. A poly-A tail is a long sequence of adenine nucleotides (often several hundred) added to the 3' end of the pre-mRNA by a process called polyadenylation. This tail promotes export from the nucleus and translation, and protects the mRNA from degradation. Polyadenylation is the addition of a poly(A) tail to a messenger RNA. The poly(A) tail consists of multiple adenosine monophosphates; in other words, it is a stretch of RNA that has only adenine bases. In eukaryotes, polyadenylation is part of the process that produces mature messenger RNA (mRNA) for translation. As used herein, a poly-A tail relates to a sequence of adenine nucleotides located at the 3' end of the RNA. A poly-A tail is commonly added to the 3' end of the RNA by a process called polyadenylation. Thus, the present invention relates to any of the above-described RNA, wherein the RNA molecule comprises a poly-A tail at the 3' end.

The length of the poly-A tail is not particularly limited. Yet, in preferred embodiments, the RNA molecule of the present invention comprises a poly-A tail at the 3' end wherein the poly-A tail has a length of at least 50, 60, 70, 80, 90, 100 or 110 nucleotides. In a more preferred embodiment, the RNA molecule of the present invention comprises a poly-A tail at the 3' end wherein the poly-A tail has a length of at least 120 nucleotides. In other preferred embodiments, the RNA molecule of the present invention comprises a poly-A tail at the 3' end wherein the poly-A tail has a length of at least 150, 200, 250, 300, 350, 400, 500, 600, 700, 800, 900 or 1000 nucleotides.

In a preferred embodiment, the composition comprising a modified polyribonucleotide/RNA molecule of the present invention comprising one or more untranslated regions (UTR) derived from the sequence of a cytochrome b-245 alpha polypeptide (CYBA) is a composition wherein the one or more UTR(s) comprise(s) the sequence as shown in SEQ ID NO:14 or a sequence which shows 1 to 4 substitutions in comparison to SEQ ID NO:14 and which results in an RNA molecule having the same or a higher translation efficiency as an RNA molecule comprising an UTR comprising SEQ ID NO:14.

"One or more" in this context means that the RNA molecule may harbor one UTR comprising the sequence as shown in SEQ ID NO:14 or a sequence which shows 1 to 4 substitutions in comparison to SEQ ID NO:14 and which results in an RNA molecule having the same or a higher translation efficiency as an RNA molecule comprising an UTR comprising SEQ ID NO:14 of the present invention. The RNA molecule may also harbor two, three or four of these UTRs of the present invention. Alternatively, the RNA molecule may also harbor five or even more of these UTRs of the present invention.

In another preferred embodiment, the composition comprising a modified polyribonucleotide/RNA molecule of the present invention comprising one or more untranslated regions (UTR) derived from the sequence of a cytochrome b-245 alpha polypeptide (CYBA) is a composition wherein the one or more UTR(s) comprises the sequence as shown in SEQ ID NO:15 or a sequence which shows 1 to 7 substitutions in comparison to SEQ ID NO:15 and which results in an RNA molecule having the same or a higher translation efficiency as an RNA molecule comprising an UTR comprising SEQ ID NO:15.

"One or more" in this context means that the RNA molecule may harbor one UTR comprising the sequence as shown in SEQ ID NO:15 or a sequence which shows 1 to 7 substitutions in comparison to SEQ ID NO:15 and which results in an RNA molecule having the same or a higher translation efficiency as an RNA molecule comprising an UTR comprising SEQ ID NO:15 of the present invention. The RNA molecule may also harbor two, three or four of these UTRs of the present invention. Alternatively, the RNA molecule may also harbor five or even more of these UTRs of the present invention.

However, the UTRs derived from the cytochrome b-245 alpha polypeptide gene as used in the present invention are not particularly limited to the above specific sequence of SEQ ID NO:14 but may also be a UTR sequence which comprises a sequence which shows 1 to 4 substitutions in comparison to SEQ ID NO:14. Alternatively, the UTR sequence may also be a sequence which comprises a sequence which shows 1 to 3 substitutions in comparison to SEQ ID NO:14. The UTR sequence may also be a sequence which comprises a sequence which shows 1 to 2 substitutions in comparison to SEQ ID NO:14. Most preferably, the UTR sequence may also be a sequence which comprises a sequence which shows 1 substitution, in comparison to SEQ ID NO:14.

Preferably, the position of the above nucleotide substitution in comparison to SEQ ID NO:14 is performed at position 32 in the sequence of SEQ ID NO: 14. Preferably, the nucleotide "U" at this position is substituted by a "C". This substitution is preferred since it brings the Kozak element of CYBA which is (partially) present in SEQ ID NO:1 closer to the Kozak consensus sequence of vertebrates. The Kozak consensus sequence of vertebrates has the sequence of GCCRCCAUGG (the start codon is underlined while "R" indicates any purine) while the Kozak element of CYBA has the sequence of GuCGCCAUGG (the start codon is underlined while the deviation from the vertebrate consensus sequence is indicated by the lower case letter "u").

The UTR sequence(s) which have one or more of the above substitutions in comparison to SEQ ID NO:14 may result in an RNA molecule in the same or similar capability in terms of the translation efficiency as an RNA molecule comprising an UTR comprising SEQ ID NO:14, preferably a higher capability in terms of the translation efficiency as an RNA molecule comprising an UTR comprising SEQ ID NO: 14. The property/capability of a given modified UTR sequence in comparison to in terms of the translation efficiency as an RNA molecule comprising an UTR comprising SEQ ID NO:14 with respect to the translation efficiency can be determined by the skilled person by methods known in the art and as outlined in the appended examples.

The translation efficiency is the rate of mRNA translation into polypeptides or proteins within cells. The translation efficiency of a given mRNA is measured as the number of proteins or polypeptides which are translated per mRNA per time unit. Translation is the process in which cellular ribosomes create proteins and is well-known to the skilled person. Briefly, in translation, messenger RNA (mRNA) which is produced by transcription from DNA is decoded by a ribosome to produce a specific amino acid chain or a polypeptide or a protein.

Thus, the translation efficiency of a given RNA molecule of the present invention harboring a modified UTR sequence is preferably higher in comparison to a translation efficiency of the same given RNA but harboring an UTR of SEQ ID NO: 14. Accordingly, the number of proteins or polypeptides encoded by the gene of the ABC-binding cassette (ABC) family member of the RNA molecule harboring a modified UTR sequence which are translated per RNA per time unit is higher than the number of proteins or polypeptides encoded by the gene of the ABC-binding cassette (ABC) family member of the RNA molecule harboring an UTR of SEQ ID NO: 14 which are translated per RNA per time unit.

In case the translation efficiency of a given RNA molecule harboring a modified UTR sequence is similar or the same in comparison to a translation efficiency of the same given RNA but harboring an UTR of SEQ ID NO: 14, the number of proteins or polypeptides encoded by the gene of the ABC-binding cassette (ABC) family member of the RNA molecule harboring a modified UTR sequence which are translated per RNA per time unit is similar to or the same as the number of proteins or polypeptides encoded by the gene of the ABC-binding cassette (ABC) family member of the RNA molecule harboring an UTR of SEQ ID NO:14 which are translated per RNA per time unit. The "translation efficiency" can, e.g., be determined by methods described in the appended examples and as outlined in the following.

Translation efficiency, in the context of the present invention, is the rate of mRNA translated into protein within a cell at a certain time point in relation to the amount of mRNA encoding the respective protein in said cell at the same time point. Thus, the translation efficiency is the quotient of the mRNA translated into protein within a cell at a certain time point and the amount of mRNA encoding the respective protein. Both parameters, i.e., the mRNA translated into a protein as well as the amount of mRNA encoding the respective protein, can be determined by methods known in the art. As it has been done in the appended examples, as non-limiting examples, the amount of mRNA translated into protein within a cell can, e.g., be determined by as determined by flow cytometry (FC) while the amount of mRNA encoding the respective protein can, e.g., be measured by qPCR.

The UTR(s) comprising the sequence as shown in SEQ ID NO:14 or a sequence which shows 1 to 4 substitutions in comparison to SEQ ID NO:14 and which results in an RNA molecule having the same or a higher translation efficiency as an RNA molecule comprising an UTR comprising SEQ ID NO:14 as used in the present invention is/are not particularly limited to the above specific sequences and the above described substitutions but may also relate to (an) UTR sequence(s) which comprise(s) a sequence which shows (a) nucleotide(s) addition(s) in comparison to SEQ ID NO:14. The addition of (a) nucleotide(s) can be flanking. Thus, the additional nucleotide(s) may be added at the 3'-end or 5'-end of the UTR(s) of the present invention. The additional nucleotide(s) comprise polynucleotide chains of up to 0 (no changes), 1, 2, 3, 4, 5, 6, 7, 8, 9 or 10 nucleotides, preferably of up to 20 nucleotides or even more preferably of up to 30 nucleotides. In light of the rationale that the addition of nucleotides is likely not to change the above functional properties of the UTR(s) of the invention the addition of the nucleotides may also have a length of up to 40, 50, 60, 70, 80, 90, or even 100 nucleotides or even more, up to 200, 300, 400 or 500 nucleotides as long as these sequences have a similar capability (in terms of the above-described translation efficiency) as SEQ ID NO: 14, preferably higher translation efficiency as SEQ ID NO:14 as defined above.

Alternatively, or in addition to these flanking additions of (a) nucleotide(s) the addition of (a) nucleotide(s) can be interspersed. Thus, the additional nucleotide(s) may be added/inserted within the nucleotide sequence of the UTR(s) of the present invention. These nucleotide(s) insertions comprise 1, 2, or 3 nucleotides as long as these sequences have a similar capability (in terms of the above-described translation efficiency) as SEQ ID NO:14, preferably higher translation efficiency as SEQ ID NO:14 as defined above.

The UTRs as used in the present invention are not particularly limited to the above specific sequence of SEQ ID NO:14 and modifications thereof. Rather, the specific sequence of SEQ ID NO:14 and modifications thereof merely define the CYBA 5' core region. Thus, in a preferred embodiment, the UTR as shown in SEQ ID NO:14 is extended on the 5' end (i.e., upstream) by at least 1 nucleotide. In another preferred embodiment, the UTR as shown in SEQ ID NO:14 is extended on the 5' end (i.e., upstream) by 1 to 20 nucleotides. Hence, in a preferred embodiment, the sequence of SEQ ID NO:14 extends by 20 nucleotides on the 5' end (i.e., upstream). In other preferred embodiments, the sequence of SEQ ID NO:14 extends by 18, 15, 13, 10, 7 or 5 nucleotides on the 5' end (i.e., upstream). In other preferred embodiments, the sequence of SEQ ID NO:14 extends by 4, 5 or 2 nucleotides on the 5' end (i.e., upstream). In other preferred embodiment, the sequence of SEQ ID NO:14 extends by 1 nucleotide on the 5' end (i.e., upstream).

These UTR sequences which are extended on the 5' end (i.e., upstream) may also be modified as defined herein above for SEQ ID NO:14. Accordingly, the same applies, mutatis mutandis, to the UTRs which are extended on the 5' end as defined above as has been set forth above in the context of the UTR of SEQ ID NO:14.

Moreover, the UTRs as used in the present invention are also not particularly limited to the above specific sequence of SEQ ID NO:15 but may also be a UTR sequence which comprises a sequence which shows 1 to 7 substitutions in comparison to SEQ ID NO: 15. Alternatively, the UTR sequence may also be a sequence which comprises a sequence which shows 1 to 6 substitutions in comparison to SEQ ID NO:15. The UTR sequence may also be a sequence which comprises a sequence which shows 1 to 5 substitutions in comparison to SEQ ID NO: 15. The UTR sequence may also be a sequence which comprises a sequence which shows 1 to 4 substitutions in comparison to SEQ ID NO:15. The UTR sequence may also be a sequence which comprises a sequence which shows 1 to 3 substitutions in comparison to SEQ ID NO: 15. The UTR sequence may also be a sequence which comprises a sequence which shows 1 to 2 substitutions in comparison to SEQ ID NO:15. The UTR sequence may also be a sequence which comprises a sequence which shows 1 to 3 substitutions in comparison to SEQ ID NO:15. Most preferably, the UTR sequence may also be a sequence which comprises a sequence which shows 1 substitution, in comparison to SEQ ID NO:15.

The UTR sequence(s) which have one or more of the above substitutions in comparison to SEQ ID NO:15 may result in an RNA molecule in the same or similar capability in terms of the translation efficiency as an RNA molecule comprising an UTR comprising SEQ ID NO: 15, preferably a higher capability in terms of the translation efficiency as an RNA molecule comprising an UTR comprising SEQ ID NO:15. The property/capability of a given modified UTR sequence in comparison to in terms of the translation efficiency as an RNA molecule comprising an UTR comprising SEQ ID NO:15 with respect to the translation efficiency can be determined by the skilled person by methods known in the art and as outlined in the appended examples.

The translation efficiency is the rate of mRNA translation into polypeptides or proteins within cells. The translation efficiency of a given mRNA is measured as the number of proteins or polypeptides which are translated per mRNA per time unit. Translation is the process in which cellular ribosomes create proteins and is well-known to the skilled person. Briefly, in translation, messenger RNA (mRNA) which is produced by transcription from DNA is decoded by a ribosome to produce a specific amino acid chain or a polypeptide or a protein.

Thus, the translation efficiency of a given RNA molecule harboring a modified UTR sequence is preferably higher in comparison to a translation efficiency of the same given RNA but harboring an UTR of SEQ ID NO:15. Accordingly, the number of proteins or polypeptides encoded by the gene of the ABC-binding cassette (ABC) family member of the RNA molecule harboring a modified UTR sequence which are translated per RNA per time unit is higher than the number of proteins or polypeptides encoded by the coding region of the RNA molecule harboring an UTR of SEQ ID NO:15 which are translated per RNA per time unit.

In case the translation efficiency of a given RNA molecule harboring a modified UTR sequence is similar or the same in comparison to a translation efficiency of the same given RNA but harboring an UTR of SEQ ID NO:15, the number of proteins or polypeptides encoded by the coding region of the RNA molecule harboring a modified UTR sequence which are translated per RNA per time unit is similar to or the same as the number of proteins or polypeptides encoded by the gene of the ABC-binding cassette (ABC) family member of the RNA molecule harboring an UTR of SEQ ID NO:15 which are translated per RNA per time unit.

The "translation efficiency" can, e.g., be determined by methods described in the appended examples and as outlined above.

The UTR(s) comprising the sequence as shown in SEQ ID NO:15 or a sequence which shows 1 to 7 substitutions in comparison to SEQ ID NO:15 and which results in an RNA molecule having the same or a higher translation efficiency as an RNA molecule comprising an UTR comprising SEQ ID NO:15 as used in the present invention is/are not particularly limited to the above specific sequences and the above described substitutions but may also relate to (an) UTR sequence(s) which comprise(s) a sequence which shows (a) nucleotide(s) addition(s) in comparison to SEQ ID NO:15. The addition of nucleotide(s) can be flanking or interspersed. Thus, the additional nucleotide(s) may be added at the 3 '-end or 5'-end of the UTR(s) of the present invention. Alternatively, or in addition to these flanking additional nucleotide(s), the additional nucleotide(s) may also be within the nucleotide sequence of the UTR(s) of the present invention. The additional nucleotide(s) comprise polynucleotide chains of up to 0 (no changes), 1, 2, 3, 4, 5, 6, 7, 8, 9 or 10 nucleotides, preferably of up to 20 nucleotides or even more preferably of up to 30 nucleotides. In light of the rationale that the addition of nucleotides is likely not to change the above functional properties of the UTR(s) of the invention the addition of the nucleotides may also have a length of up to 40, 50, 60, 70, 80, 90, or even 100 nucleotides or even more, up to 200, 300, 400 or 500 nucleotides as long as these sequences have a similar capability (in terms of the above-described translation efficiency) as SEQ ID NO:15, preferably higher translation efficiency as SEQ ID NO:15 as defined above.

The UTR(s) of the present invention as well as RNA molecules containing such UTR(s) may be recombinantly (e.g., in an in vivo or an in vitro system) or synthetically generated/synthesized by methods known to the person skilled in the art.

More specifically, the UTRs of the present invention and RNA molecules containing such UTR(s) may be produced either recombinantly in in vivo systems by methods known to the person skilled in the art.

Alternatively, the UTRs of the present invention and RNA molecules containing such UTR(s) may be produced in an in vitro system using, for example, an in vitro transcription system. In vitro transcription systems are commonly known and usually require a purified linear DNA template containing a DNA sequence "encoding" module (b) and/or module (c) as outlined in detail further below wherein said DNA sequence is under the control of an appropriate promoter. Moreover, an in vitro transcription system also commonly requires ribonucleoside triphosphates, a buffer system that includes DTT and magnesium ions, and an appropriate RNA polymerase which provides the enzymatic activity for the in vitro transcription of the DNA sequence "encoding" said UTR(s) into the UTR(s) of the present invention.

Furthermore, the UTRs of the present invention and RNA molecules containing such UTR(s) may be chemically synthesized, e.g., by conventional chemical synthesis on an automated nucleotide sequence synthesizer using a solid-phase support and standard techniques or by chemical synthesis of the respective DNA-sequences and subsequent in vitro or in vivo transcription of the same.

In molecular biology and genetics, upstream and downstream both refer to a relative position in an RNA molecule. In the context of the present invention, upstream is toward the 5' end of the RNA molecule and downstream is toward the 3' end of the molecule.

Accordingly, in one embodiment, the UTR comprising the sequence as shown in SEQ ID NO:14 or a sequence which has 1 to 4 substitutions in comparison to SEQ ID NO:14 and which results in an RNA molecule having the same or a higher translation efficiency as an RNA molecule comprising an UTR comprising SEQ ID NO:14 as defined hereinabove (in the following referred to as "module (b)") is located upstream of the modified polyribonucleotide encoding the ABC-binding cassette (ABC) family member as described herein above and below (in the following referred to as "module (a)"). Moreover, in one embodiment, the UTR comprising the sequence as shown in SEQ ID NO:15 or a sequence which shows 1 to 7 substitutions in comparison to SEQ ID NO:15 and which results in an RNA molecule having the same or a higher translation efficiency as an RNA molecule comprising an UTR comprising SEQ ID NO:15 as defined hereinabove (in the following referred to as "module (c)") is located downstream of the modified polyribonucleotide encoding the ABC-binding cassette (ABC) family member as described herein above and below (in the following referred to as "module (a)"). Yet, preferably, the gene encoding the ABC-binding cassette (ABC) family member ("module (a)") is located between the UTR module (b) and the UTR module (c) and, accordingly, the RNA molecule preferably has the arrangement of 5'-(b)-(a)-(c)-3'.

In case the RNA molecule only harbors one UTR module (i.e., either module (b) (i.e., the one or more UTR(s) comprising the sequence as shown in SEQ ID NO:14 or a sequence which shows 1 to 4 substitutions in comparison to SEQ ID NO:14 and which results in an RNA molecule having the same or a higher translation efficiency as an RNA molecule comprising an UTR comprising SEQ ID NO:14 as defined hereinabove) or module (c) (i.e., the one or more UTR(s) comprising the sequence as shown in SEQ ID NO:15 or a sequence which shows 1 to 7 substitutions in comparison to SEQ ID NO:15 and which results in an RNA molecule having the same or a higher translation efficiency as an RNA molecule comprising an UTR comprising SEQ ID NO:15 as defined hereinabove)) the RNA molecule preferably has the arrangement of 5'-(b)-(a)-3' or 5'-(a)-(c)-3'.

In the following, preferred arrangements of the UTR modules (b) and/or (c) of the present invention in relation to the modified polyribonucleotide encoding the ABC-binding cassette (ABC) family member ("module (a)") are described wherein the UTR module (b) (corresponding to the above-defined 5' UTR fragment of the CYBA mRNA) is located upstream of the coding region (i.e., at the 5' end of the coding region) and/or the UTR module (c) (corresponding to the above-defined 3' UTR of the CYBA mRNA) is located downstream of the coding region (i.e., at the 3' end of the coding region).

Thus, in a preferred embodiment, and in accordance with the foregoing, the present invention relates to an RNA molecule comprising (a) a modified polyribonucleotide, i.e., a coding region encoding the ABC-binding cassette (ABC) family member; and (b) one or more UTR(s) comprising the sequence as shown in SEQ ID NO:14 or a sequence which shows 1 to 4 substitutions in comparison to SEQ ID NO:14 and which results in an RNA molecule having the same or a higher translation efficiency as an RNA molecule comprising an UTR comprising SEQ ID NO:14, and wherein said UTR(s) as defined in (b) is/are located at the 5' end of the coding region as defined in (a).

In a preferred embodiment, and in accordance with the foregoing, the present invention relates to an RNA molecule comprising (a) a coding region coding for an ABC-binding cassette (ABC) family member; and (c) one or more UTR(s) comprising the sequence as shown in SEQ ID NO:15 or a sequence which shows 1 to 7 substitutions in comparison to SEQ ID NO:15 and which results in an RNA molecule having the same or a higher translation efficiency as an RNA molecule comprising an UTR comprising SEQ ID NO:15, wherein said UTR(s) as defined in (c) is/are located at the 3' end of the coding region as defined in (a).

In a preferred embodiment, and in accordance with the foregoing, the present invention relates to an RNA molecule comprising (a) a coding region coding an ABC-binding cassette (ABC) family member; and (b) one or more UTR(s) comprising the sequence as shown in SEQ ID NO:14 or a sequence which shows 1 to 4 substitutions in comparison to SEQ ID NO:14 and which results in an RNA molecule having the same or a higher translation efficiency as an RNA molecule comprising an UTR comprising SEQ ID NO: 14; and (c) one or more UTR(s) comprising the sequence as shown in SEQ ID NO:15 or a sequence which shows 1 to 7 substitutions in comparison to SEQ ID NO:15 and which results in an RNA molecule having the same or a higher translation efficiency as an RNA molecule comprising an UTR comprising SEQ ID NO:15, wherein said UTR(s) as defined in (b) is/are located at the 5' end of the coding region as defined in (a) and wherein said UTR(s) as defined in (c) is/are located at the 3' end of the coding region as defined in (a).

In a preferred embodiment, and in accordance with the foregoing, the present invention relates to an RNA molecule comprising (a) a coding region coding for an ABC-binding cassette (ABC) family member; and (b) one UTR comprising the sequence as shown in SEQ ID NO:14 or a sequence which shows 1 to 4 substitutions in comparison to SEQ ID NO:14 and which results in an RNA molecule having the same or a higher translation efficiency as an RNA molecule comprising an UTR comprising SEQ ID NO:14; and (c) two UTRs comprising the sequence as shown in SEQ ID NO:15 or a sequence which shows 1 to 7 substitutions in comparison to SEQ ID NO:15 and which results in an RNA molecule having the same or a higher translation efficiency as an RNA molecule comprising an UTR comprising SEQ ID NO:15; wherein said RNA molecule comprises said one UTR as defined in (b) at the 5' end of the coding region as defined in (a) and which comprises said two UTRs as defined in (c) at the 3' end of the coding region as defined in (a).

In a preferred embodiment, and in accordance with the foregoing, the present invention relates to an RNA molecule comprising (a) a coding region coding an ABC-binding cassette (ABC) family member; and (c) two UTRs comprising the sequence as shown in SEQ ID NO:15 or a sequence which shows 1 to 7 substitutions in comparison to SEQ ID NO:15 and which results in an RNA molecule having the same or a higher translation efficiency as an RNA molecule comprising an UTR comprising SEQ ID NO:15, wherein said RNA molecule comprises said two UTRs as defined in (c) at the 3' end of the coding region as defined in (a).

The construct according to the present invention may not only comprise the above three main modules (a), (b) and/or (c). Rather, it may be desirable that between the individual modules (a) linker moiety/moieties and/or (a) multiple cloning site(s) is/are placed which may, e.g., facilitate the construction of the construct. Suitable linker moieties and multiple cloning sites are known to the skilled person.

The position of the UTR modules (b) and/or (c) within the RNA molecule of the present invention in relation to module (a) (i.e., the coding region coding an ABC-binding cassette (ABC) family member), is not particularly limited and, accordingly, between the individual modules of the RNA molecule of the present invention there may be a spacing or a gap filled with one or more nucleotides G, A, U and/or C which are not part of the main modules (a), (b) and/or (c).

"One or more nucleotides G, A, U and/or C" in this context means that the spacing or gap between the individual modules of the RNA molecule of the present invention is/are filled with 1, 2, 3, 4, 5, 6, 7, 8, 9 or 10 nucleotides G, A, U and/or C. In other preferred embodiments, the spacing or gap between the individual modules of the RNA molecule of the present invention are filled with 20, 30, 40, 50, 60, 70, 80, 90, 100 or 110 or more nucleotides G, A, U and/or C.

Yet, in a preferred embodiment, the UTR module (b) or (c), within the RNA molecule of the present invention in relation to module (a) (i.e., the coding region coding an ABC-binding cassette (ABC) family member), is directly placed adjacent to the start codon of the coding region of module (a) without any spacing or gap in between, i.e., directly upstream of the start codon of the coding region of module (a).

In another preferred embodiment, the UTR module (b) or (c), within the RNA molecule of the present invention in relation to module (a) (i.e., the coding region), is directly placed adjacent to the termination codon (i.e., the stop codon) of the coding region of module (a) coding an ABC-binding cassette (ABC) family member without any spacing or gap in between, i.e., directly downstream of the termination codon/stop codon of the coding region of module (a) coding an ABC-binding cassette (ABC) family member.

In a preferred embodiment, the UTR module (b), within the RNA molecule of the present invention in relation to module (a) (i.e., the coding region coding an ABC-binding cassette (ABC) family member), is directly placed adjacent to the start codon of the coding region of module (a) coding an ABC-binding cassette (ABC) family member without any spacing or gap in between, i.e., directly upstream of the start codon of the coding region of module (a) coding an ABC-binding cassette (ABC) family member and the UTR module (c), within the RNA molecule of the present invention in relation to module (a) (i.e., the coding region coding an ABC-binding cassette (ABC) family member), is directly placed adjacent to the termination codon (i.e., the stop codon) of the coding region of module (a) coding an ABC-binding cassette (ABC) family member without any spacing or gap in between, i.e., directly downstream of the termination codon/stop codon of the coding region of module (a) coding an ABC-binding cassette (ABC) family member.

In even more preferred embodiments, the composition comprising a modified polyribonucleotide of the present invention comprising one or more untranslated regions (UTRs) derived from the sequence of a cytochrome b-245 alpha polypeptide (CYBA) as defined herein above is a composition wherein the modified polynucleotide encodes the ABC-binding cassette (ABC) 3 protein (ABCA3), preferably encodes the human ABCA3 protein, wherein said modified polynucleotide includes a codon sequence that is optimized for translation within cells of the subject exposed to the modified polyribonucleotide.

In a most preferred embodiment, the composition comprising a modified polyribonucleotide of the present invention comprising one or more untranslated regions (UTRs) derived from the sequence of a cytochrome b-245 alpha polypeptide (CYBA) as defined herein above is the mRNA sequence of a codon optimized ABCA3 DNA sequence with a 5' CYBA UTR and a 3' CYBA UTR (SEQ ID NO: 12), or a codon optimized ABCA3 mRNA sequence with a 5' CYBA UTR and a 3' CYBA UTR (SEQ ID NO: 13).

Other non-UTR sequences can be incorporated into the 5' (or 3' UTR) UTRs of the polyribonucleotides of the present disclosure. The 5' and/or 3' UTRs can provide stability and/or translation efficiency of polyribonucleotides. For example, introns or portions of intron sequences can be incorporated into the flanking regions of an engineered polyribonucleotide. Incorporation of intronic sequences can also increase the rate of translation of the polyribonucleotide.

3' UTRs may have stretches of Adenosines and Uridines embedded therein. These AU rich signatures are particularly prevalent in genes with high rates of turnover. Based on their sequence features and functional properties, the AU rich elements (AREs) can be separated into classes: Class I AREs contain several dispersed copies of an AUUUA motif within U-rich regions. C-Myc and MyoD contain class I AREs. Class II AREs possess two or more overlapping UUAUUUA(U/A)(U/A) nonamers. Molecules containing this type of AREs include GM-CSF and TNF-α. Class III ARES are less well defined. These U rich regions do not contain an AUUUA motif c-Jun and Myogenin are two well-studied examples of this class. Proteins binding to the AREs may destabilize the messenger, whereas members of the ELAV family, such as HuR, may increase the stability of mRNA. HuR may bind to AREs of all the three classes. Engineering the HuR specific binding sites into the 3' UTR of nucleic acid molecules can lead to HuR binding and thus, stabilization of the message *in vivo.*

Engineering of 3' UTR AU rich elements (AREs) can be used to modulate the stability of an engineered polyribonucleotide. One or more copies of an ARE can be engineered into a polyribonucleotide to modulate the stability of a polyribonucleotide. AREs can be identified, removed or mutated to increase the intracellular stability and thus increase translation and production of the resultant protein. Transfection experiments can be conducted in relevant cell lines, using engineered polyribonucleotides and protein production can be assayed at various time points post-transfection. For example, cells can be transfected with different ARE-engineering molecules and by using an ELISA kit to the relevant protein and assaying protein produced at 6 hours, 12 hours, 24 hours, 48 hours, and 7 days post-transfection.

An untranslated region can comprise any number of nucleotides. An untranslated region can comprise a length of about 1 to about 10 bases or base pairs, about 10 to about 20 bases or base pairs, about 20 to about 50 bases or base pairs, about 50 to about 100 bases or base pairs, about 100 to about 500 bases or base pairs, about 500 to about 1000 bases or base pairs, about 1000 to about 2000 bases or base pairs, about 2000 to about 3000 bases or base pairs, about 3000 to about 4000 bases or base pairs, about 4000 to about 5000 bases or base pairs, about 5000 to about 6000 bases or base pairs, about 6000 to about 7000 bases or base pairs, about 7000 to about 8000 bases or base pairs, about 8000 to about 9000 bases or base pairs, or about 9000 to about 10000 bases or base pairs in length. An untranslated region can comprise a length of for example, at least 1 base or base pair, 2 bases or base pairs, 3 bases or base pairs, 4 bases or base pairs, 5 bases or base pairs, 6 bases or base pairs, 7 bases or base pairs, 8 bases or base pairs, 9 bases or base pairs, 10 bases or base pairs, 20 bases or base pairs, 30 bases or base pairs, 40 bases or base pairs, 50 bases or base pairs, 60 bases or base pairs, 70 bases or base pairs, 80 bases or base pairs, 90 bases or base pairs, 100 bases or base pairs, 200 bases or base pairs, 300 bases or base pairs, 400 bases or base pairs, 500 bases or base pairs, 600 bases or base pairs, 700 bases or base pairs, 800 bases or base pairs, 900 bases or base pairs, 1000 bases or base pairs, 2000 bases or base pairs, 3000 bases or base pairs, 4000 bases or base pairs, 5000 bases or base pairs, 6000 bases or base pairs, 7000 bases or base pairs, 8000 bases or base pairs, 9000 bases or base pairs, or 10000 bases or base pairs in length.

An engineered polyribonucleotide of the disclosure can comprise one or more introns. An intron can comprise any number of modified or unmodified nucleotides. An intron can comprise, for example, at least 1 base or base pair, 50 bases or base pairs, 100 bases or base pairs, 150 bases or base pairs, 200 bases or base pairs, 300 bases or base pairs, 400 bases or base pairs, 500 bases or base pairs, 600 bases or base pairs, 700 bases or base pairs, 800 bases or base pairs, 900 bases or base pairs, 1000 bases or base pairs, 2000 bases or base pairs, 3000 bases or base pairs, 4000 bases or base pairs, or 5000 bases or base pairs. In some cases, an intron can comprise, for example, at most 10000 bases or base pairs, 5000 bases or base pairs, 4000 bases or base pairs, 3000 bases or base pairs, 2000 bases or base pairs, 1000 bases or base pairs, 900 bases or base pairs, 800 bases or base pairs, 700 bases or base pairs, 600 bases or base pairs, 500 bases or base pairs, 400 bases or base pairs, 300 bases or base pairs, 200 bases or base pairs, or 100 bases or base pairs.

In some cases, a percentage of the nucleotides in an intron are modified. For instance, in some cases, fewer than 99%, 95%, 90%, 85%, 80%, 75%, 70%, 65%, 60%, 55%, 50%, 45%, 40%, 35%, 30%, 25%, 20%, 15%, 10%, 5% or 1% of the nucleotides in an intron are modified. In some cases, all of the nucleotides in an intron are modified.

An engineered polyribonucleotide of the disclosure can comprise a polyA sequence. A polyA sequence (e.g., polyA tail) can comprise any number of nucleotides. A polyA sequence can comprise a length of about 1 to about 10 bases or base pairs, about 10 to about 20 bases or base pairs, about 20 to about 50 bases or base pairs, about 50 to about 100 bases or base pairs, about 100 to about 500 bases or base pairs, about 500 to about 1000 bases or base pairs, about 1000 to about 2000 bases or base pairs, about 2000 to about 3000 bases or base pairs, about 3000 to about 4000 bases or base pairs, about 4000 to about 5000 bases or base pairs, about 5000 to about 6000 bases or base pairs, about 6000 to about 7000 bases or base pairs, about 7000 to about 8000 bases or base pairs, about 8000 to about 9000 bases or base pairs, or about 9000 to about 10000 bases or base pairs in length. In some examples, a polyA sequence is at least about 100, 110, 120, 130, 140, 150, 160, 170, 180, 190, or 200 nucleotides in length. A polyA sequence can comprise a length of for example, at least 1 base or base pair, 2 bases or base pairs, 3 bases or base pairs, 4 bases or base pairs, 5 bases or base pairs, 6 bases or base pairs, 7 bases or base pairs, 8 bases or base pairs, 9 bases or base pairs, 10 bases or base pairs, 20 bases or base pairs, 30 bases or base pairs, 40 bases or base pairs, 50 bases or base pairs, 60 bases or base pairs, 70 bases or base pairs, 80 bases or base pairs, 90 bases or base pairs, 100 bases or base pairs, 200 bases or base pairs, 300 bases or base pairs, 400 bases or base pairs, 500 bases or base pairs, 600 bases or base pairs, 700 bases or base pairs, 800 bases or base pairs, 900 bases or base pairs, 1000 bases or base pairs, 2000 bases or base pairs, 3000 bases or base pairs, 4000 bases or base pairs, 5000 bases or base pairs, 6000 bases or base pairs, 7000 bases or base pairs, 8000 bases or base pairs, 9000 bases or base pairs, or 10000 bases or base pairs in length. A polyA sequence can comprise a length of at most 100 bases or base pairs, 90 bases or base pairs, 80 bases or base pairs, 70 bases or base pairs, 60 bases or base pairs, 50 bases or base pairs, 40 bases or base pairs, 30 bases or base pairs, 20 bases or base pairs, 10 bases or base pairs, or 5 bases or base pairs.

In some cases, a percentage of the nucleotides in a poly-A sequence are modified. For instance, in some cases, fewer than 99%, 95%, 90%, 85%, 80%, 75%, 70%, 65%, 60%, 55%, 50%, 45%, 40%, 35%, 30%, 25%, 20%, 15%, 10%, 5% or 1% of the nucleotides in a poly-A sequence are modified. In some cases, all of the nucleotides in a poly-A are modified.

A linker sequence can comprise any number of nucleotides. A linker can be attached to the modified nucleobase at an N-3 or C-5 position. The linker attached to the nucleobase can be diethylene glycol, dipropylene glycol, triethylene glycol, tripropylene glycol, tetraethylene glycol, tetraethylene glycol, divalent alkyl, alkenyl, alkynyl moiety, ester, amide, or an ether moiety. A linker sequence can comprise a length of about 1 to about 10 bases or base pairs, about 10 to about 20 bases or base pairs, about 20 to about 50 bases or base pairs, about 50 to about 100 bases or base pairs, about 100 to about 500 bases or base pairs, about 500 to about 1000 bases or base pairs, about 1000 to about 2000 bases or base pairs, about 2000 to about 3000 bases or base pairs, about 3000 to about 4000 bases or base pairs, about 4000 to about 5000 bases or base pairs, about 5000 to about 6000 bases or base pairs, about 6000 to about 7000 bases or base pairs, about 7000 to about 8000 bases or base pairs, about 8000 to about 9000 bases or base pairs, or about 9000 to about 10000 bases or base pairs in length. A linker sequence can comprise a length of for example, at least 1 base or base pair, 2 bases or base pairs, 3 bases or base pairs, 4 bases or base pairs, 5 bases or base pairs, 6 bases or base pairs, 7 bases or base pairs, 8 bases or base pairs, 9 bases or base pairs, 10 bases or base pairs, 20 bases or base pairs, 30 bases or base pairs, 40 bases or base pairs, 50 bases or base pairs, 60 bases or base pairs, 70 bases or base pairs, 80 bases or base pairs, 90 bases or base pairs, 100 bases or base pairs, 200 bases or base pairs, 300 bases or base pairs, 400 bases or base pairs, 500 bases or base pairs, 600 bases or base pairs, 700 bases or base pairs, 800 bases or base pairs, 900 bases or base pairs, 1000 bases or base pairs, 2000 bases or base pairs, 3000 bases or base pairs, 4000 bases or base pairs, 5000 bases or base pairs, 6000 bases or base pairs, 7000 bases or base pairs, 8000 bases or base pairs, 9000 bases or base pairs, or at least 10000 bases or base pairs in length. A linker at most 10000 bases or base pairs, 5000 bases or base pairs, 4000 bases or base pairs, 3000 bases or base pairs, 2000 bases or base pairs, 1000 bases or base pairs, 900 bases or base pairs, 800 bases or base pairs, 700 bases or base pairs, 600 bases or base pairs, 500 bases or base pairs, 400 bases or base pairs, 300 bases or base pairs, 200 bases or base pairs, or 100 bases or base pairs in length.

In some cases, a percentage of the nucleotides in a linker sequence are modified. For instance, in some cases, fewer than 99%, 95%, 90%, 85%, 80%, 75%, 70%, 65%, 60%, 55%, 50%, 45%, 40%, 35%, 30%, 25%, 20%, 15%, 10%, 5% or 1% of the nucleotides in a linker sequence are modified. In some cases, all of the nucleotides in a linker sequence are modified.

In some cases, an engineered polyribonucleotide can include at least one stop codon before the 3'untranslated region (UTR). In some cases, an engineered polyribonucleotide includes multiple stop codons. The stop codon can be selected from TGA, TAA and TAG. The stop codon may be modified or unmodified. In some cases, the engineered polyribonucleotide includes the stop codon TGA and one additional stop codon. In some cases, the engineered polyribonucleotide includes the addition of the TAA stop codon.

### Encoded Polypeptides

The encoded polypeptides are polymer chains comprised of amino acid residue monomers which are joined together through amide bonds (peptide bonds). The amino acids may be the L-optical isomer or the D-optical isomer. A polypeptide can be a chain of at least three amino acids, peptide-mimetics, a protein, a recombinant protein, an antibody (monoclonal or polyclonal), an antigen, an epitope, an enzyme, a receptors, a vitamin, or a structure analogue or combinations thereof. A polyribonucleotide that is translated within a subject's body can generate an ample supply of specific peptides or proteins within a cell, a tissue, or across many cells and tissues of a subject. In some cases, a polyribonucleotide can be translated *in vivo* within the cytosol of a specific target cell(s) type or target tissue. In some cases, a polyribonucleotide can be translated *in vivo* to provide an ABCA3 protein, a functional fragment thereof, or a protein that is at least 70% homologous to a human ABCA3 protein. In some cases, a polyribonucleotide can be translated *in vivo* in various non-target cell types or target tissue(s). Non-limiting examples of cells that be target or non-target cells include: a) skin cells, e.g.: keratinocytes, melanocytes, urothelial cells; b) neural cells, e.g.: neurons, Schwann cells, oligodentrocytes, astrocytes; c) liver cells, e.g.: hepatocytes; d) intestinal cells, e.g.: globlet cell, enterocytes; e) blood cells; e.g.: lymphoid or myeloid cells. Non-limiting examples of tissues include connective tissue, muscle tissue, nervous tissue, or epithelial tissue. In some cases, a target cell or a target tissue is a cancerous cell, tissue, or organ.

A polynucleotide sequence can be derived from one or more species. For example, a polynucleotide sequence can be derived from a human (*Homo sapiens*)*,* a mouse (e.g., *Mus musculus*), a rat (e.g., *Rattus norvegicus* or *Rattus rattus*), a camel (e.g., *Camelus dromedarius* or *Camelus bactrianus*), a llama (*Lama vicugna*), or any other suitable creature. A polynucleotide sequence can be a chimeric combination of the sequence of one or more species.

In some cases, the endogenous translational machinery can add a post-translational modification to the encoded peptide. A post-translational modification can involve the addition of hydrophobic groups that can target the polypeptide for membrane localization, the addition of cofactors for increased enzymatic activity, or the addition of smaller chemical groups. The encoded polypeptide can also be post-translationally modified to receive the addition of other peptides or protein moieties. For instance, ubiquitination can lead to the covalent linkage of ubiquitin to the encoded polypeptide, SUMOylation can lead to the covalent linkage of SUMO (Small Ubiquitin-related MOdifier) to the encoded polypeptide, ISGylation can lead to the covalent linkage of ISG15 (Interferon-Stimulate Gene 15).

In some cases, the encoded polypeptide can be post-translationally modified to undergo other types of structural changes. For instance, the encoded polypeptide can be proteolytically cleaved, and one or more proteolytic fragments can modulate the activity of an intracellular pathway. The encoded polypeptide can be folded intracellularly. In some cases, the encoded polypeptide is folded in the presence of co-factors and molecular chaperones. A folded polypeptide can have a secondary structure and a tertiary structure. A folded polypeptide can associate with other folded peptides to form a quaternary structure. A folded-peptide can folio a functional multi-subunit complex, such as an antibody molecule, which has a tetrameric quaternary structure. Various polypeptides that form classes or isotypes of antibodies can be expressed from a polyribonucleotide.

The encoded polypeptide can be post-translationally modified to change the chemical nature of the encoded amino acids. For instance, the encoded polypeptide can undergo post-translational citrullination or deimination, the conversion of arginine to citrulline. The encoded polypeptide can undergo post-translation deamidation; the conversion of glutamine to glutamic acid or asparagine to aspartic acid. The encoded polypeptide can undergo eliminylation, the conversion of an alkene by beta-elimination of phosphothreonine and phosphoserine, or dehydration of threonine and serine, as well as by decarboxylation of cysteine. The encoded peptide can also undergo carbamylation, the conversion of lysine to homocitrulline. An encoded peptide can also undergo racemization, for example, racemization of proline by prolyl isomerase or racemization of serine by protein-serine epimerase.

The activity of a plurality of biomolecules can be modulated by a molecule encoded by a polyribonucleotide. Non-limiting examples of molecules whose activities can be modulated by an encoded polynucleotide include: amino acids, peptides, peptide-mimetics, proteins, recombinant proteins antibodies (monoclonal or polyclonal), antibody fragments, antigens, epitopes, carbohydrates, lipids, fatty acids, enzymes, natural products, nucleic acids (including DNA, RNA, nucleosides, nucleotides, structure analogues or combinations thereof), nutrients, receptors, and vitamins.

Non-limiting examples of nucleotide sequences that can be a part of a polynucleotide of the disclosure are disclosed in **TABLE 6**.

| TABLE 6 | |
|---|---|
| Name | Sequence |
| ABCA3 *Homo sapiens* | NC_000016.10 |
| ABCA3 *mus musculus* | NC_000083.6 |
| ABCA3 *Rattus norvegicus* | NC_005109.4 |
| abcA3 *Dictyostelium discoideum* | NC_007092.3 |
| ABCA3 *Bos taurus* | AC_000182.1 |
| ABCA3 *Pan troglodytes* | NC_006483.3 |
| ABCA3 *Canis lupus familiaris* | NC_006588.3 |
| ABCA3 *Gallus gallus* | NC_006101.3 |
| ABCA3 *Leishmania infantum* | NC_009395.2 |
| ABCA3 *Xenopus tropicalis* | NW_004668244.1 |

A polypeptide sequence can share a % homology to an amino acid sequence of an endogenous polypeptide. A polypeptide sequence can share at most 10% homology, at most 20% homology, at most 30% homology, at most 40% homology, at most 50% homology, at most 60% homology, at most 70% homology, at most 80% homology, at most 90% homology, or at most 99% homology with an amino acid sequence of an endogenous polypeptide. Various methods and software programs can be used to determine the homology between two or more peptides, such as NCBI BLAST, Clustal W, MAFFT, Clustal Omega, AlignMe, Praline, or another suitable method or algorithm.

### Immunogenicity

Many pharmaceutical agents, including compositions comprising molecules of various sizes (polynucleotides, proteins, or enzymes) can trigger an immune response when administered to a subject. In many cases, the immune system recognizes the composition as a foreign body and neutralizes its pharmaceutical action. A polyribonucleotide and a composition of the present disclosure can have low immunogenicity or be non-immunogenic, thereby triggering a small response by the immune system, or not triggering any immune response at all.

The immunogenicity can also be determined by measurement of, for example, the TNF-α and IL-8 levels and the binding capacity to TLR-3, TLR-7, TLR-8 and helicase RIG-1. In order thereby to establish whether a polyribonucleotide has a desired low immunogenicity, the quantity of one or more of the factors can be measured after administration of the polyribonucleotide to a subject. The immunogenicity of a polypeptide can be determined in relation to an increase in the number of white blood cells upon administration of the polypeptide to the subject. In some cases, upon administration of the composition to the subject, the subject exhibits an increase in the number of white blood cells that is less than 90%, less than 80%, less than 70%, less than 60%, less than 50%, less than 40%, less than 30%, less than 20%, or less than 10%. A polyribonucleotide of the disclosure can trigger minimum or insignificant inflammatory or immunological reactions.

For the determination of the immunogenicity of a polyribonucleotide, various methods can be used. A very suitable method is the determination of inflammatory markers in cells or a simple white cell blood count, as a reaction to the administration of the polyribonucleotide. Such a method is described in the examples. Cytokines which are associated with inflammation, such as, for example TNF-α, IFN-α, IFN-β, IL-8, IL-6, and/or IL-12, can be measured. The expression of dendritic cell activation markers can also be used for the estimation of immunogenicity. A further indication of an immunological reaction can be the detection of binding to the Toll-like receptors TLR-3, TLR-7 and TLR-8 and to helicase RIG-1.

The immunogenicity of a polyribonucleotide can be determined as an overall increase in the level of inflammatory marker or white blood cell count as compared to a level prior to the administration of the polyribonucleotide. For instance, an engineered polyribonucleotide that is unmodified or modified can be administered to cells, or to a subject, and the secretion of inflammatory markers in a defined time interval as a reaction to the administration of the polyribonucleotide can be measured.

### Compositions

"Naked" polynucleotide compositions can be successfully administered to a subject, and uptaken by a subject's cell, without the aid of carriers, stabilizers, diluents, dispersing agents, suspending agents, thickening agents, and/or excipients (Wolff et al. 1990, Science, 247, 1465-1468). However, in many instances, encapsulation of polynucleotides with formulations that can increase the endocytotic uptake can increase the effectiveness of a composition of the disclosure.

Another technical challenge underlying the delivery of polyribonucleotides to multicellular organisms is to identify a composition that provides a high efficiency delivery of polyribonucleotides that are translated within a cell or a tissue of a subject. It has been recognized that administration of naked nucleic acids may be highly inefficient and may not provide a suitable approach for administration of a polynucleotide to a multicellular organism.

To solve this challenge, a composition comprising an engineered polyribonucleotide can be encapsulated or formulated with a pharmaceutical carrier. The formulation may be, but is not limited to, nanoparticles, poly(lactic-co-glycolic acid)(PLGA) microspheres, lipidoids, lipoplex, liposome, polymers, carbohydrates (including simple sugars), cationic lipids, fibrin gel, fibrin hydrogel, fibrin glue, fibrin sealant, fibrinogen, thrombin, rapidly eliminated lipid nanoparticles (reLNPs) and combinations thereof. A composition comprising an engineered polyribonucleotide disclosed herein can comprise from about 1% to about 99% weight by volume of a carrier system. The amount of carrier present in a carrier system is based upon several different factors or choices made by the formulator, for example, the final concentration of the polyribonucleotide and the amount of solubilizing agent. Various carriers have been shown useful in delivery of different classes of therapeutic agents. Among these carriers, biodegradable nanoparticles formulated from biocompatible polymers poly(D, L-lactide-co-glycolide) (PLGA) and polylactide (PLA) have shown the potential for sustained intracellular delivery of different therapeutic agents.

The loading weight percent of the engineered polynucleotide in a composition may be at least 0.05%, 0.1%, 0.2%, 0.3%, 0.4%, 0.5%, 1%, 2%, 4%, 5%, 6%, 7%, 8%, 9%, or 10 %. The encapsulation efficiency of the modified mRNA in the PLGA microsphere may be at least 50%, at least 70%, at least 90%, at least 95%, at least 96%, at least 97%, at least 98%, or at least 99%.

The present disclosure describes nanoparticles, oligomers, polymers or lipidoids comprising oligo(alkylene amines) containing alternating, non-identical alkylene amine units which are useful for delivering a polynucleotide, in some cases an engineered polyribonucleotides, into a cell or into a tissue. A composition disclosed herein can be stable for at least about 1 minute, 5 minutes, 10 minutes, 30 minutes, 1 hour, 2 hours, 3 hours, 4 hours, 5 hours, 6 hours, 12 hours, 1 day, 2 days, 3 days, 4 days, 5 days, 6 days, 7 days, 8 days, 9 days, 10 days, 2 weeks, 4 weeks, 6 weeks, 8 weeks, 10 weeks, 12 weeks, 3 months, 4 months, 5 months, 6 months, 7 months, 8 months, 9 months, 10 months, 11 months, or one year. A formulation disclosed herein can be stable, for example, at a temperature of at least about 0°C, 5°C, 10°C, 15°C, 20°C, 25°C, 30°C, 35°C, 40°C, 45°C, 50°C, 60°C, 70°C, or 80°C. A composition of the disclosure can have a desired density. The density of a composition can improve a property of the composition, such as the rheology of the composition.

### Nanoparticles

The present disclosure also provides nanoparticle based formulations of engineered polyribonucleotides that are able to translocate following administration to a subject. In some instances, the administration is pulmonary and the engineered polyribonucleotides can move intact either actively or passively from the site of administration to the systemic blood supply and subsequently to be deposited in different cells or tissues, such as, e.g., the breast. This translocation of the nanoparticle comprising an engineered polyribonucleotide encoding a therapeutic protein, such as, e.g., ABCA3 or a functional fragment thereof, constitutes non-invasive systemic delivery of an active pharmaceutical ingredient beyond the lung to result in the production of a functional protein to systemically accessible non-lung cells or tissues.

A nanoparticle can be a particle of particle size from about 10 nanometers (nm) to 5000 nm, 10 nm to 1000 nm, or 60 nm to 500 nm, or 70 nm to 300 nm. In some examples, a nanoparticle has a particle size from about 60 nm to 225 nm. The nanoparticle can include an encapsulating agent (e.g., coating) that encapsulates one or more polyribonucleotides, which may be engineered polyribonucleotides. The nanoparticle can include engineered and/or naturally occurring polyribonucleotides. The encapsulating agent can be a polymeric material, such as PEI or PEG.

A lipidoid or lipid nanoparticle which may be used as a delivery agent may include a lipid which may be selected from the group consisting of C12-200, MD1, 98N12-5, DLin-DMA, DLin-K-DMA, DLin-KC2-DMA, DLin-MC3-DMA, PLGA, PEG, PEG-DMG, PEGylated lipids and analogues thereof. A suitable nanoparticle can comprise one or more lipids in various ratios. For example, a composition of the disclosure can comprise a 40:30:25:5 ratio of C12-200:DOPE:Cholesterol:DMG-PEG2000 or a 40:20:35:5 ratio of HGT5001:DOPE:Cholesterol: DMG-PEG2000. A nanoparticle can include at least 1, 2, 3, 4, 5, 6, 7, 8, 9, 10 lipids or another suitable number of lipids. A nanoparticle can be formed of any suitable ratio of lipids selected from the group consisting of C12-200, MD1, 98N12-5, DLin-DMA, DLin-K-DMA, DLin-KC2-DMA, DLin-MC3-DMA, PLGA, PEG, PEG-DMG.

The mean size of the nanoparticle formulation may comprise the modified mRNA between 60 nanometers (nm) and 225 nm. The polydispersity index PDI of the nanoparticle formulation comprising the modified mRNA can be between 0.03 and 0.15. The zeta potential of the nanoparticle formulation may be from -10 to +10 at a pH of 7.4. The formulations of modified mRNA may comprise a fusogenic lipid, cholesterol and a PEG lipid. The formulation may have a molar ratio 50:10:38.5:1.5-3.0 (cationic lipid: fusogenic lipid: cholesterol: polyethylene glycol (PEG) lipid). The PEG lipid may be selected from, but is not limited to PEG-c-DOMG, PEG-DMG. The fusogenic lipid may be DSPC. A lipid nanoparticle of the present disclosure can be formulated in a sealent such as, but not limited to, a fibrin sealant.

### Oligo(alkylene amine groups)

It has also been recognized that although encapsulation of polynucleotides with some formulations can increase the endocytotic uptake of a composition, the polynucleotide that is taken up by a cell may not be effectively translated within the cell. Some formulations may be effectively used for plasmid DNA and/or siRNA delivery, while not practical for use in the delivery of polyribonucleotides. The present disclosure provides formulations that can be employed for effective delivery and translation of polyribonucleotide compositions to a subject.

A composition of the disclosure can be designed to provide a polyribonucleotide that is effectively translated within a cell. A composition of the disclosure can comprise an arrangement of alkylene amine units of alternating length in groups of three or more units and containing an ethyleneamine unit in compositions for transfecting a cell with any polynucleotide, such as an engineered polyribonucleotide. A composition of the disclosure can provide a more efficacious delivery of a polyribonucleotide to a cell than analogous arrangements of alkylene amine units of non-alternating length.

Oligomers, polymers or lipidoids can be provided which share a common structural entity which is illustrated in formula (I):

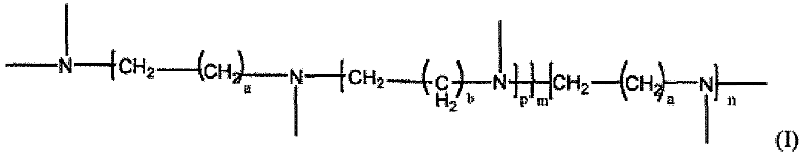

A composition of the disclosure can comprise an oligo(alkylene amine) that is selected from:
a) an oligomer or polymer comprising a plurality of groups of formula (II) as a side chain and/or as a terminal group: 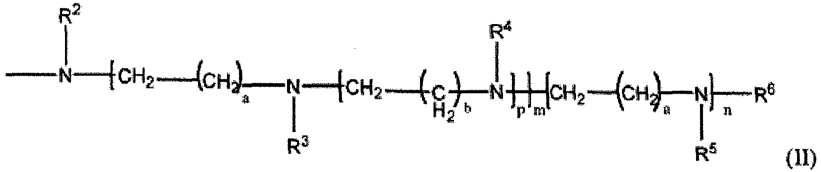 wherein the variables a, b, p, m, n, and R² to R⁶ are defined as follows, independently for each group of formula (II) in a plurality of such groups:
   a is 1 and b is an integer of 2 to 4; or a is an integer of 2 to 4 and b is 1,
   p is 1 or 2,
   m is 1 or 2; n is 0 or 1 and m + n is ≥ 2; and
   R² to R⁵ are, independently of each other, selected from hydrogen; a group -CH₂-CH(OH)-R⁷, -CH(R⁷)-CH₂-OH,-CH₂-CH₂-C=O)-O-R⁷, -CH₂-CH₂-(C=O)-NH-R⁷, or -CH₂-R⁷ wherein R⁷ is selected from C3-C18 alkyl or C3-C18 alkenyl having one C-C double bond; a protecting group for an amino group; and a poly(ethylene glycol) chain;
   R⁶ is selected from hydrogen; a group -CH2-CH(OH)-R⁷, -CH(R⁷)-CH₂-OH, -CH₂-CH₂-(C=O)-O-R⁷, -CH₂-CH₂-(C=O)-NH-R⁷, or -CH₂-R⁷ wherein R⁷ is selected from C3-C18 alkyl or C3-C18 alkenyl having one C-C double bond; a protecting group for an amino group; -C(NH)-NH; a poly(ethylene glycol) chain; and a receptor ligand,
   and wherein one or more of the nitrogen atoms indicated in formula (II) may be protonated to provide a cationic group of formula (II).
b) an oligomer or polymer comprising a plurality of groups of formula (III) as repeating units: 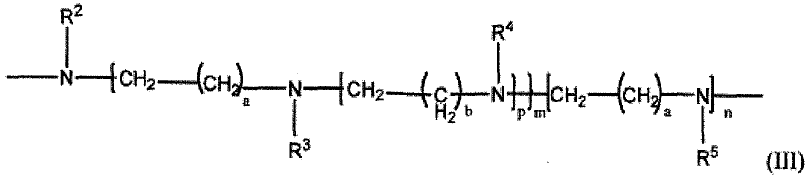 wherein the variables a, b, p, m, n, and R² to R⁵ are defined as follows, independently for each group of formula (III) in a plurality of such groups:
   a is 1 and b is an integer of 2 to 4; or a is an integer of 2 to 4 and b is 1,
   p is 1 or 2,
   m is 1 or 2; n is 0 or 1 and m + n is ≥ 2; and
   R² to R⁵ are, independently of each other, selected from hydrogen; a group -CH₂-CH(OH)-R⁷, -CH(R⁷)-CH₂-OH, -CH₂-CH₂-(C=O)-O-R⁷, -CH₂-CH₂-(C=O)-NH-R⁷, -CH₂-R⁷ or -CH₂- wherein R⁷ is selected from C3-C18 alkyl or C3-C18 alkenyl having one C-C double bond; a protecting group for an amino group; and a poly(ethylene glycol) chain;
   and wherein one or more of the nitrogen atoms indicated in formula (III) may be protonated to provide a cationic group of formula (III).
c) a lipidoid having the structure of formula (IV): 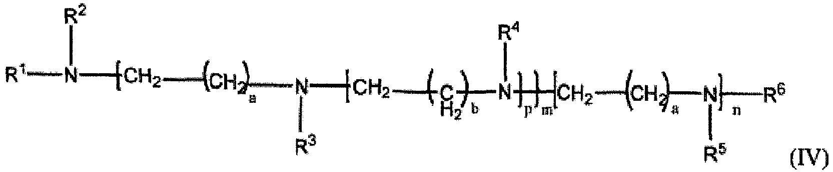 wherein the variables a, b, p, m, n, and R² to R⁶ are defined as follows:
   a is 1 and b is an integer of 2 to 4; or a is an integer of 2 to 4 and b is 1,
   p is 1 or 2,
   m is 1 or 2; n is 0 or 1 and m + n is ≥ 2; and
   R² to R⁶ are, independently of each other, selected from hydrogen; a group -CH₂-CH(OH)-R⁷, -CH(R⁷)-CH₂-OH, -CH₂-CH₂-(C=O)-O-R⁷, -CH₂-CH₂-(C=O)-NH-R⁷, or -CH₂-R⁷ wherein R⁷ is selected from C3-C18 alkyl or C3-C18 alkenyl having one C-C double bond; a protecting group for an amino group; and a poly(ethylene glycol) chain; and a receptor ligand; provided that at least two residues among R¹ to R⁶ are a group -CH₂-CH(OH)-R⁷, -CH(R⁷)-CH₂-OH, -CH₂-CH₂-(C=O)-O-R⁷, -CH₂-CH₂-(C=O)-NH-R⁷, or -CH₂-R⁷ wherein R⁷ is selected from C3-C18 alkyl or C3-C18 alkenyl having one C-C double bond;
   and wherein one or more of the nitrogen atoms indicated in formula (IV) may be protonated to provide a cationic group of formula (IV).

Non-limiting examples of alkenyl and alkenylene groups include straight, branched, and cyclic alkenyl groups. The olefin or olefins of an alkenyl group can be, for example, *E,* Z, *cis, trans,* terminal, or exo-methylene. An alkenylene group can be, for example, a C₂, C₃, C₄, C₅, C₆, C₇, C₈, C₉, C₁₀, C_{11,} C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂, C₃₃, C₃₄, C₃₅, C₃₆, C₃₇, C₃₈, C₃₉, C₄₀, C₄₁, C₄₂, C₄₃, C₄₄, C₄₅, C₄₆, C₄₇, C₄₈, C₄₉, or C₅₀ group that is substituted or unsubstituted.

The oligo(alkylene amine) structures of formulae (II), (III) and (IV) are characterized in that they can combine shorter (also referred to for illustration as "S") ethylene amine units (i.e., a or b is 1) with longer (also referred to for illustration as "L") alkylene amine units (i.e., the other one of a or b is an integer of 2 to 4) in an alternating manner. Such an arrangement of the protonatable units can provide advantages in terms of the suitability of the resulting group to provide a vehicle for delivering polyribonucleotides into a cell.

A composition of the disclosure can comprise a plurality of oligo(alkylene amine) groups of formula (II) as a side chain or as a terminal group:

-NR² {CH₂-(CH₂)ₐ-NR³-[CH₂-(CH₂)_{b}-NR⁴]ₚ}ₘ-[CH₂-(CH₂)ₐ-NR⁵]ₙ-R⁶ (II),

wherein the variables a, b, p, m, n, and R² to R⁶ are defined as follows, independently for each group of formula (II) in a plurality of such groups:
a is 1 and b is an integer of 2 to 4; or a is an integer of 2 to 4 and b is 1,
p is 1 or 2,
m is 1 or 2; n is 0 or 1 and m + n is ≥ 2; and
R² to R⁵ are, independently of each other, selected from hydrogen; a group -CH₂-CH(OH)-R⁷, -CH(R⁷)-CH₂-OH, -CH₂-CH₂-(C=O)-O-R⁷, -CH₂-CH₂-(C=O)-NH-R⁷, or -CH₂-R⁷ wherein R⁷ is selected from C3-C18 alkyl or C3-C18 alkenyl having one C-C double bond; a protecting group for an amino group; -C(NH)-NH₂-; and a poly(ethylene glycol) chain;
R⁶ is selected from hydrogen; a group -CH₂-CH(OH)-R⁷, -CH(R⁷)-CH₂-OH, -CH₂-CH₂-(C=O)-O-R⁷, -CH₂-CH₂-(C=O)-NH-R⁷, or -CH₂-R⁷ wherein R⁷ is selected from C3-C16 alkyl or C3-C16 alkenyl having one C-C double bond; a protecting group for an amino group; -C(NH)-NH; a poly(ethylene glycol) chain; and a receptor ligand.

In some cases, R² to R⁵ are hydrogen and R⁶ is selected from hydrogen, a protecting group for an amino group; -C(NH)-NH₂ and a poly(ethylene glycol) chain. In some cases, R² to R⁶ are hydrogen. In some cases, R⁷ is selected from C8-C18 alkyl or C8-C18 alkenyl having one C-C double bond, or from C8-C12 alkyl or C8-C12 alkenyl having one C-C double bond, or from C10-C12 alkyl or C10-C12 alkenyl having one C-C double bond. A composition of the disclosure can comprise one, or multiple alkylene groups of formulas (II)-(IV).

In some cases, the oligomers or polymers which can be used in the compositions in accordance with the present disclosure comprise a plurality of oligo (alkylene amine) groups of formula (III) as repeating units:

NR² {CH₂-(CH₂)ₐ-NR³-[CH₂-(CH₂)_{b}-NR⁴]ₚ}ₘ-[CH₂-(CH₂)ₐ-NR⁵]ₙ- (III)

wherein the variables a, b, p, m, n, and R² to R⁵ are defined as follows, independently for each group of formula (III) in a plurality of such groups:
a is 1 and b is an integer of 2 to 4; or a is an integer of 2 to 4 and b is 1,
p is 1 or 2,
m is 1 or 2; n is 0 or 1 and m + n is ≥ 2; and
R² to R⁵ are, independently of each other, selected from hydrogen; a group -CH₂-CH(OH)-R⁷, -CH(R⁷)-CH₂-OH, -CH₂-CH₂-(C=O)-O-R⁷, -CH₂-CH₂-(C=O)-NH-R⁷, -CH₂-R⁷ or -CH₂- wherein R⁷ is selected from C3-C18 alkyl or C3-C18 alkenyl having one C-C double bond; a protecting group for an amino group; -C(NH) -NH₂; a poly(ethylene glycol) chain; and endosomal escape effector and a receptor ligand. In some cases, R² to R⁵ are hydrogen. In some cases, R⁷ is selected from C8-C18 alkyl or C8-C18 alkenyl having one C-C. R⁷ may be selected from C8-C12 alkyl or C8-C12 alkenyl having one C-C. As an alternative, R⁷ may be selected from C10-C12 alkyl or C10-C12 alkenyl having one C-C.

One or more of the nitrogen atoms indicated in formula (III) may be protonated to provide a cationic group of formula (III).

Optionally, the oligomers or polymers which comprise a plurality of groups of formula (III) as repeating units can comprise, in addition, one or more oligo(alkylene amine) group(s) of formula (II) as a side chain and/or as a terminal group.

In a plurality of groups of formula (III) as repeating units, two, three or more of the groups of formula (III) can be contained in the oligomers or polymers. Generally, substances comprising 2 to 9 repeating units are referred to herein as oligomers, those comprising 10 and more repeating units as polymers. Thus, in the polymers containing a plurality of groups of formula (III) as repeating units, 10 or more groups of formula (III) may be present. It will be understood that the groups of formula (III) can have the same structure within a polymer or oligomer, or can have two or more different structures within the scope of formula (III). In some cases, the oligomers or polymers containing a plurality of groups of formula (III) as repeating units can be provided in the form of a library of sequence defined polymers which are prepared from different groups of formula (III) in a controlled, stepwise polymerization.

In line with formulae (II) and (III) above, an alkylene amine unit may be repeated once in an alternating chain such that oligo(alkylene amine) moieties of the type -S-L-L-S- or -L-S-S-L may result, wherein S represents a shorter ethylene amine unit, and L represents a longer alkylene amine unit. In some cases, groups of formula (II) and (III) are those wherein no repetition occurs, i.e., wherein p is 1, such that the shorter or longer units do not appear in pairs. The group of formula (II) can be an oligo(alkylene amine) group of formula (IIa) and the group of formula (III) can be an oligo(alkylene amine) group of (IIIa):

-NR²{CH₂-(CH₂)ₐ-NR³-CH₂-(CH₂)_{b}-NR⁴}ₘ-[CH2-(CH₂)ₐ-NR⁵]ₙ-R⁶ (IIa),

wherein a, b, m, n, and R² to R⁶ are defined as in formula (II), and wherein one or more of the nitrogen atoms indicated in formula (IIa) may be protonated to provide a cationic oligomer or polymer structure;

-NR²{CH₂-(CH₂)ₐ-NR³-CH₂-(CH₂)_{b}-NR⁴}ₘ-[CH₂-(CH₂)ₐNR⁵]ₙ- (IIIa),

wherein a, b, m, n, and R² to R⁵ are defined as in formula (III), and wherein one or more of the nitrogen atoms indicated in formula (Ilia) can be protonated to provide a cationic oligomer or polymer structure.

Moreover, in some cases, the oligo(alkylene amine) group of formulae (II) and (III) can have an n of 1. In some cases, m is 1 and n is 1. In some cases, the group of formula (II) is an oligo(alkylene amine) group of formula (IIb), and the group of formula (III) is an oligo(alkylene amine) group of formula (IIIb):

-NR²-CH₂-(CH₂)ₐ-NR³-CH₂-(CH₂)_{b}-NR⁴-CH₂-(CH₂)ₐ-(NR⁵)-R⁶ (IIb),

wherein a, b, and R² to R⁶ are defined as in formula (II), and wherein one or more of the nitrogen atoms indicated in formula (IIb) can be protonated to provide a cationic oligomer or polymer structure;

-NR²-CH₂-(CH₂)ₐ-NR³-CH₂-(CH₂)_{b}-NR⁴-CH₂-(CH₂)ₐ-NR⁵- (IIIb)

wherein a, b, and R² to R⁵ are defined as in formula (III) and wherein one or more of the nitrogen atoms indicated in formula (IIIb) can be protonated to provide a cationic oligomer or polymer structure.

With respect to the length of the alkylene amine units in the oligo(alkylene amine) groups of formula (II), (IIa), (IIb) and (III), (IIIa), (IIIb), one of the alternating units can be an ethylene amine unit (i.e., either a or b is 1). The other alternating unit can be a propylene amine unit, a butylene amine unit or a pentylene amine unit (i.e., the other one of a or b can be an integer from 2 to 4. In some cases, the other of a or b can be 2 or 3, and in some cases, a is 1 and b is 2, or a is 2 and b is 1. In some cases, an oligo(alkylene amine) group of formula (IIc) is employed instead of or in addition to group (II), and/or an oligo(alkylene amine) group of formula (IIIc) is employed instead of or in addition to group (III). The formulae of group (IIc) and group (IIIc) are as follows:

-NR²-CH₂-CH₂-NR³-CH₂-CH₂-CH₂-NR⁴-CH₂-CH₂-NR⁵- R⁶ (IIc),

wherein R² to R⁶ are as defined in formula (II), and wherein R² to R⁶ are hydrogen, and wherein one or more of the nitrogen atoms indicated in formula (IIc) can be protonated to provide a cationic oligomer or polymer structure;

-NR²-CH₂-CH₂-NR³-CH₂-CH₂-CH₂-NR⁴-CH₂-CH₂-NR⁵- (IIIc),

wherein R² to R⁵ are as defined in formula (III), and wherein one or more of the nitrogen atoms indicated in formula (IIIc) can be protonated to provide a cationic oligomer or polymer structure.

In some cases, the groups R² to R⁶ in formula (II), (IIa), (IIb) and (IIc) or the groups R² to R⁵ in formula (III), (IIIa), (IIIb) and (IIIc) can be protecting group for an amino group. Non-limiting examples of protecting groups include t-butoxycarbonyl (Boc), 9-fluorenylmethoxycarbonyl (Fmoc), or carbobenzyloxy (Cbz).

In some cases, the groups R¹ to R⁶ in formula (II), (IIa), (IIb) and (IIIc) or the groups R² to R⁵ in formula (III), (IIIa), (IIIb) and (IIIc) are a receptor ligand, such as the receptor ligands described in Philipp and Wagner in "Gene and Cell Therapy - Therapeutic Mechanisms and Strategy", 3rd Edition, Chapter 15, CRC Press, Taylor & Francis Group LLC, Boca Raton 2009. Examples of receptor ligands that target the lung tissue are described in Pfeifer et al. 2010, Ther. Deliv. 1 (1): 133-48. Receptor ligands can include synthetic cyclic or linear peptides such as derived from screening peptide libraries for binding to a particular cell surface structure or particular cell type, cyclic or linear RGD peptides, synthetic or natural carbohydrates such as sialic acid, galactose or mannose or synthetic ligands derived from reacting a carbohydrate for example with a peptide, antibodies specifically recognizing cell surface structures, folic acid, epidermal growth factor and peptides derived thereof, transferrin, anti-transferrin receptor antibodies, nanobodies and antibody fragments, approved drugs that may bind to cell surface molecules (e.g., cell surface receptors), etc.

As far as any of the groups R¹ to R⁶ in formula (II), (IIa), (IIb) and (IIc) or the groups R² to R⁵ in formula (III), (IIIa), (IIIb) and (IIIc) are a poly(ethylene glycol) chain, the molecular weight of the poly(ethylene glycol) chain can be from about 100 g/mol to 20,000 g/mol, from about 1,000 g/mol to 10,000 g/mol or from about 1,000 g/mol to 5,000 g/mol.

In some cases, group (II) can be an oligo(alkylene amine) group of formula (IId):

-NH-CH₂-CH₂-NH-CH₂-CH₂-CH₂-NH-CH₂-CH₂-NH-H (IId),

wherein one or more of the nitrogen atoms indicated in formula (IId) may be protonated to provide a cationic polymer or dendrimer structure. In some cases, group (III) is an oligo(alkylene amine) group of formula (IIId):

-NH-CH₂-CH₂-NH-CH₂-CH₂-CH₂-NH-CH₂-CH₂-NH- (IIId)

wherein one or more of the nitrogen atoms indicated in formula (IIId) may be protonated to provide a cationic polymer or dendrimer structure.

### Lipidoids

An engineered polyribonucleotide can be encapsulated in a lipidoid formulation. A lipidoid formulation can be any material that has characteristics of a lipid, such as fats, waxes, sterols, fat-soluble vitamins (such as vitamins A, D, E, and K), monoglycerides, diglycerides, triglycerides, phospholipids, and others. For example, a lipid or lipidoid formulation can include lipids such as cholesterol, DOPE, DOPC or DSPC which are referred to as helper lipids in the scientific literature, and/or PEGylated lipids or any other lipid useful for preparing lipoplexes. The formulation comprising the engineered polyribonucleotide may be a nanoparticle which may comprise at least one lipid. A lipidoid formulation can be a lipid nanoparticle. The lipid may be selected from, but is not limited to, DOPE, DOPC, DSPC, cholesterol, DLin-DMA, DLin-K-DMA, 98N12-5, C12-200, DLin-MC3-DMA, DLin-KC2-DMA, DODMA, PLGA, PEG, PEG-DMG and PEGylated lipids. In another aspect, the lipid may be a cationic lipid such as, but not limited to, DLin-DMA, DLin-D-DMA, DLin-MC3-DMA, DLin-KC2-DMA and DODMA.

The composition containing a lipidoid may be about 40-60% lipidoid, about 40-60 % cholesterol, and about 5-20% PEG-lipid (in percent by weight, based on the total weight of the composition). The composition containing a lipidoid may be about 50-60% lipidoid, about 40- 50 % cholesterol, and about 5-10% PEG-lipid. The composition containing a lipidoid may be about 50-75% lipidoid, about 20-40% cholesterol, and about 1-10% PEG-lipid. The composition containing a lipidoid may be about 60-70% lipidoid, about 25-35% cholesterol, and about 5-10% PEG-lipid. The composition may be provided with techniques described in, for example, Akinc et al, 2007, Nat Biotech, 26, 561-569; Akinc et al, 2009, Mol Ther, 17, 872-9; Love et al, 2010, PNAS, 107, 1864-9; US 8,450,298, O2006/138380). RNA/lipidoid complexes may form particles that are useful in the delivery of RNA, such as single-stranded RNAs or mRNAs, into cells.

A composition of the disclosure cab be an engineered polyribonucleotide encapsulated by a lipidoid of formula (IV)

R¹-NR²{CH₂-(CH₂)ₐ-NR³-[CH₂-(CH₂)_{b}-NR⁴]ₚ}ₘ-[CH₂-(CH₂)ₐ-NR⁵]ₙ-R⁶ (IV),

wherein the variables a, b, p, m, n and R1 to R6 are defined as follows:
a is 1 and b is an integer of 2 to 4; or a is an integer of 2 to 4 and b is 1,
p is 1 or 2,
m is 1 or 2; n is 0 or 1 and m + n is ≥ 2; and
R¹ to R⁶ are independently of each other selected from hydrogen; a group -CH₂-CH(OH)-R⁷,-CH(R⁷)-CH₂-OH, -CH₂-CH₂-(C=O)-O-R⁷, -CH₂-CH₂-(C=O)-NH-R⁷ or -CH₂-R⁷ wherein R⁷ is selected from C3-C18 alkyl or C3-C18 alkenyl having one C-C double bond; a protecting group for an amino group; -C(NH) -NH₂; a poly(ethylene glycol) chain; and a receptor ligand; provided that at least two residues among R¹ to R⁶ are a group -CH₂-CH(OH)-R⁷, -CH(R⁷)-CH₂-OH, -CH₂-CH₂-(C=O)-O-R⁷, -CH₂-CH₂-(C=O) -NH-R⁷ or -CH₂-R⁷ wherein R⁷ is selected from C3-C18 alkyl or C3-C18 alkenyl having one C-C double bond.

In some cases, R¹ to R⁶ are independently selected from hydrogen; a group -CH₂-C(OH)H-R⁷ or -CH(R⁷)-CH₂-OH, wherein R⁷ is selected from C3-C18 alkyl or C3-C18 alkenyl having one C-C double bond; a protecting group for an amino group; and a poly(ethylene glycol) chain; provided that at least two residues among R¹ to R⁶ are a group -CH2-C(OH)H-R⁷ or -CH(R⁷)-CH₂ OH, wherein R⁷ is selected from C3-C18 alkyl or C3-C18 alkenyl having one C-C double bond. In some cases, R¹ to R⁶ are independently selected from hydrogen; and a group -CH₂-CH(OH)-R⁷ or -CH(R⁷)-CH₂-OH wherein R⁷ is selected from C3-C16 alkyl or C3-C16 alkenyl having one C-C double bond; provided that at least two residues among R¹ to R⁶ are a group -CH₂-CH(OH)-R⁷ or -CH(R⁷)-CH₂-OH, wherein R⁷ is selected from C3-C18 alkyl or C3-C18 alkenyl having one C-C double bond. In some cases, R¹ and R⁶ are independently selected from hydrogen; and a group - CH₂-CH(OH)-R⁷ or -CH(R⁷)-CH2-OH wherein R⁷ is selected from C3-C18 alkyl or C3-C18 alkenyl having one C-C double bond; and R² to R⁵ are all a group -CH2-CH(OH)-R⁷ or -CH(R⁷)-CH₂-OH wherein R⁷ is selected from C3-C18 alkyl or C3-C18 alkenyl having one C-C double bond. In some cases, R⁷ is selected from C8-C16 alkyl or C8-C18 alkenyl having one C-C double bond, or from C8-C12 alkyl or C8-C12 alkenyl having one C-C double bond, or from C10-C12 alkyl or C10-C12 alkenyl having one C-C double bond.

One or more of the nitrogen atoms indicated in formula (IV) may be protonated to provide a cationic lipidoid of formula (IV).

In line with formula (IV) above, an alkylene amine unit may be repeated once in an alternating chain such that oligo(alkylene amine) moieties of the type -S-L-L-S- or -L-S-S-L-may result, wherein S represents a shorter ethylene amine unit, and L represents a longer alkylene amine unit. In some cases, a lipidoid of formula (IV) is one wherein no repetition occurs, i.e., wherein p is 1, such that the shorter or longer units do not appear in pairs. The lipidoid of formula (IV) can be a lipidoid of (IVa):

R¹-NR² {CH₂-(CH₂)ₐ-NR³-CH₂-(CH₂)_{b}-NR}ₘ-[CH₂-(CH₂)ₐ-NR⁵]ₙ-R⁶ (IVa),

wherein a, b, m, n, and R¹ to R⁶ are defined as in formula (IV) and wherein one or more of the nitrogen atoms indicated in formula (IVa) may be protonated to provide a cationic lipidoid;

In some cases, the lipidoid is a lipidoid of formula (IV). In some cases 'n' is 1 in a lipidoid of formula (IV). In some cases, 'm' is 1 and n is 1 in a lipidoid of formula (IV). In some cases, the lipidoid of formula (IV) is a lipidoid of formula (IVb):

R-NR²-CH₂-(CH₂)ₐ-NR³-CH₂-(CH₂)_{b}-NR⁴-CH₂-(CH₂)ₐ-NR⁵-R⁶ (IVb),

wherein a, b, and R¹ to R⁶ are defined as in formula (IV) wherein one or more of the nitrogen atoms indicated in formula (IVb) may be protonated to provide a cationic lipidoid.

As regards the length of the alkylene amine units in the lipidoid of formula (IV), (IVa) and (IVb), it will be understood that one of the alternating units needs to be an ethylene amine unit (i.e., either a or b is 1). The other alternating unit can be a propylene amine unit, a butylene amine unit, a pentylene amine unit, or another suitable unit (i.e., the other one of a or b is an integer of 2 to 4. In some cases, a lipidoid of formula (N) is a lipidoid of formula (IVc):

R¹-NR²-CH₂-CH₂-NR³-CH₂-CH₂-CH₂-NR⁴-CH₂-CH₂-NR⁵-R^{S} (IVc)

wherein R¹ to R⁶ are as defined in formula (IV) and wherein one or more of the nitrogen atoms indicated in formula (IVc) can be protonated to provide a cationic lipidoid;

In some cases, the groups R¹ to R⁶ in formula (IV), (IVa), (IVb) and (IVc) are a protecting group for an amino group. Non-limiting examples of protecting groups include t-butoxycarbonyl (Boc), 9-fluorenylmethoxycarbonyl (Fmoc), or carbobenzyloxy (Cbz).

As far as the groups R¹ to R⁶ in formula (IV), (IVa), (IVb) and (IVc) are a receptor ligand, such as the receptor ligands described in Philipp and Wagner in "Gene and Cell Therapy - Therapeutic Mechanisms and Strategy", 3rd Edition, Chapter 15, CRC Press, Taylor & Francis Group LLC, Boca Raton 2009. Examples of receptor ligands that target the lung tissue are described in Pfeifer et al. 2010, Ther. Deliv. 1 (1): 133-48. Receptor ligands can include synthetic cyclic or linear peptides such as derived from screening peptide libraries for binding to a particular cell surface structure or particular cell type, cyclic or linear RGD peptides, synthetic or natural carbohydrates such as sialic acid, galactose or mannose or synthetic ligands derived from reacting a carbohydrate for example with a peptide, antibodies specifically recognizing cell surface structures, folic acid, epidermal growth factor and peptides derived thereof, transferrin, anti-transferrin receptor antibodies, nanobodies and antibody fragments, approved drugs that may bind to cell surface molecules (e.g., cell surface receptors), etc.

As far as the groups R¹ to R⁶ in formula (IV), (IVa), (IVb) and (IVc) are a poly(ethylene glycol) chain, the molecular weight of the poly(ethylene glycol) chain can be from about 100 g/mol to 20,000 g/mol, from about 1,000 g/mol to 10,000 g/mol or from about 1,000 g/mol to 5,000 g/mol. In some cases, a molecular weight of the PEG chain can provide a composition with a desired density.

Multiple lipidoid molecules can be associated with an engineered polyribonucleotide. For example, a composition can comprise 1 engineered polyribonucleotide to 100 lipidoid molecules, 1 engineered polyribonucleotide to 1,000 lipidoid molecules, 10 engineered polyribonucleotide to 1,000 lipidoid molecules, or 100 engineered polyribonucleotide to 10,000 lipidoid molecules. The complex of engineered polyribonucleotide and lipidoid can form a particle. The diameter of the particles may range, e.g., from 10 nanometers to 1,200 nanometers. In some cases the diameter of the particles ranges from 10 nanometers to 500 nanometers. In some cases, the diameters of the particles are from 20 nanometers to 150 nanometers.

### Administration to a subject

Further described herein are methods for the administration of a polynucleotide (e.g., polyribonucleotide) to a subject. The polyribonucleotide can be provided to the subject via a delivery agent, such as a particle or capsule with an encapsulating agent that encapsulates the polyribonucleotide. The delivery agent can be a therapeutic agent. The subject can be a human, such as a human afflicted with cancer. The delivery agent can be administered to the subject (e.g., self-administration or administration by a third party, such as a healthcare provider) at a given dosage, and the dosage can be increased with time, decreased with time, or kept constant. The dosage can be changed based on a progression or regression of a disease in the subject, such as a rare disease or a cancer.

A polyribonucleotide of the disclosure can be formulated with one or more pharmaceutically acceptable carrier(s) to be administered to a subject. In some cases, the polyribonucleotide can be formulated for targeted delivery to a target cell or cell population. In some cases, the polyribonucleotide can be formulated for untargeted delivery to a cell or cell population. The encoded polypeptide product of the polyribonucleotide is then transcribed and it accumulates within the recipient cell.

A composition can be a combination of any engineered polyribonucleotide described herein with other chemical components, such as carriers, stabilizers, diluents, dispersing agents, suspending agents, thickening agents, and/or excipients. The composition facilitates administration of the compound to an organism. Pharmaceutical compositions can be administered in therapeutically-effective amounts as pharmaceutical compositions by various forms and routes including, for example, intravenous, subcutaneous, intramuscular, oral, rectal, aerosol, parenteral, ophthalmic, pulmonary, transdermal, vaginal, otic, nasal, and topical administration.

A composition can be administered in a local or systemic manner, for example, via injection of the compound directly into an organ, optionally in a depot or sustained release formulation. Pharmaceutical compositions can be provided in the form of a rapid release formulation, in the form of an extended release formulation, or in the form of an intermediate release formulation. A rapid release form can provide an immediate release. An extended release formulation can provide a controlled release or a sustained delayed release.

For administration by inhalation, the active compounds can be in a form as an aerosol, a mist, a vapor, a spray, or a powder. Pharmaceutical compositions are conveniently delivered in the form of an aerosol spray presentation from pressurized packs or a nebuliser, with the use of a suitable propellant, for example, dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges of, for example, gelatin for use in an inhaler or insufflator can be formulated containing a powder mix of the compounds and a suitable powder base such as lactose or starch.

The eye comprises several structurally and functionally distinct vascular beds that supply ocular components critical to the maintenance of vision. These beds include the retinal and choroidal vasculatures, which supply the inner and outer portions of the retina, respectively, and the limbal vasculature located at the periphery of the cornea.

A pharmaceutical composition comprising an engineered polyribonucleotide can be administered to the eye via any suitable form or route including, for example, topical, oral, systemic, intravitreal, intracameral, subconjunctival, subtenon, retrobulbar, intraocular, posterior juxtascleral, periocular, subretinal, and suprachoroidal administration. The compositions can be administered by injecting the formulation in any part of the eye including anterior chamber, posterior chamber, vitreous chamber (intravitreal), retina proper, and/or subretinal space. The compositions can also be delivered via a non-invasive method. Non-invasive modes of administering the formulation can include using a needleless injection device. Multiple administration routes can be employed for efficient delivery of the pharmaceutical compositions.

An engineered polynucleotide of the disclosure can be delivered to any suitable ocular cell including for example, endothelial cells such as vascular endothelial cells, cells of the retina such as retinal pigment epilthelium (RPE), corneal cells, fibroblasts, astrocytes, glial cells, pericytes, iris epithelial cells, cells of neural origin, ciliary epithelial cells, mueller cells, muscle cells surrounding and attached to the eye such as cells of the lateral rectus muscle, orbital fat cells, cells of the sclera and episclera, cells of the trabecular meshwork, and connective tissue cells.

A composition that is disclosed herein, upon administration to a subject, can have a transfection efficiency of at least about 80%, 90%, or 95% by the cell of the subject. In some cases, the transfection efficiency of an encapsulated composition , upon administration to a subject, is at least about 50%, 60%, 70%, 80%, 90%, 95%, 100%, 110%, 120%, 130%, 140%, 150%, 160%, 170%, 180%, 190%, 200%, 225%, 250%, 275%, 300%, 325%, 350%, 375%, 400%, 450%, or 500% relative to an unencapsulated polyribonucleotide. In some situations, transfection efficiency of a composition comprising a modified polyribonucleotide (in some cases also comprising an unmodified polyribonucleotide), upon administration to a subject, is at least about 50%, 60%, 70%, 80%, 90%, 95%, 100%, 110%, 120%, 130%, 140%, 150%, 160%, 170%, 180%, 190%, 200%, 225%, 250%, 275%, 300%, 325%, 350%, 375%, 400%, 450%, or 500% relative to composition solely containing an unmodified polyribonucleotide. The transfection efficiency of a composition can be increased by addition of a carrier, such as a cell penetrating peptide or a cationic coating to the outer layer of the composition. The transfection efficiency of a composition can be modulated by the density of a composition.

Methods for the preparation of compositions comprising the engineered polyribonucleotides described herein include formulating the compounds with one or more inert, pharmaceutically-acceptable excipients or carriers to form a solid, semi-solid, or liquid composition. Solid compositions include, for example, powders, tablets, dispersible granules, capsules, cachets, and suppositories. Liquid compositions include, for example, solutions in which a compound is dissolved, emulsions comprising a compound, or a solution containing liposomes, micelles, or nanoparticles comprising a compound as disclosed herein. Semi-solid compositions include, for example, gels, suspensions and creams. The compositions can be in liquid solutions or suspensions, solid forms suitable for solution or suspension in a liquid prior to use, or as emulsions. These compositions can also contain minor amounts of nontoxic, auxiliary substances, such as wetting or emulsifying agents, pH buffering agents, and other pharmaceutically-acceptable additives.

Non-limiting examples of pharmaceutically-acceptable excipients can be found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999), each of which is incorporated by reference in its entirety.

A composition comprising a polynucleotide (e.g., polyribonucleotide) can be provided in various dosages. A dose of a polynucleotide, or a polyribonucleotide, can be from about 1 µg to about 1000 µg, about 1 µg to about 500 µg, about 1 µg to about 1000 µg, about 10 µg to about 500 µg, about 20 µg to about 500 µg, about 25 µg to about 500 µg, about 30 µg to about 500 µg, about 40 µg to about 500 µg, about 50 µg to about 500 µg, about 10 µg to about 250 µg, about 20 µg to about 250 µg, about 30 µg to about 250 µg, about 40 µg to about 250 µg, about 50 µg to about 250 µg, about 1 µg to about 200 µg, about 10 µg to about 200 µg, about 20 µg to about 200 µg, about 30 µg to about 200 µg, about 40 µg to about 200 µg, about 50 µg to about 200 µg, about 25 µg to about 50 µg, about 25 µg to about 100 µg, about 25 µg to about 150 µg, about 25 µg to about 200 µg, about 25 µg to about 250 µg, about 25 µg to about 300 µg, about 25 µg to about 350 µg, about 25 µg to about 400 µg, about 25 µg to about 450 µg, about 25 µg to about 500 µg, about 50 µg to about 750 µg, or about 25 µg to about 1000 µg of the engineered polyribonucleotide. In some cases, a dose of a polynucleotide is about 1 mg to about 100 mg, about 1 mg to about 50 mg, about 10 mg to about 50 mg, about 20 mg to about 50 mg, about 25 mg to about 50 mg, about 30 mg to about 50 mg, about 40 mg to about 50 mg, about 50 mg to about 100 mg, about 1 mg to about 25 mg, about 2 mg to about 25 mg, about 3 mg to about 25 mg, about 4 mg to about 25 mg, about 5 mg to about 25 mg, about 1 mg to about 20 mg, about 1 mg to about 20 mg, about 2 mg to about 20 mg, about 3 mg to about 20 mg, about 4 mg to about 20 mg, or about 5 mg to about 20 mg of an engineered polyribonucleotide.

The percentage of a polyribonucleotide in a formulation (e.g., within an encapsulated agent) can be greater than or equal to 0.25 % polyribonucleotide, 0.5 % polyribonucleotide, 0.75 % polyribonucleotide, 1 % polyribonucleotide, 1.25 % polyribonucleotide, 1.5 % polyribonucleotide, 1.75 % polyribonucleotide, 2 % polyribonucleotide, 2.25 % polyribonucleotide, 2.5 % polyribonucleotide, 2.75 % polyribonucleotide, 3 % polyribonucleotide, 3.25 % polyribonucleotide, 3.5 % polyribonucleotide, 3.75 % polyribonucleotide, 4 % polyribonucleotide, 4.25 % polyribonucleotide, 4.5 % polyribonucleotide, 4.75 % polyribonucleotide, 5 % polyribonucleotide, 5.25 % polyribonucleotide, 5.5 % polyribonucleotide, 5.75 % polyribonucleotide, 6 % polyribonucleotide, 6.25 % polyribonucleotide, 6.5 % polyribonucleotide, 6.75 % polyribonucleotide, 7 % polyribonucleotide, 7.25 % polyribonucleotide, 7.5 % polyribonucleotide, 7.75 % polyribonucleotide, 8 % polyribonucleotide, 8.25 % polyribonucleotide, 8.5 % polyribonucleotide, 8.75 % polyribonucleotide, 9 % polyribonucleotide, 9.25 % polyribonucleotide, 9.5 % polyribonucleotide, 9.75 % polyribonucleotide, 10 % polyribonucleotide, 10.25 % polyribonucleotide, 10.5 % polyribonucleotide, 10.75 % polyribonucleotide, 11 % polyribonucleotide, 11.25 % polyribonucleotide, 11.5 % polyribonucleotide, 11.75 % polyribonucleotide, 12 % polyribonucleotide, 12.25 % polyribonucleotide, 12.5 % polyribonucleotide, 12.75 % polyribonucleotide, 13 % polyribonucleotide, 13.25 % polyribonucleotide, 13.5 % polyribonucleotide, 13.75 % polyribonucleotide, 14 % polyribonucleotide, 14.25 % polyribonucleotide, 14.5 % polyribonucleotide, 14.75 % polyribonucleotide, 15 % polyribonucleotide, 15.25 % polyribonucleotide, 15.5 % polyribonucleotide, 15.75 % polyribonucleotide, 16 % polyribonucleotide, 16.25 % polyribonucleotide, 16.5 % polyribonucleotide, 16.75 % polyribonucleotide, 17 % polyribonucleotide, 17.25 % polyribonucleotide, 17.5 % polyribonucleotide, 17.75 % polyribonucleotide, 18 % polyribonucleotide, 18.25 % polyribonucleotide, 18.5 % polyribonucleotide, 18.75 % polyribonucleotide, 19 % polyribonucleotide, 19.25 % polyribonucleotide, 19.5 % polyribonucleotide, 19.75 % polyribonucleotide, 20 % polyribonucleotide, 20.5 % polyribonucleotide, 21 % polyribonucleotide, 21.5 % polyribonucleotide, 22 % polyribonucleotide, 22.5 % polyribonucleotide, 23 % polyribonucleotide, 23.5 % polyribonucleotide, 24 % polyribonucleotide, 24.5 % polyribonucleotide, or 25% polyribonucleotide by weight. Alternatively, the percentage of the polyribonucleotide in the formulation (e.g., within an encapsulated agent) can be less than about 25 % polyribonucleotide, 24.5 % polyribonucleotide, 24 % polyribonucleotide, 23.5 % polyribonucleotide, 23 % polyribonucleotide, 22.5 % polyribonucleotide, 22 % polyribonucleotide, 21.5 % polyribonucleotide, 21% polyribonucleotide, 20.5 % polyribonucleotide, 20 % polyribonucleotide, 19.5 % polyribonucleotide, 19% polyribonucleotide, 18.5 % polyribonucleotide, 18 % polyribonucleotide, 17.5 % polyribonucleotide, 17 % polyribonucleotide, 16.5 % polyribonucleotide, 16 % polyribonucleotide, 15.5 % polyribonucleotide, 15% polyribonucleotide, 14.5 % polyribonucleotide, 14 % polyribonucleotide, 13.5 % polyribonucleotide, 13 % polyribonucleotide, 12.5 % polyribonucleotide, 12 % polyribonucleotide, 11.5 % polyribonucleotide, 11 % polyribonucleotide, 10.5 % polyribonucleotide, 10 % polyribonucleotide, 9.5 % polyribonucleotide, 9 % polyribonucleotide, 8.5 % polyribonucleotide, 8 % polyribonucleotide, 7.5 % polyribonucleotide, 7 % polyribonucleotide, 6.5 % polyribonucleotide, 6 % polyribonucleotide, 5.5 % polyribonucleotide, 5 % polyribonucleotide, 4.5 % polyribonucleotide, 4% polyribonucleotide, 3.5 % polyribonucleotide, 3% polyribonucleotide, 2.5 % polyribonucleotide, 2 % polyribonucleotide, 1.5 % polyribonucleotide, 1% polyribonucleotide, 0.5% polyribonucleotide, or 0.1 % polyribonucleotide.

In some cases, an encapsulated composition of the disclosure can produce a plasma, serum or blood concentration of the polyribonucleotide, pharmaceutical carrier, encapsulating agent, or polymeric material (e.g.: polyethylene glycol or polyethyenimine) in a subject within about 1 second to about 30 minutes, about 1 second to 20 minutes, about 1 second to 10 minutes, about 1 second to 5 minutes, about 1 second to 2 minutes, about 1 second to 1 minute, about 1 second to about 30 seconds, about 30 seconds to 30 minutes, about 30 seconds to 20 minutes, about 30 seconds to 10 minutes, about 30 seconds to 5 minutes, about 30 seconds to 2 minutes, about 30 seconds to about 1 minute, about 1 minute to about 30 minutes, about 1 minute to about 25 minutes, about 1 minute to about 20 minutes, about 1 minute to about 15 minutes, about 1 minute to about 10 minutes, about 5 minutes to about 30 minutes, about 5 minutes to about 25 minutes, about 5 minutes to about 20 minutes, about 5 minutes to about 15 minutes, about 5 minutes to about 10 minutes, about 10 minutes to about 30 minutes, about 10 minutes to about 25 minutes, about 10 minutes to about 20 minutes, or about 10 minutes to about 15 minutes of use of the device. The plasma, serum or blood concentration of the polyribonucleotide, pharmaceutical carrier, encapsulating agent, or polymeric material (e.g.: polyethylene glycol or polyethyenimine) concentration can be a peak concentration or an average concentration.

### Treatments and Conditions

The methods, polyribonucleotides, and pharmaceutical compositions of this disclosure provide a method to treat a condition. The treatment may comprise treating a subject (e.g., a patient with a disease and/or a lab animal with a condition). In some cases the condition is a cancer. In some cases, the condition is lung cancer. In some cases, the condition is breast cancer. In some cases, the subject is a human. Treatment may be provided to the subject before clinical onset of disease. Treatment may be provided to the subject after clinical onset of disease. Treatment may be provided to the subject on or after 1 minute, 5 minutes, 10 minutes, 30 minutes, 1 hour, 2 hours, 3 hours, 4 hours, 5 hours, 6 hours, 12 hours, 1 day, 1 week, 6 months, 12 months, or 2 years after clinical onset of the disease. Treatment may be provided to the subject for a time period that is greater than or equal to 1 minutes, 10 minutes, 30 minutes, 1 hours, 2 hours, 3 hours, 4 hours, 5 hours, 6 hours, 12 hours, 1 day, 1 week, 1 month, 6 months, 12 months, 2 years or more after clinical onset of the disease. Treatment may be provided to the subject for a time period that is less than or equal to 2 years, 12 months, 6 months, 1 month, 1 week, 1 day, 12 hours, 6 hours, 5 hours, 4 hours, 3 hours, 2 hours, 1 hour, 30 minutes, 10 minutes, or 1 minute after clinical onset of the disease. Treatment may also include treating a human in a clinical trial.

Compositions containing the engineered polyribonucleotides described herein can be administered for prophylactic and/or therapeutic treatments. In therapeutic applications, the engineered polyribonucleotides can be administered to a subject already suffering from a disease, such as a lung or a breast cancer, in the amount sufficient to provide the amount of the encoded polyribonucleotide that cures or at least improves the symptoms of the disease. Engineered polyribonucleotides can also be administered to lessen a likelihood of developing, contracting, or worsening a disease. Amounts effective for this use can vary based on the severity and course of the disease or condition, the efficiency of transfection of an engineered polynucleotide, the affinity of an encoded polypeptide to a target molecule, previous therapy, the subject's health status, weight, response to the drugs, and the judgment of the treating physician.

The polyribonucleotides of the disclosure can be used, for example, to treat a condition associated with a defect or malfunction of a gene in the ATP-binding cassette (ABC) family. Non-limiting examples of conditions associated with a gene in the ATP-binding cassette (ABC) family include: age-related macular degeneration, benign recurrent intrahepatic cholestasis, Cantu syndrome, congenital bilateral absence of the vas deferens, congenital hyperinsulinism, cystic fibrosis, Dubin-Johnson syndrome, familial dilated cardiomyopathy, familial HDL deficiency, generalized arterial calcification of infancy, harlequin ichthyosis, hereditary pancreatitis, intrahepatic cholestasis of pregnancy, lamellar ichthyosis, permanent neonatal diabetes mellitus, progressive familial intrahepatic cholestasis, pseudoxanthoma elasticum, retinitis pigmentosa, sitosterolemia, Stargardt macular degeneration, surfactant dysfunction, Tangier disease, X-linked adrenoleukodystrophy, X-linked sideroblastic anemia and ataxia.

A polyribonucleotide, a method, and a pharmaceutical composition of the disclosure can be used, for example, to treat a cancer. Non-limiting examples of cancers can include: acute lymphoblastic leukemia, acute myeloid leukemia, adrenocortical carcinoma, AIDS-related cancers, AIDS-related lymphoma, anal cancer, appendix cancer, astrocytomas, basal cell carcinoma, bile duct cancer, bladder cancer, bone cancers, brain tumors, such as cerebellar astrocytoma, cerebral astrocytoma/malignant glioma, ependymoma, medulloblastoma, supratentorial primitive neuroectodermal tumors, visual pathway and hypothalamic glioma, breast cancer, bronchial adenomas, Burkitt lymphoma, carcinoma of unknown primary origin, central nervous system lymphoma, cerebellar astrocytoma, cervical cancer, childhood cancers, chronic lymphocytic leukemia, chronic myelogenous leukemia, chronic myeloproliferative disorders, colon cancer, cutaneous T-cell lymphoma, desmoplastic small round cell tumor, endometrial cancer, ependymoma, esophageal cancer, Ewing's sarcoma, germ cell tumors, gallbladder cancer, gastric cancer, gastrointestinal carcinoid tumor, gastrointestinal stromal tumor, gliomas, hairy cell leukemia, head and neck cancer, heart cancer, hepatocellular (liver) cancer, Hodgkin lymphoma, Hypopharyngeal cancer, intraocular melanoma, islet cell carcinoma, Kaposi sarcoma, kidney cancer, laryngeal cancer, lip and oral cavity cancer, liposarcoma, liver cancer, lung cancers, such as non-small cell and small cell lung cancer, lymphomas, leukemias, macroglobulinemia, malignant fibrous histiocytoma of bone/osteosarcoma, medulloblastoma, melanomas, mesothelioma, metastatic squamous neck cancer with occult primary, mouth cancer, multiple endocrine neoplasia syndrome, myelodysplastic syndromes, myeloid leukemia, nasal cavity and paranasal sinus cancer, nasopharyngeal carcinoma, neuroblastoma, non-Hodgkin lymphoma, non-small cell lung cancer, oral cancer, oropharyngeal cancer, osteosarcoma/malignant fibrous histiocytoma of bone, ovarian cancer, ovarian epithelial cancer, ovarian germ cell tumor, pancreatic cancer, pancreatic cancer islet cell, paranasal sinus and nasal cavity cancer, parathyroid cancer, penile cancer, pharyngeal cancer, pheochromocytoma, pineal astrocytoma, pineal germinoma, pituitary adenoma, pleuropulmonary blastoma, plasma cell neoplasia, primary central nervous system lymphoma, prostate cancer, rectal cancer, renal cell carcinoma, renal pelvis and ureter transitional cell cancer, retinoblastoma, rhabdomyosarcoma, salivary gland cancer, sarcomas, skin cancers, skin carcinoma merkel cell, small intestine cancer, soft tissue sarcoma, squamous cell carcinoma, stomach cancer, T-cell lymphoma, throat cancer, thymoma, thymic carcinoma, thyroid cancer, trophoblastic tumor (gestational), cancers of unknown primary site, urethral cancer, uterine sarcoma, vaginal cancer, vulvar cancer, Waldenstrom macroglobulinemia, and Wilms tumor. In some cases, an engineered polyribonucleotide is administered to a subject to treat a lung cancer. In some cases, an engineered polyribonucleotide is administered to a subject to treat a breast cancer.

In some cases, a polynucleotide of the disclosure can encode a polypeptide that is at least 80% homologous to a protein of the ATP-binding cassette, sub-family A, such as ABCA3.

Multiple engineered polyribonucleotides can be administered in any order or simultaneously. The engineered polyribonucleotides can be packed together or separately, in a single package comprising polyribonucleotides that target the same target molecule or in a plurality of packages. One or all of the engineered polyribonucleotides can be given in multiple doses. If not simultaneous, the timing between the multiple doses may vary.

The engineered polyribonucleotides can be administered to a subject as soon as possible after the onset of the symptoms. An engineered polyribonucleotides can be administered as soon as is practical after the onset of a disease or condition is detected or suspected, and for a length of time necessary for the treatment of the disease, such as, for example, for about 1 month, for about 6 months, for about 12 months, for about 18 months, for about 24 months, or any appropriate length of time. The length of treatment can vary for each subject.

### EXAMPLES (examples relating to UTR-CYBA are according to the present invention; the other examples are given as reference)

### EXAMPLE 1: Formulation of a Composition Comprising an Engineered Polyribonucleotide for the Treatment of Human Subjects Afflicted with a Lung Disorder

Compositions are formulated as follows:
An engineered polynucleotide encoding the ABCA3 gene sequence, NCBI Reference Sequence: NM_001089.2, is prepared as described by WO2011012316, WO2014207231, WO2014153052, WO2013185069. Branched polyethylenimine (PEI, average MW = 25 kDa) is purchased from Sigma-Aldrich™ (Schnelldorf, Germany) and used without further purification. PEI is diluted in endotoxin free water and adjusted to pH 7.4 with HC1. Endotoxin free water is purchased from B. Braun (Melsungen, Germany).

The engineered polyribonucleotide and PEI can be diluted in 4.0 ml of double distilled water resulting in concentrations of 250 µg/ml mRNA and 326.3 µg/ml PEI, respectively (roughly at a polynucleotide to PEI ratio of 10). The engineered polyribonucleotide solution can be pipetted into the PEI solution, mixed by pipetting up and down, to yield a final polyribonucleotide concentration of 125 µg/ml. The complexes can be incubated for 20 min at room temperature prior to being administered to a subject.

### EXAMPLE 2: Design, Synthesis, Formulation, and Cell-Based Characterization of Engineered Polyribonucleotides for the Synthesis of Human ABCA3

Eight different versions of Engineered Polyribonucleotides encoding variations of the human ABCA3 are prepared comprising the following sequences

| **Table 7** |
|---|
| Native ORF alone (no UTRs) |
| Native ORF + native UTRs |
| Native ORF + CYBA UTRs |
| Native ORF + α-globin 5' UTR ETH |
| Codon-optimized ORF |
| Codon-optimized ORF + native UTRs |
| Codon optimized native ORF + CYBA UTRs |
| Codon-optimized ORF + UTR-ETH |

The constructs are sub-cloned into a vector and amplified with standard procedures (e.g.: maxi prep). The nucleic acid sequence of each construct is confirmed with sequencing techniques.

### EXAMPLE 3: Expression of Engineered Polyribonucleotides in Human Alveolar Epithelial Cell Lines

Different constructs expressing the sequences described in **TABLE 6** are administered to the human alveolar epithelial cell lines A549, HepG2, and HEK293 cells. Each construct is incorporated into a formulation comprising an N/P ratio of about 8 (N/P = the ratios of moles of the amine groups of cationic polymers to those of the phosphate ones of DNA). Examples of formulations and the molar ratio of the components are shown in **TABLE 8.**

| **TABLE 8** | | | | |
|---|---|---|---|---|
| **Formulation** | **Cationic Lipid** | **DPPC** | **Cholesterol** | **DMG-PEG2000** |
| Formulation 1 | 40 % dL_01 and 60 % dL_05 | 5.29 | 4.41 | 0.88 |
| Formulation 2 | 60 % dL_01 and 40 % dL_05 | 5.29 | 4.41 | 0.88 |
| Formulation 3 | 100 % dL_05 | 5.29 | 4.41 | 0.88 |
| Formulation 4 | 100 % dL_01 | 5.29 | 4.41 | 0.88 |

As used herein dL_01 is

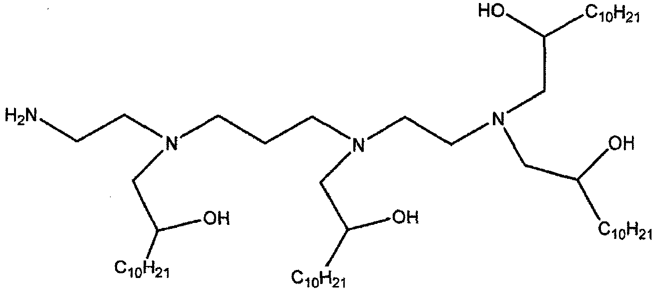

As used herein dL_05 is

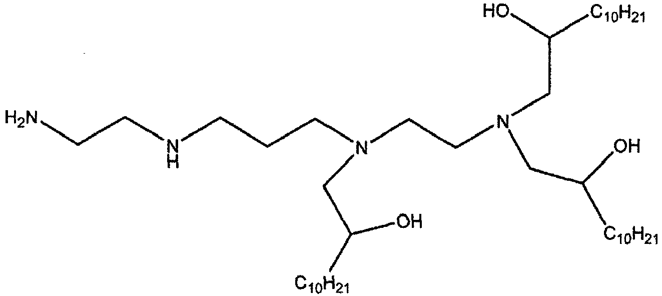

The polyplex size and the zeta potential of each formulation is measured. The stability of each formulation is measured at various pHs, temperatures and after vigorous shaking.

The half-life, RNA uptake, and immunogenicity of each polyribonucleotide formulation is measured in all three lung epithelial cell lines (A549, HepG2 and HEK293).

### EXAMPLE 4: Uptake, Expression and Distribution of an Engineered Polyribonucleotide Encoding a Reporter Gene

Engineered polyribonucleotides encoding a firefly luciferase (FFL, 1.64 kb), a Tomato Red (TR, 1.86 kb), an Enhanced Green Fluorescent Protein (EGFP, 0.94 kb), and a LacZ (3.3 kb) reporter. This study also describes a comparison between polyethyleneimine ("PEI") and EPE (a statistical polymer of aminoethyl and aminopropyl units; see EP-A1 3034539, which is incorporated by reference herein) formulations, cellular distribution (*e.g*., alveolar type II epithelial cells, alveolar type I epithelial cells, pulmonary microvascular endothelial cells) and variability in dose ranges for optimal uptake, expression and distribution of different engineered polyribonucleotides encoding a reporter gene. **TABLE 9** illustrates different dosing regimens that are tested in a mouse model.

| **TABLE 9** | | | | | |
|---|---|---|---|---|---|
| Group # | Engineered Polyribonucleotides | Dose * | Dosing Regimen | N | Time Point |
| 1 | polyethyleneimine ("PEI") vehicle | - | Single Dose | 3 | 24 hours |
| 2 | EPE vehicle | - | Single Dose | 3 | 24 hours |
| 3 | PEI + FFL | 0.1 to 1 mg/kg | Single Dose | 3 | 24 hours |
| 4 | PEI + FFL | 1.0 to 10.0 mg/kg | Single Dose | 3 | 24 hours |
| 5 | EPE + FFL | 0.1 to 1 mg/kg | Single Dose | 3 | 24 hours |
| 6 | EPE + FFL | 1.0 to 10.0 mg/kg | Single Dose | 3 | 24 hours |
| 7 | PEI + Tomato Red | 0.1 to 1 mg/kg | Single Dose | 3 | 24 hours |
| 8 | PEI + Tomato Red | 1.0 to 10.0 mg/kg | Single Dose | 3 | 24 hours |
| 9 | EPE + Tomato Red | 0.1 to 1 mg/kg | Single Dose | 3 | 24 hours |
| 10 | EPE + Tomato Red | 1.0 to 10.0 mg/kg | Single Dose | 3 | 24 hours |
| 11 | PEI + EGFP | 0.1 to 1 mg/kg | Single Dose | 3 | 24 hours |
| 12 | PEI + EGFP | 1.0 to 10.0 mg/kg | Single Dose | 3 | 24 hours |
| 13 | EPE + EGFP | 0.1 to 1 mg/kg | Single Dose | 3 | 24 hours |
| 14 | EPE + EGFP | 1.0 to 10.0 mg/kg | Single Dose | 3 | 24 hours |
| 15 | PEI + LacZ | 0.1 to 1 mg/kg | Single Dose | 3 | 24 hours |
| 16 | PEI + LacZ | 1.0 to 10.0 mg/kg | Single Dose | 3 | 24 hours |
| 17 | EPE + LacZ | 0.1 to 1 mg/kg | Single Dose | 3 | 24 hours |
| 18 | EPE + LacZ | 1.0 to 10.0 mg/kg | Single Dose | 3 | 24 ours |

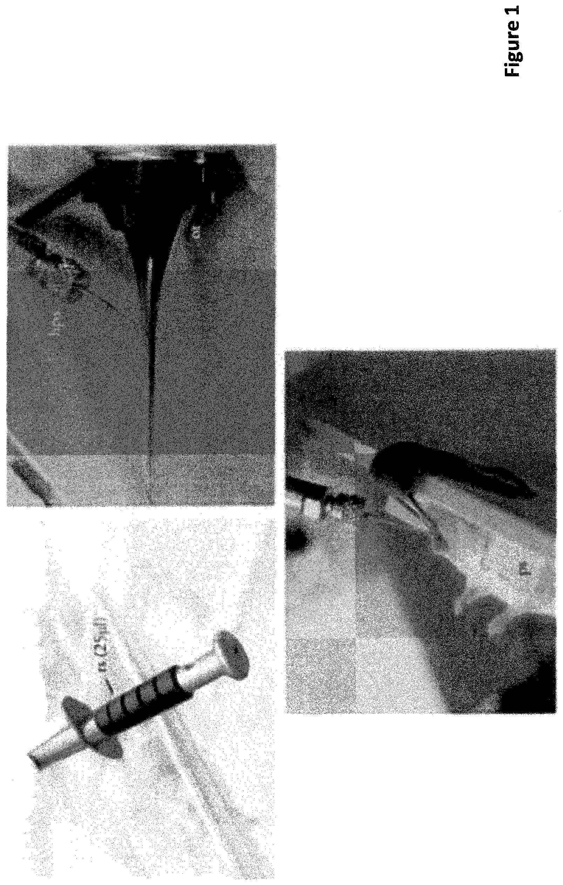

Route of administration: inhalation (aerosol). **FIGURE 1** illustrates an example of aerosol administration of engineered polyribonucleotides to mice. The experiment can be conducted in any mice, such as wild-type C57B16 mice. The expression of a firefly luciferase (FFL, 1.64 kb), a Tomato Red (TR, 1.86 kb), an Enhanced Green Fluorescent Protein (EGFP, 0.94 kb), and a LacZ (3.3 kb) reporter is measured in a total number of 54 mice.

### EXAMPLE 5: In vivo Uptake, Expression and Distribution of an Engineered Polyribonucleotide Encoding an ABCA3 protein

A total of 36 wild-type C57B16 mice are administered one or more constructs comprising the polyribonucleotides of **TABLE 7** prepared with one or more formulations listed in **TABLE 8.** An endotracheal tube is used as the route of administration.

Lung tissue of each mouse is collected about 24 hours after endotracheal administration of each peptide. The tissue of one lung per mouse is flash frozen and analyzed by qPCR, western blot, and ELISA for the expression of ABCA3 protein. The tissue of one lung per mouse is fixed in formalin and analyzed for *in situ* presence of the engineered polyribonucleotide and histological analysis. Bronchoalveolar Lavage Fluid (BALF) is collected and the expression levels of the markers IL-10, MIP-1α, INF-α, TNF-α, IL-12, and IL-6 are analyzed to determine the immunogenicity of each engineered polyribonucleotide.

### EXAMPLE 6: In vivo Dosing Study of an Engineered Polyribonucleotide Encoding an ABCA3 protein

Wild-type C57B16 mice are administered one or more constructs comprising the polyribonucleotides of **TABLE 7** prepared in one or more formulations listed in **TABLE 8.** An endotracheal tube is used to administer the engineered polyribonucleotides to each mouse. Different dosages of each engineered polyribonucleotide are tested, including dosages ranging from 0.1 mg/kg to 1 mg/kg and 1 mg/kg to 10 mg/kg.

Lung tissue of each mouse is collected about 24 hours after endotracheal administration of each peptide. The tissue of one lung per mouse is flash frozen and analyzed by qPCR, western blot, and ELISA for the expression of ABCA3 protein. The tissue of one lung per mouse is fixed in formalin and analyzed for *in situ* presence of the engineered polyribonucleotide and histological analysis. Bronchoalveolar Lavage Fluid (BALF) is collected and the expression levels of the markers IL-10, MIP-1α, INF-α, TNF-α, IL-12, and IL-6 are analyzed to determine the immunogenicity of each engineered polyribonucleotide.

The metrics determined from the lung and biological samples are used to evaluate the safety and efficacy of each dosage.

### EXAMPLE 7: In vivo Dosing Study of an Engineered Polyribonucleotide Encoding an ABCA3 protein

Wild-type C57B16 mice repeatedly inhale an aerosol comprising one or more constructs with the polyribonucleotides listed in **TABLE 7.** Different dosages of each engineered polyribonucleotide are tested, including dosages ranging from 0.1 mg/kg to 1 mg/kg and 1 mg/kg to 10 mg/kg.

Different mice receive repeated dosages at 8 hours, 24 hours, 48 hours, 72 hours, 96 hours, and 120 hours after an initial dosage. Lung tissue and biological sample of different mice is collected about 8 hours, 24 hours, 48 hours, 72 hours, 96 hours, and 120 hours after administration of each peptide. At each specified time, the tissue of one lung per mouse is flash frozen and analyzed by qPCR, western blot, and ELISA for the expression of ABCA3 protein. The tissue of one lung per mouse is fixed in formalin and analyzed for *in situ* presence of the engineered polyribonucleotide and histological analysis. Bronchoalveolar Lavage Fluid (BALF) is collected and the expression levels of the markers IL-10, MIP-1α, INF-α, TNF-α, IL-12, and IL-6 are analyzed to determine the immunogenicity of each engineered polyribonucleotide. Blood, liver and spleen are also collected, flash frozen, and processed for histological analysis.

The metrics determined from the lung and biological samples are used to evaluate the safety and efficacy of repeated administrations of aerosolized engineered polyribonucleotides.

### EXAMPLE 8: Expression of ABCA3 Ribonucleic Acid in Mammalian Cells

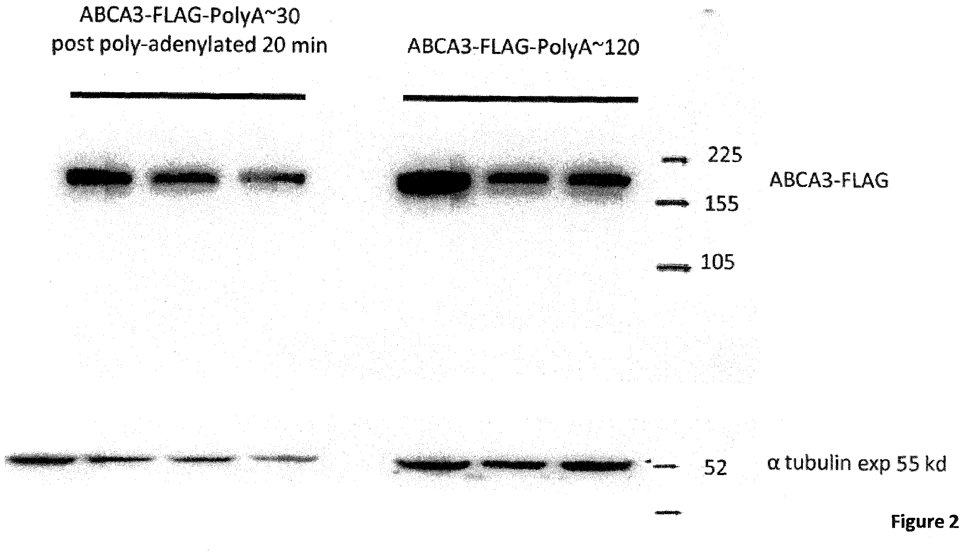

This experiment demonstrates the expression (translation) of two ABCA3-FLAG mRNAs in-*vitro.* For this experiment, ABCA3-FLAG DNA lacking a 5' UTR (SEQ ID NO: 17) **(****FIGURE 2****:** ABCA3-FLAG-PolyA∼30 post poly-adenylated 20 minutes; made from a template with a 30A polyA tail that was post polyadenylated for 20 minutes to extend the tail) and ABCA3-FLAG DNA comprising a 5' human α-globin UTR (SEQ ID NO: 18) **(****FIGURE 2****:** ABCA3-FLAG-PolyA∼120, comprising a 5' UTR of SEQ ID NO: 5 at the DNA level and SEQ ID NO: 16 at the RNA level) were used to transcribe the corresponding mRNAs *in vitro* with the four canonical nucleotides, capped with a cap1 structure, and poly adenylated. These two ABCA3-FLAG mRNAs, ABCA3-FLAG-PolyA^{∼}30 post polyadenylated and ABCA3-FLAG-PolyA^{∼}100, were transfected into A549 cells. **FIGURE 2** is a western blot illustrating the translation of ABCA3 mRNAs in A549 cells 6 hours post-transfection. Biological triplicates are shown in the figure.

For this experiment, 2 x 10⁶ A549 cells (p7) were transfected with 7.5 µg of ABCA3-FLAG-PolyA^{∼}30 or ABCA3-FLAG-PolyA^{∼}100and harvested 6 hours post-transfection. Cells were scraped from the wells, pelleted, and the pellet was lysed in RIPA buffer. 75 µg of total protein was loaded on the gel. The blot was probed with M2 anti flag-HRP conjugated 1:10000 O/N, and developed using Femto Super signal substrate. Similar Western blot results have been obtained for MLE-15 cells.

Additional experiments demonstrating successful expression of ABC3A protein from transfected mRNA in A549 cells were conducted (data not shown). Briefly, A549 cells were transfected with mRNA encoding human ABC3A. 6 hours following transfection, cells were harvested and lysed, and ABC3A protein expression was detected by Western blot. For this experiment, codon optimized ABCA3- DNA having a CYBA 5' UTR and CYBA 3' UTR was used to transcribe the corresponding mRNAs *in vitro.*

### EXAMPLE 9: ABCA3 mRNA expression in vitro

The following experiment was conducted to compare the effect of incorporating specific chemically-modified nucleotides, in varying ratios and combinations, on translation efficiency in different cell types. The experiment described herein evaluated the translation *in vitro* from ABCA3 mRNAs (SEQ ID NO: 17) that had been prepared by *in vitro* transcription reactions comprising: 1) 50% Ψ; 2) 100% Ψ; 3) 25% s²U + 25% m⁵C (Mod 1); 4) 50% I⁵U (Mod 2); or 5) 35% I⁵U + 7.5% I⁵C (Mod 3). The ABCA3 mRNA template used in this experiment included a codon optimized open reading frame for ABCA3 in humans a polyA of about 30 A's in length, a cap1 structure, and it did not include a UTR (SEQ ID NO: 17).

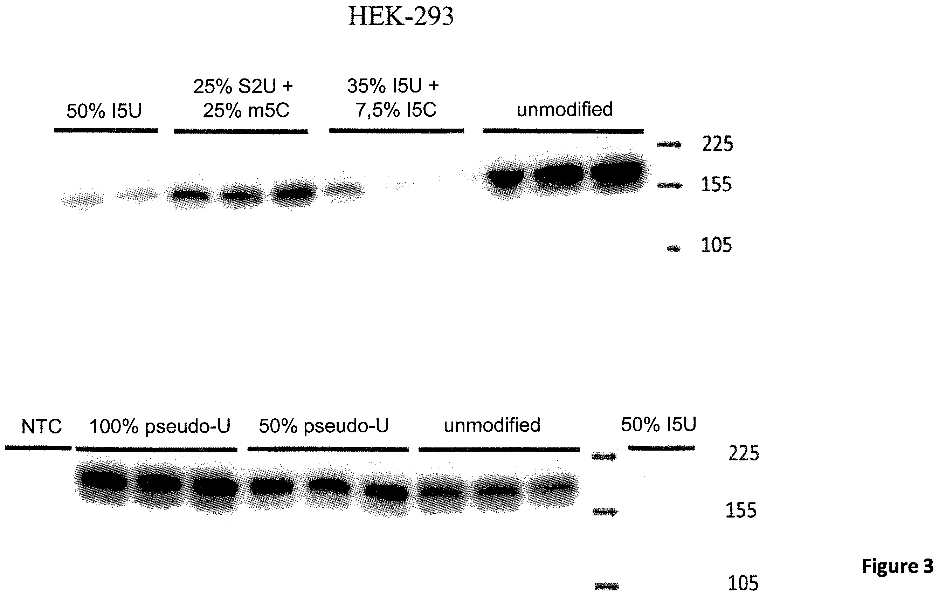

ABCA-FLAG mRNA expression from HEK-293 cells (biological triplicates): Briefly, 1 x 10⁶ A549 cells (biological triplicates) were transfected with 7.5 µg of ABCA3-FLAG /11.25 µl Messenger Max and harvested after 6 hrs. 50 µg of total protein was loaded on the gel. For ABCA3-FLAG detection: WB was probed with M2 anti flag-HRP conjugated 1:10000 overnight, and developed using Femto Super signal substrate. **FIGURE** 3 is a western blot illustrating protein expression from different ABCA-FLAG mRNAs transcribed by *in vitro* transcription reactions comprising: 1) 50% Ψ; 2) 100% Ψ; 3) 25% s²U + 25% m⁵C (Mod 1); 4) 50% I⁵U (Mod 2); 5) 35% I⁵U + 7.5% I⁵C (Mod 3); or a control in HEK-293 cells. As shown in FIGURE 3, NTC = mock transfected cells (no mRNA or plasmid).

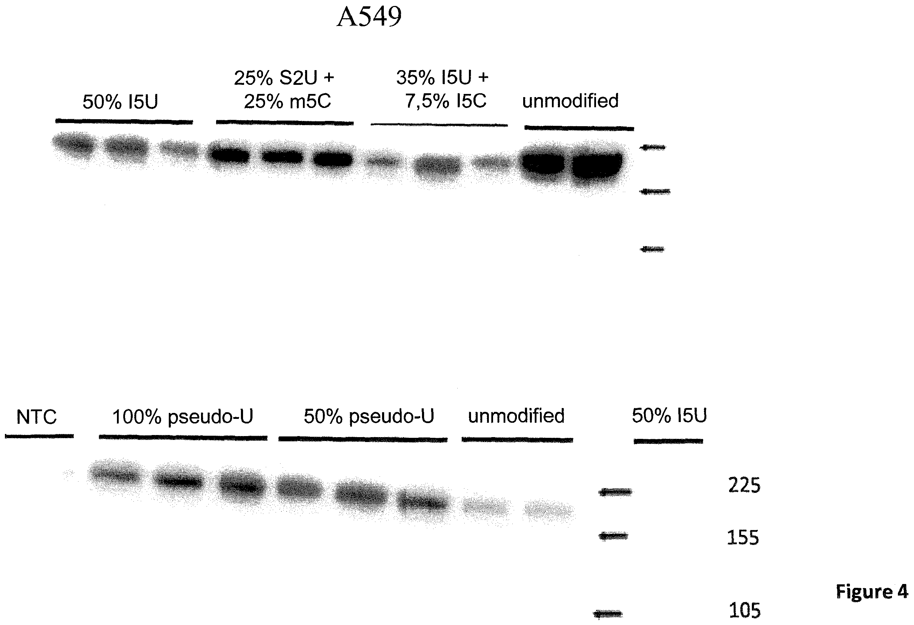

**FIGURE** 4 is a western blot illustrating the results of a similar experiment in A549 cells. **FIGURE 4** illustrates the protein expression from different ABCA-FLAG mRNAs transcribed by *in vitro* transcription reactions comprising: 1) 50% Ψ; 2) 100% Ψ; 3) 25% s²U + 25% m⁵C (Mod 1); 4) 50% I⁵U (Mod 2); or 5) 35% I⁵U + 7.5% I⁵C (Mod 3) in A549 cells.

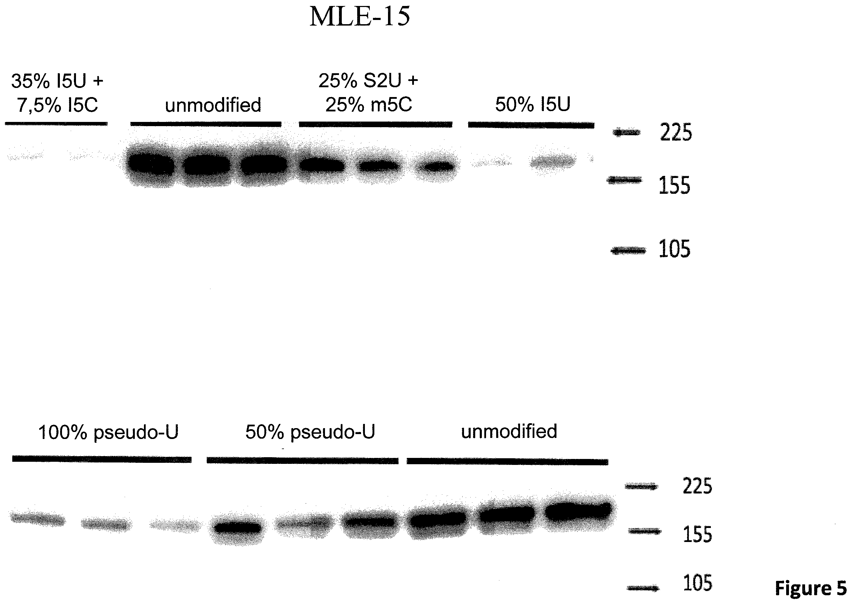

Similarly, **FIGURE 5** is a western blot illustrating the results of ABCA3 mRNA expression *in vitro* in MLE-15 cells. **FIGURE 5** illustrates the protein expression from different ABCA-FLAG mRNAs transcribed by *in vitro* transcription reactions comprising: 1) 50% Ψ; 2) 100% Ψ; 3) 25% s²U + 25% m⁵C (Mod 1); 4) 50% I⁵U (Mod 2); or 5) 35% I⁵U + 7.5% I⁵C (Mod 3) in MLE-15 cells.

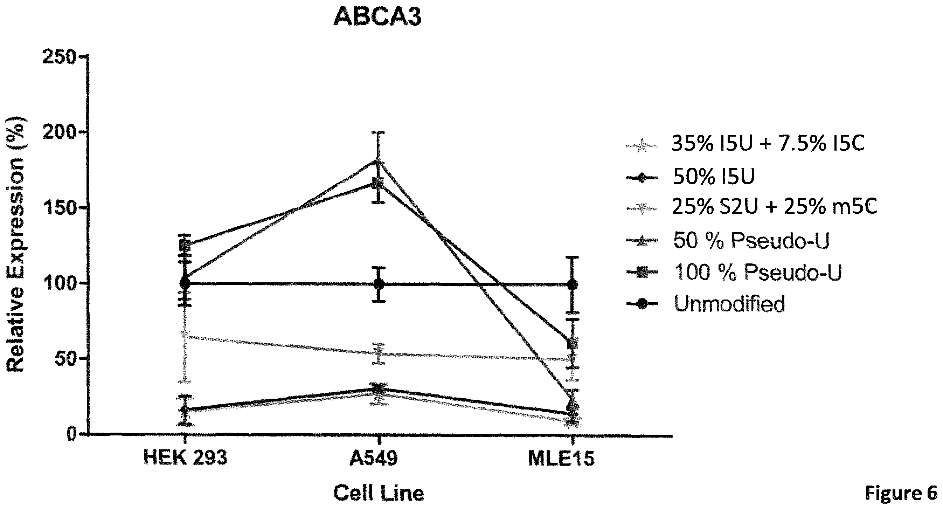

**FIGURE 6** illustrates a comparison of the ABCA3 protein expression levels measured by Western Blot 6 hours post mRNA transfection in HEK-293 cells, A549 cells, and MLE-15 cells. The results are normalized to total protein staining (SYPRO Ruby). Each data point is the mean of 3 biological (transfection) replicates, error bars +/- standard deviation. All ABCA3 mRNA shown on this figure have the same sequence and were made from the same plasmid. The ABCA3 mRNA template used in this experiment included no UTRs, it had a codon optimized open reading frame for ABCA3, a polyA of about 30 A's in length, and a cap1 structure.

### EXAMPLE 10: Immunogenicity of ABCA3 mRNAs in vitro

The immunogenicity of the aforementioned ABCA-FLAG mRNAs was tested in two cell lines by measuring cytokine production, namely, A549 adenocarcinomic human alveolar basal epithelial cells and HepG2 human liver carcinoma cells. Production of IL-6 in response to the transcripts was measured in A549 cells, while production of IP-10 was measured in HepG2 cells. Each cell line was transfected in triplicate with a titration of each RNA. Briefly, either 20,000 (A549) or 40,000 (HepG2) cells per well were plated 24 hours prior to transfection in 96 well plates. The cells were then transfected with a titration of each transcript, from 250 ng to 7 ng per well, using MessengerMax reagent at a RNA:MessengerMax ration of 1:1.5.

Culture supernatants were harvested at 18 hours post-transfection. Cell viability was measured immediately following supernatant removal using the CellTiter-Glo assay kit (Promega) which measures ATP levels as an indication of metabolically active cells. For IL-6 detection, A549 cell culture supernatants were diluted 1:20 in assay buffer and IL-6 levels were measured using the IL-6 High Sensitivity Human ELISA kit (Abcam ab46042). IP-10 was detected in undiluted HepG2 cell culture supernatants using the Human IP-10 ELISA Kit SimpleStep (Abcam ab173194).

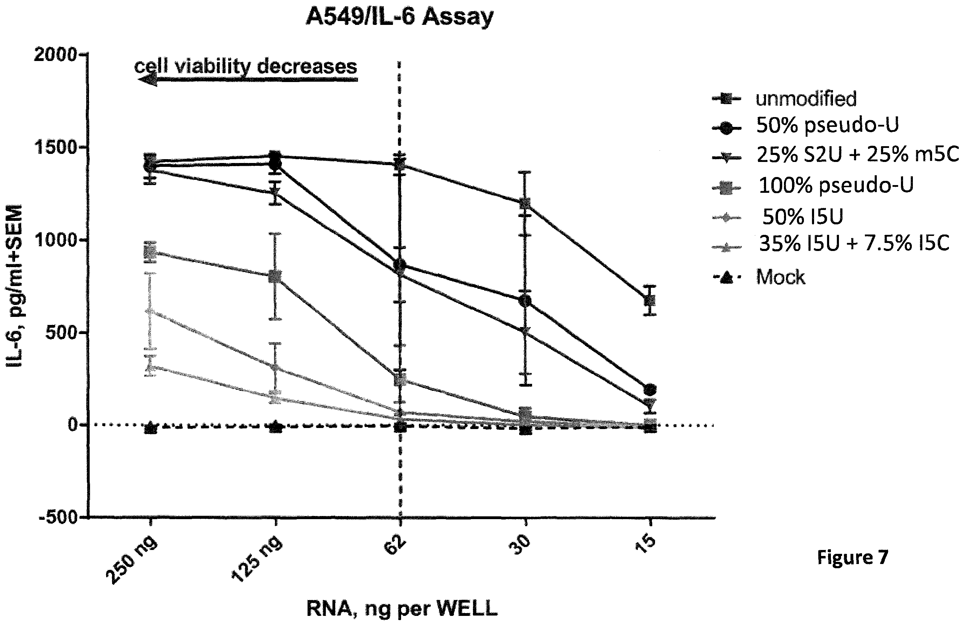

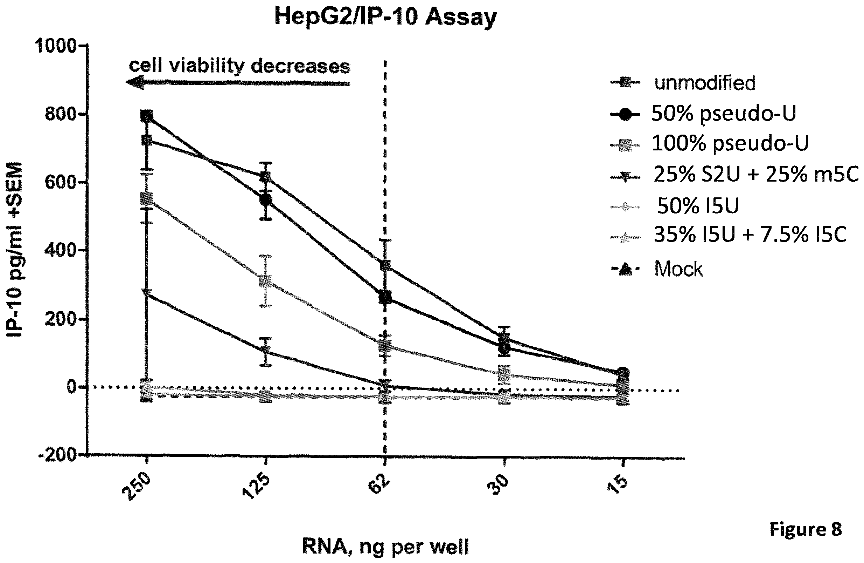

**FIGURE 7** illustrates the induction of IL-6 in A549 cells translating the ABCA3 protein from ABCA3-FLAG mRNAs comprising various nucleotide modifications. IL-6 expression was induced by various amounts of each ABCA-FLAG mRNA measured by ELISA. **FIGURE 7** illustrates cell viability after transfection with various amounts of each ABCA-FLAG mRNA measured using the CellTiter-Glo assay. **FIGURE 8** illustrates induction of IP-10 in HepG2 cells translating the ABCA3 protein from ABCA3-FLAG mRNAs comprising various nucleotide modifications. IP-10 expression was induced by various amounts of each ABCA-FLAG mRNA measured by ELISA. **FIGURE 8** illustrates cell viability after transfection with various amounts of each DNAI1 mRNA measured using the CellTiter-Glo assay.

### EXAMPLE 11: Cell Viability and Cytokine Induction

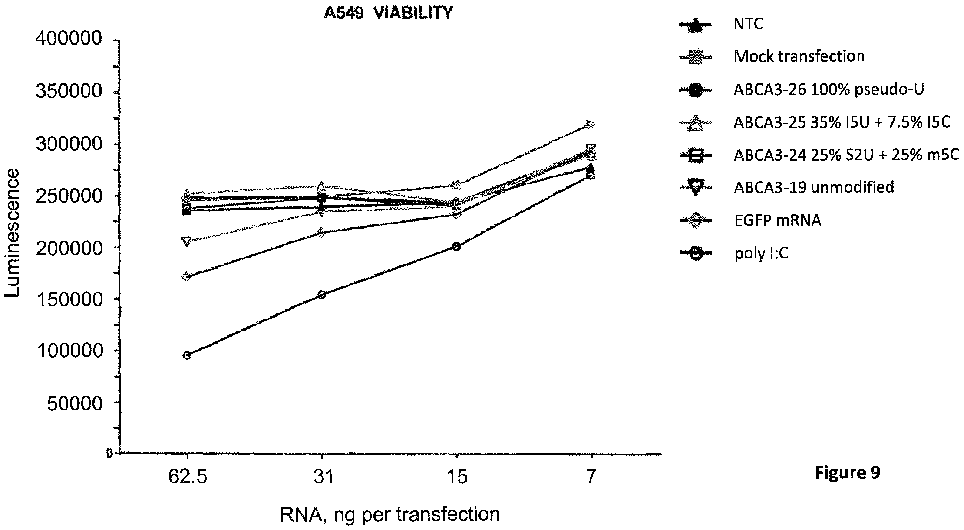

**FIGURES 9-12** illustrate the results of a Cell Viability and Cytokine induction *in vitro.*

**Method:** either A549 cells (2 x 10⁴/well) or HEPG2 cells (4 X 10⁴/well) were plated 24 hr prior to transfection on 96 well plates. A transfection was performed with messenger MAX, with an RNA to transfection reagent ratio of 1:1.5. This experiment use the following controls: Positive controls: 1. poly I:C was used as snim and transfected at the same concentrations; and 2. mRNA eGFP (trilink). Negative controls: 1. A549/HEP G2 treated with Opti Mem+Max reagent mix; and 2. A549/ HEP G2 cells untreated. Biological duplicates were plated on 3 different 96 well plates.

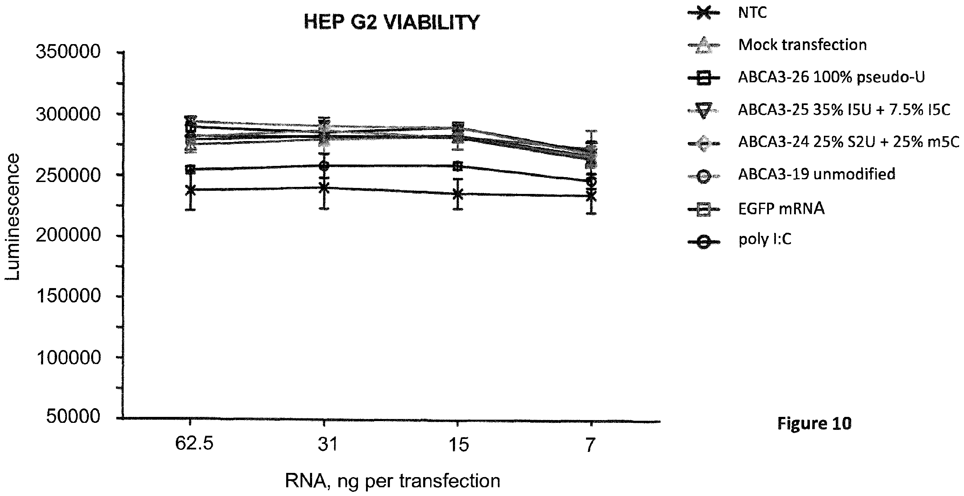

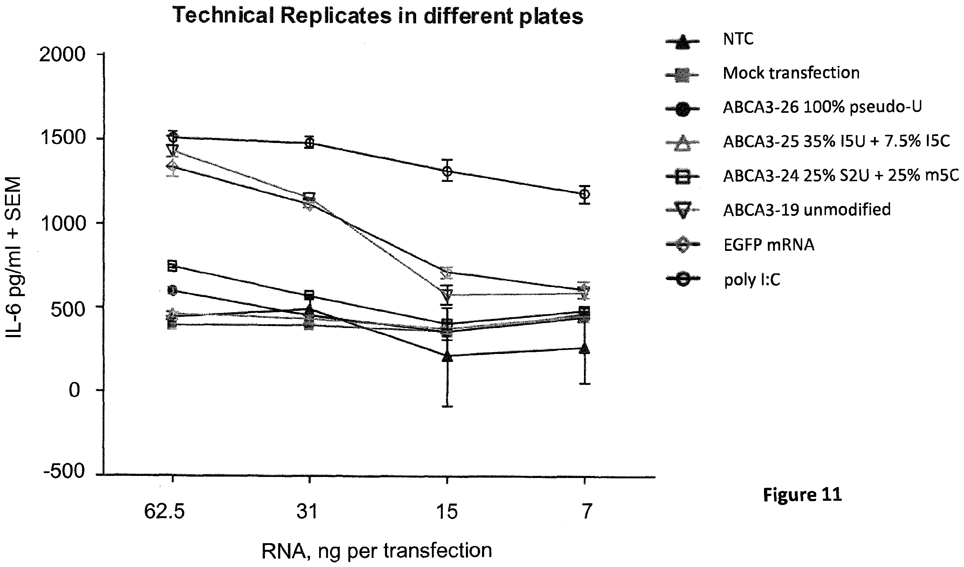

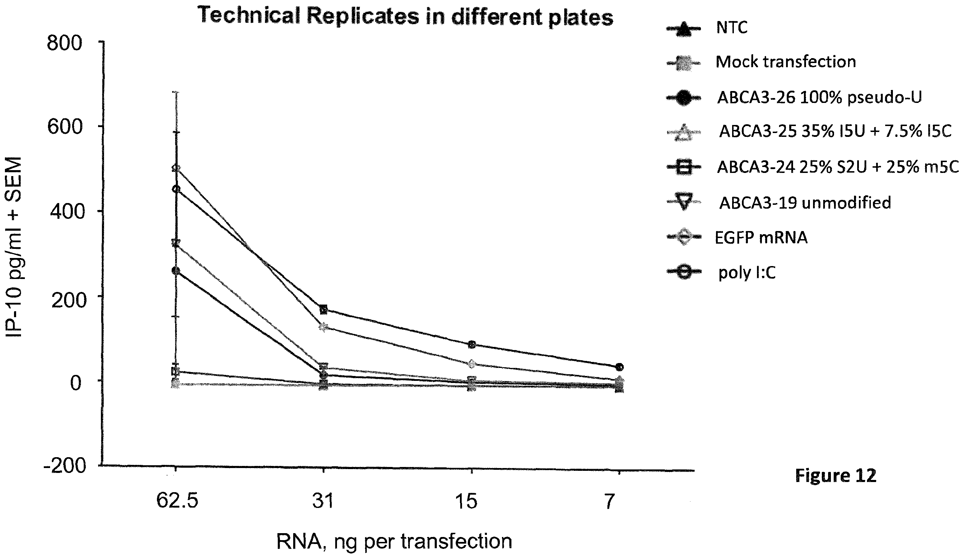

The supernatant was harvested 18 hr post transfection and cell viability was assessed immediately after supernatant removal. **FIGURE 9** and **FIGURE 10** illustrate the cell viability of the A549 cells and HEP G2 respectively. **FIGURE 11** and **FIGURE 12** illustrate the results of the ELISA assays measuring the levels of IL-6 and IP-10. For IL-6 ELISA, the sample was diluted in assay buffer was 1:20 throughout the assay. For IP-10 an undiluted sample was tested.

### EXAMPLE 12: Time Course for Translation of ABCA3 mRNA in Lung Cells

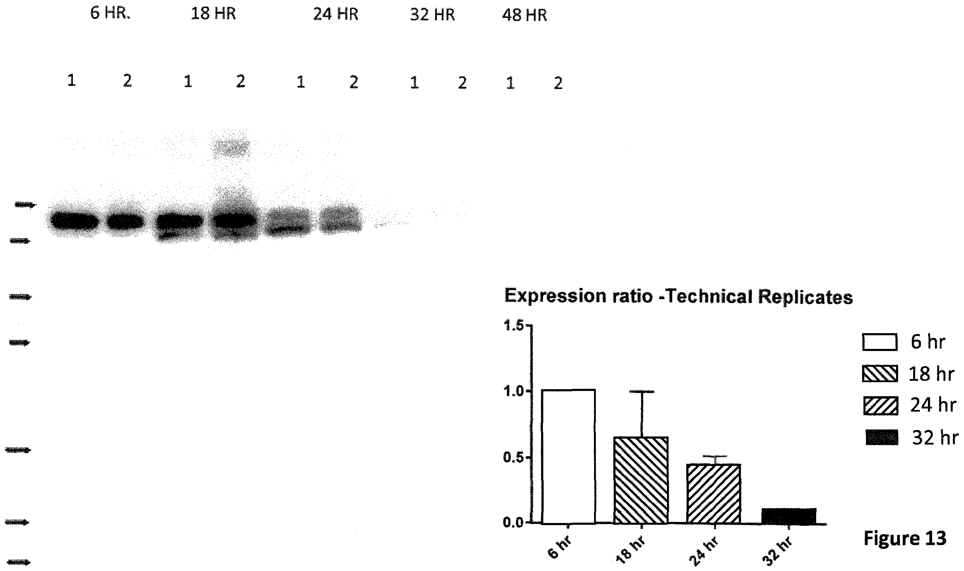

1 x 10⁶ A549 cells (human lung cells), passage 10, were transfected with 7.5 ug of RNA/11.5 Messenger Max in duplicates. The transfection media was changed at 4 hr post transfection and cells were harvested at different time points post transfection: 6 hr , 18 hr, 24 hr, 32 hr and 48 hr. The cells were subsequently washed with PBS and trypsinized for 5 min at room temperature. The trypsin reaction was stopped by adding FBS containing media, cells were collected, centrifuged and the pellet was washed twice with cold PBS. The pellet was then frozen. Once all the samples were collected, the frozen pellets were thawed on ice and lysed in RIPA buffer according to TTX protocol. 50 ug of protein was added to SDS-PAGE. **FIGURE 13** is a western blot illustrating the time-course of translation of the ABCA3 polypeptide expressed from SEQ ID NO: 17 in lung cells in vitro. For ABCA3 -FLAG detection :WB was probed with M2 anti flag-HRP conjugated 1:10000 O/N , and developed using Femto Super signal substrate.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

Exemplary sequences described in the application are provided below. The disclosure provides, in some embodiments, polynucleotides comprising, for example, the sequence set forth in SEQ ID NO: 17, 18, or 19, or a sequence at least 95%, 96%, 97%, 98%,or 99% identical to such sequences, or a polyribonucleotide sequence, such as an mRNA, corresponding to or encoded by any of the foregoing. In some embodiments, the disclosure provides polynucleotides comprising the sequence set forth in SEQ ID NO: 17 or 18, but in the absence of a FLAG and/or myc tag. In some embodiments, a 5'-UTR for use as part of a polynucleotide that encodes an ABC family member, comprises an untranslated region derived from a cytochrome b-245 alpha polypeptide gene or an untranslated region derived from an alpha-globin polypeptide gene, such as from a human gene. In some embodiments, a 5'-UTR for use as part of a polynucleotide that encodes an ABC family member, comprises the sequence set forth in SEQ ID NO: 5 or SEQ ID NO: 16. In certain embodiments of any of the foregoing, the polynucleotide or polyribonucleotide is modified (e.g., comprises nucleotide analogues, as described herein).

### LISTING OF SEQUENCES

**1) SEQ ID NO: 1**
   Summary: CYBA 5'
   CGCGCCTAGCAGTGTCCCAGCCGGGTTCGTGTCGCC
**2) SEQ ID NO: 2**
   Summary: CYBA 3' 
CCTCGCCCCGGACCTGCCCTCCCGCCAGGTGCACCCACCTGCAATAAATGCAGCGAA
GCCGGGA
**3) SEQ ID NO: 3**
   Summary: α-globin 5' UTR (HBA1) 
CATAAACCCTGGCGCGCTCGCGGCCCGGCACTCTTCTGGTCCCCACAGACTCAGAGA
GAACCCACC
**4) SEQ ID NO: 4**
   Summary: α-globin 5' UTR (HBA2) 
CATAAACCCTGGCGCGCTCGCGGCCGGCACTCTTCTGGTCCCCACAGACTCAGAGA
GAACCCACC (SEQ ID NO: 4)
**5) SEQ ID NO: 5**
   Summary: α -globin 5' UTR ETH
   TCTTCTGGTCCCCACAGACTCAGAGAGAAC
**6) SEQ ID NO: 6**
   Summary: ABCA3 5' 
GCGGCCGCTGCGTCCGCCAGTAGCGGGTTGCAGGCGCACCCTCCCCTCCAGGGCG
GCCACGCAGCTGTCAGTGCCGCCGCCACTGCGAGGCTGGAGCGGAGCCCGGGTGG
CCGAGGGAGGGGACCCCGCGAGAGGCCGCGCCGGCCGCCGCCCCGGCG
CCCAGGCTCGGTGCTGGAGAGTCATGCCTGTGAGCCCTGGGCACCTCCTGATGTCCT
GCGAGGTCACGGTGTTCCCAAACCTCAGGGTTGCCCTGCCCCACTCCAGAGGCTCTC
AGGCCCCACCCCGGAGCCCTCTGTGCGGAGCCGCCTCCTCCTGGCCAGTTCCCCAG
TAGTCCTGAAGGGAGACCTGCTGTGTGGAGCCTCTTCTGGGACCCAGCCATGAGTGT
GGAGCTGAGCAACTGAACCTGAAACTCTTCCACTGTGAGTCAAGGAGGCTTTTCCGCA
CATGAAGGACGCTGAGCGGGAAGGACTCCTCTCTGCCTGCAGTTGTAGCGAGTGGAC
CAGCACCAGGGGCTCTCTAGACTGCCCCTCCTCCATCGCCTTCCCTGCCTCTCCAGG
ACAGAGCAGCCACGTCTGCACACCTCGCCCTCTTTACACTCAGTTTTCAGAGCACGTT
TCTCCTATTTCCTGCGGGTTGCAGCGCCTACTTGAACTTACTCAGACCACCTACTTCTC
TAGCAGCACTGGGCGTCCCTTTCAGCAAGACG
**7) SEQ ID NO: 7**
   Summary: ABCA3 5' 
GGGGTGGCGGCTGTCTCGCCATCAGGCAGGGACAGGACGGGCAAGCAGGGCCCAT
CTTACATCCTCTCTCTCCAAGTTTATCTCATCCTTTATTTTTAATCACTTTTTTCTATGAT
GGATATGAAAAATTCAAGGCAGTATGCACAGAATGGACGAGTGCAGCCCAGCCCTCA
TGCCCAGGATCAGCATGCGCATCTCCATGTCTGCATACTCTGGAGTTCACTTTCCCAG
AGCTGGGGCAGGCCGGGCAGTCTGCGGGCAAGCTCCGGGGTCTCTGGGTGGAGAG
CTGACCCAGGAAGGGCTGCAGCTGAGCTGGGGGTTGAATTTCTCCAGGCACTCCCTG
GAGAGAGGACCCAGTGACTTGTCCAAGTTTACACACGACACTAATCTCCCCTGGGGA
GGAAGCGGAAGCCAGCCAGGTTGAACTGTAGCGAGGCCCCCAGGCCGCCAGGAAT
GGACCATGCAGATCACTGTCAGTGGAGGGAAGCTGCTGACTGTGATTAGGTGCTGGG
GTCTTAGCGTCCAGCGCAGCCCGGGGGCATCCTGGAGGCTCTGCTCCTTAGGGCAT
GGTAGTCACCGCGAAGCCGGGCACCGTCCCACAGCATCTCCTAGAAGCAGCCGGCA
CAGGAGGGAAGGTGGCCAGGCTCGAAGCAGTCTCTGTTTCCAGCACTGCACCCTCAG
GAAGTCGCCCGCCCCAGGACACGCAGGGACCACCCTAAGGGCTGGGTGGCTGTCTC
AAGGACACATTGAATACGTTGTGACCATCCAGAAAATAAATGCTGAGGGGACACAGTC
**8) SEQ ID NO: 8**
   Summary: ABCA3 mRNA 
AUGGCUGUGCUCAGGCAGCUGGCGCUCCUCCUCUGGAAGAACUACACCCUGCAGA
AGCGGAAGGUCCUGGUGACGGUCCUGGAACUCUUCCUGCCAUUGCUGUUUUCUGG
GAUCCUCAUCUGGCUCCGCUUGAAGAUUCAGUCGGAAAAUGUGCCCAACGCCACCA
UCUACCCGGGCCAGUCCAUCCAGGAGCUGCCUCUGUUCUUCACCUUCCCUCCGCC
AGGAGACACCUGGGAGCUUGCCUACAUCCCUUCUCACAGUGACGCUGCCAAGACC
GUCACUGAGACAGUGCGCAGGGCACUUGUGAUCAACAUGCGAGUGCGCGGCUUUC
CCUCCGAGAAGGACUUUGAGGACUACAUUAGGUACGACAACUGCUCGUCCAGCGU
GCUGGCCGCCGUGGUCUUCGAGCACCCCUUCAACCACAGCAAGGAGCCCCUGCCG
CUGGCGGUGAAAUAUCACCUACGGUUCAGUUACACACGGAGAAUUACAUGUGGAC
CCAAACAGGCUCCUUUUUCCUGAAAGAGACAGAAGGCUGGCACACUACUUCCCUUU
UCCCGCUUUUCCAAACCCAGGACCAAGGGAACCUACAUCCCCUGAUGGCGGAGAA
CCUGGGUACAUCCGGGAAGGCUUCCUGGCCGUGCAGCAUGCUGUGGACCGGGCCA
UCAUGGAGUACCAUGCCGAUGCCGCCACACGCCAGCUGUUCCAGAGACUGACGGU
GACCAUCAAGAGGUUCCCGUACCCGCCGUUCAUCGCAGACCCCUUCCUCGUGGCC
AUCCAGUACCAGCUGCCCCUGCUGCUGCUGCUCAGCUUCACCUACACCGCGCUCA
CCAUUGCCCGUGCUGUCGUGCAGGAGAAGGAAAGGAGGCUGAAGGAGUACAUGCG
CAUGAUGGGGCUCAGCAGCUGGCUGCACUGGAGUGCCUGGUUCCUCUUGUUCUUC
CUCUUCCUCCUCAUCGCCGCCUCCUUCAUGACCCUGCUCUUCUGUGUCAAGGUGA
AGCCAAAUGUAGCCGUGCUGUCCCGCAGCGACCCCUCCCUGGUGCUCGCCUUCCU
GCUGUGCUUCGCCAUCUCUACCAUCUCCUUCAGCUUCAUGGUCAGCACCUUCUUCA
GCAAAGCCAACAUGGCAGCAGCCUUCGGAGGCUUCCUCUACUUCUUCACCUACAUC
CCCUACUUCUUCGUGGCCCCUCGGUACAACUGGAUGACUCUGAGCCAGAAGCUCU
GCUCCUGCCUCCUGUCUAAUGUCGCCAUGGCAAUGGGAGCCCAGCUCAUUGGGAA
AUUUGAGGCGAAAGGCAUGGGCAUCCAGUGGCGAGACCUCCUGAGUCCCGUCAAC
GUGGACGACGACUUCUGCUUCGGGCAGGUGCUGGGGAUGCUGCUGCUGGACUCU
GUGCUCUAUGGCCUGGUGACCUGGUACAUGGAGGCCGUCUUCCCAGGGCAGUUCG
GCGUGCCUCAGCCCUGGUACUUCUUCAUCAUGCCCUCCUAUUGGUGUGGGAAGCC
AAGGGCGGUUGCAGGGAAGGAGGAAGAAGACAGUGACCCCGAGAAAGCACUCAGAA
ACGAGUACUUUGAAGCCGAGCCAGAGGACCUGGUGGCGGGGAUCAAGAUCAAGCA
CCUGUCCAAGGUGUUCAGGGUGGGAAAUAAGGACAGGGCGGCCGUCAGAGACCUG
AACCUCAACCUGUACGAGGGACAGAUCACCGUCCUGCUGGGCCACAACGGUGCCG
GGAAGACCACCACCCUCUCCAUGCUCACAGGUCUCUUUCCCCCCACCAGUGGACGG
GCAUACAUCAGCGGGUAUGAAAUUUCCCAGGACAUGGUUCAGAUCCGGAAGAGCCU
GGGCCUGUGCCCGCAGCACGACAUCCUGUUUGACAACUUGACAGUCGCAGAGCAC
CUUUAUUUCUACGCCCAGCUGAAGGGCCUGUCACGUCAGAAGUGCCCUGAAGAAG
UCAAGCAGAUGCUGCACAUCAUCGGCCUGGAGGACAAGUGGAACUCACGGAGCCG
CUUCCUGAGCGGGGGCAUGAGGCGCAAGCUCUCCAUCGGCAUCGCCCUCAUCGCA
GGCUCCAAGGUGCUGAUACUGGACGAGCCCACCUCGGGCAUGGACGCCAUCUCCA
GGAGGGCCAUCUGGGAUCUUCUUCAGCGGCAGAAAAGUGACCGCACCAUCGUGCU
GACCACCCACUUCAUGGACGAGGCUGACCUGCUGGGAGACCGCAUCGCCAUCAUG
GCCAAGGGGGAGCUGCAGUGCUGCGGGUCCUCGCUGUUCCUCAAGCAGAAAUACG
GUGCCGGCUAUCACAUGACGCUGGUGAAGGAGCCGCACUGCAACCCGGAAGACAU
CUCCCAGCUGGUCCACCACCACGUGCCCAACGCCACGCUGGAGAGCAGCGCUGGG
GCCGAGCUGUCUUUCAUCCUUCCCAGAGAGAGCACGCACAGGUUUGAAGGUCUCU
UUGCUAAACUGGAGAAGAAGCAGAAAGAGCUGGGCAUUGCCAGCUUUGGGGCAUC
CAUCACCACCAUGGAGGAAGUCUUCCUUCGGGUCGGAAGCUGGUGGACAGCAGU
AUGGACAUCCAGGCCAUCCAGCUCCCUGCCCUGCAGUACCAGCACGAGAGGCGCG
 
CCAGCGACUGGGCUGUGGACAGCAACCUCUGUGGGGCCAUGGACCCCUCCGACGG
CAUUGGAGCCCUCAUCGAGGAGGAGCGCACCGCUGUCAAGCUCAACACUGGGCUC
GCCCUGCACUGCCAGCAAUUCUGGGCCAUGUUCCUGAAGAAGGCCGCAUACAGCU
GGCGCGAGUGGAAAAUGGUGGCGGCACAGGUCCUGGUGCCUCUGACCUGCGUCAC
CCUGGCCCUCCUGGCCAUCAACUACUCCUCGGAGCUCUUCGACGACCCCAUGCUG
AGGCUGACCUUGGGCGAGUACGGCAGAACCGUCGUGCCCUUCUCAGUUCCCGGGA
CCUCCCAGCUGGGUCAGCAGCUGUCAGAGCAUCUGAAAGACGCACUGCAGGCUGA
GGGACAGGAGCCCCGCGAGGUGCUCGGUGACCUGGAGGAGUUCUUGAUCUUCAGG
GCUUCUGUGGAGGGGGGCGGCUUUAAUGAGCGGUGCCUUGUGGCAGCGUCCUUC
AGAGAUGUGGGAGAGCGCACGGUCGUCAACGCCUUGUUCAACAACCAGGCGUACC
ACUCUCCAGCCACUGCCCUGGCCGUCGUGGACAACCUUCUGUUCAAGCUGCUGUG
CGGGCCUCACGCCUCCAUUGUGGUCUCCAACUUCCCCCAGCCCCGGAGCGCCCUG
CAGGCUGCCAAGGACCAGUUUAACGAGGGCCGGAAGGGAUUCGACAUUGCCCUCA
ACCUGCUCUUCGCCAUGGCAUUCUUGGCCAGCACGUUCUCCAUCCUGGCGGUCAG
CGAGAGGGCCGUGCAGGCCAAGCAUGUGCAGUUUGUGAGUGGAGUCCACGUGGCC
AGUUUCUGGCUCUCUGCUCUGCUGUGGGACCUCAUCUCCUUCCUCAUCCCCAGUC
UGCUGCUGCUGGUGGUGUUUAAGGCCUUCGACGUGCGUGCCUUCACGCGGGACG
GCCACAUGGCUGACACCCUGCUGCUGCUCCUGCUCUACGGCUGGGCCAUCAUCCC
CCUCAUGUACCUGAUGAACUUCUUCUUCUUGGGGGCGGCCACUGCCUACACGAGG
CUGACCAUCUUCAACAUCCUGUCAGGCAUCGCCACCUUCCUGAUGGUCACCAUCAU
GCGCAUCCCAGCUGUAAAACUGGAAGAACUUUCCAAAACCCUGGAUCACGUGUUCC
UGGUGCUGCCCAACCACUGUCUGGGGAUGGCAGUCAGCAGUUUCUACGAGAACUA
CGAGACGCGGAGGUACUGCACCUCCUCCGAGGUCGCCGCCCACUACUGCAAGAAA
UAUAACAUCCAGUACCAGGAGAACUUCUAUGCCUGGAGCGCCCCGGGGGUCGGCC
GGUUUGUGGCCUCCAUGGCCGCCUCAGGGUGCGCCUACCUCAUCCUGCUCUUCCU
CAUCGAGACCAACCUGCUUCAGAGACUCAGGGGCAUCCUCUGCGCCCUCCGGAGG
AGGCGGACACUGACAGAAUUAUACACCCGGAUGCCUGUGCUUCCUGAGGACCAAGA
UGUAGCGGACGAGAGGACCCGCAUCCUGGCCCCCAGCCCGGACUCCCUGCUCCAC
ACACCUCUGAUUAUCAAGGAGCUCUCCAAGGUGUACGAGCAGCGGGUGCCCCUCC
UGGCCGUGGACAGGCUCUCCCUCGCGGUGCAGAAAGGGGAGUGCUUCGGCCUGC
UGGGCUUCAAUGGAGCCGGGAAGACCACGACUUUCAAAAUGCUGACCGGGGAGGA
GAGCCUCACUUCUGGGGAUGCCUUUGUCGGGGGUCACAGAAUCAGCUCUGAUGUC
GGAAAGGUGCGGCAGCGGAUCGGCUACUGCCCGCAGUUUGAUGCCUUGCUGGACC
ACAUGACAGGCCGGGAGAUGCUGGUCAUGUACGCUCGGCUCCGGGGCAUCCCUGA
GCGCCACAUCGGGGCCUGCGUGGAGAACACUCUGCGGGGCCUGCUGCUGGAGCCA
CAUGCCAACAAGCUGGUCAGGACGUACAGUGGUGGUAACAAGCGGAAGCUGAGCA
CCGGCAUCGCCCUGAUCGGAGAGCCUGCUGUCAUCUUCCUGGACGAGCCGUCCAC
UGGCAUGGACCCCGUGGCCCGGCGCCUGCUUUGGGACACCGUGGCACGAGCCCGA
GAGUCUGGCAAGGCCAUCAUCAUCACCUCCCACAGCAUGGAGGAGUGUGAGGCCC
UGUGCACCCGGCUGGCCAUCAUGGUGCAGGGGCAGUUCAAGUGCCUGGGCAGCCC
CCAGCACCUCAAGAGCAAGUUCGGCAGCGGCUACUCCCUGCGGGCCAAGGUGCAG
AGUGAAGGGCAACAGGAGGCGCUGGAGGAGUUCAAGGCCUUCGUGGACCUGACCU
UUCCAGGCAGCGUCCUGGAAGAUGAGCACCAAGGCAUGGUCCAUUACCACCUGCC
GGGCCGUGACCUCAGCUGGGCGAAGGUUUUCGGUAUUCUGGAGAAAGCCAAGGAA
AAGUACGGCGUGGACGACUACUCCGUGAGCCAGAUCUCGCUGGAACAGGUCUUCC
UGAGCUUCGCCCACCUGCAGCCGCCCACCGCAGAGGAGGGGCGA
**9) SEQ ID NO: 9**
   Summary: ABCA3 DNA ORF (Wild-Type) 
ATGGCTGTGCTCAGGCAGCTGGCGCTCCTCCTCTGGAAGAACTACACCCTGCAGAAG
CGGAAGGTCCTGGTGACGGTCCTGGAACTCTTCCTGCCATTGCTGTTTTCTGGGATCC
TCATCTGGCTCCGCTTGAAGATTCAGTCGGAAAATGTGCCCAACGCCACCATCTACCC
GGGCCAGTCCATCCAGGAGCTGCCTCTGTTCTTCACCTTCCCTCCGCCAGGAGACAC
CTGGGAGCTTGCCTACATCCCTTCTCACAGTGACGCTGCCAAGACCGTCACTGAGAC
AGTGCGCAGGGCACTTGTGATCAACATGCGAGTGCGCGGCTTTCCCTCCGAGAAGGA
CTTTGAGGACTACATTAGGTACGACAACTGCTCGTCCAGCGTGCTGGCCGCCGTGGT
CTTCGAGCACCCCTTCAACCACAGCAAGGAGCCCCTGCCGCTGGCGGTGAAATATCA
CCTACGGTTCAGTTACACACGGAGAAATTACATGTGGACCCAAACAGGCTCCTTTTTC
CTGAAAGAGACAGAAGGCTGGCACACTACTTCCCTTTTCCCGCTTTTCCCAAACCCAG
GACCAAGGGAACCTACATCCCCTGATGGCGGAGAACCTGGGTACATCCGGGAAGGCT
TCCTGGCCGTGCAGCATGCTGTGGACCGGGCCATCATGGAGTACCATGCCGATGCCG
CCACACGCCAGCTGTTCCAGAGACTGACGGTGACCATCAAGAGGTTCCCGTACCCGC
CGTTCATCGCAGACCCCTTCCTCGTGGCCATCCAGTACCAGCTGCCCCTGCTGCTGC
TGCTCAGCTTCACCTACACCGCGCTCACCATTGCCCGTGCTGTCGTGCAGGAGAAGG
AAAGGAGGCTGAAGGAGTACATGCGCATGATGGGGCTCAGCAGCTGGCTGCACTGG
AGTGCCTGGTTCCTCTTGTTCTTCCTCTTCCTCCTCATCGCCGCCTCCTTCATGACCCT
GCTCTTCTGTGTCAAGGTGAAGCCAAATGTAGCCGTGCTGTCCCGCAGCGACCCCTC
CCTGGTGCTCGCCTTCCTGCTGTGCTTCGCCATCTCTACCATCTCCTTCAGCTTCATG
GTCAGCACCTTCTTCAGCAAAGCCAACATGGCAGCAGCCTTCGGAGGCTTCCTCTACT
TCTTCACCTACATCCCCTACTTCTTCGTGGCCCCTCGGTACAACTGGATGACTCTGAG
CCAGAAGCTCTGCTCCTGCCTCCTGTCTAATGTCGCCATGGCAATGGGAGCCCAGCT
CATTGGGAAATTTGAGGCGAAAGGCATGGGCATCCAGTGGCGAGACCTCCTGAGTCC
CGTCAACGTGGACGACGACTTCTGCTTCGGGCAGGTGCTGGGGATGCTGCTGCTGGA
CTCTGTGCTCTATGGCCTGGTGACCTGGTACATGGAGGCCGTCTTCCCAGGGCAGTT
CGGCGTGCCTCAGCCCTGGTACTTCTTCATCATGCCCTCCTATTGGTGTGGGAAGCCA
AGGGCGGTTGCAGGGAAGGAGGAAGAAGACAGTGACCCCGAGAAAGCACTCAGAAA
CGAGTACTTTGAAGCCGAGCCAGAGGACCTGGTGGCGGGGATCAAGATCAAGCACCT
GTCCAAGGTGTTCAGGGTGGGAAATAAGGACAGGGCGGCCGTCAGAGACCTGAACC
TCAACCTGTACGAGGGACAGATCACCGTCCTGCTGGGCCACAACGGTGCCGGGAAG
ACCACCACCCTCTCCATGCTCACAGGTCTCTTTCCCCCCACCAGTGGACGGGCATACA
TCAGCGGGTATGAAATTTCCCAGGACATGGTTCAGATCCGGAAGAGCCTGGGCCTGT
GCCCGCAGCACGACATCCTGTTTGACAACTTGACAGTCGCAGAGCACCTTTATTTCTA
CGCCCAGCTGAAGGGCCTGTCACGTCAGAAGTGCCCTGAAGAAGTCAAGCAGATGCT
GCACATCATCGGCCTGGAGGACAAGTGGAACTCACGGAGCCGCTTCCTGAGCGGGG
GCATGAGGCGCAAGCTCTCCATCGGCATCGCCCTCATCGCAGGCTCCAAGGTGCTGA
TACTGGACGAGCCCACCTCGGGCATGGACGCCATCTCCAGGAGGGCCATCTGGGAT
CTTCTTCAGCGGCAGAAAAGTGACCGCACCATCGTGCTGACCACCCACTTCATGGAC
GAGGCTGACCTGCTGGGAGACCGCATCGCCATCATGGCCAAGGGGGAGCTGCAGTG
CTGCGGGTCCTCGCTGTTCCTCAAGCAGAAATACGGTGCCGGCTATCACATGACGCT
GGTGAAGGAGCCGCACTGCAACCCGGAAGACATCTCCCAGCTGGTCCACCACCACGT
GCCCAACGCCACGCTGGAGAGCAGCGCTGGGGCCGAGCTGTCTTTCATCCTTCCCAG
AGAGAGCACGCACAGGTTTGAAGGTCTCTTTGCTAAACTGGAGAAGAAGCAGAAAGA
GCTGGGCATTGCCAGCTTTGGGGCATCCATCACCACCATGGAGGAAGTCTTCCTTCG
GGTCGGGAAGCTGGTGGACAGCAGTATGGACATCCAGGCCATCCAGCTCCCTGCCCT
 
GCAGTACCAGCACGAGAGGCGCGCCAGCGACTGGGCTGTGGACAGCAACCTCTGTG
GGGCCATGGACCCCTCCGACGGCATTGGAGCCCTCATCGAGGAGGAGCGCACCGCT
GTCAAGCTCAACACTGGGCTCGCCCTGCACTGCCAGCAATTCTGGGCCATGTTCCTG
AAGAAGGCCGCATACAGCTGGCGCGAGTGGAAAATGGTGGCGGCACAGGTCCTGGT
GCCTCTGACCTGCGTCACCCTGGCCCTCCTGGCCATCAACTACTCCTCGGAGCTCTT
CGACGACCCCATGCTGAGGCTGACCTTGGGCGAGTACGGCAGAACCGTCGTGCCCT
TCTCAGTTCCCGGGACCTCCCAGCTGGGTCAGCAGCTGTCAGAGCATCTGAAAGACG
CACTGCAGGCTGAGGGACAGGAGCCCCGCGAGGTGCTCGGTGACCTGGAGGAGTTC
TTGATCTTCAGGGCTTCTGTGGAGGGGGGCGGCTTTAATGAGCGGTGCCTTGTGGCA
GCGTCCTTCAGAGATGTGGGAGAGCGCACGGTCGTCAACGCCTTGTTCAACAACCAG
GCGTACCACTCTCCAGCCACTGCCCTGGCCGTCGTGGACAACCTTCTGTTCAAGCTG
CTGTGCGGGCCTCACGCCTCCATTGTGGTCTCCAACTTCCCCCAGCCCCGGAGCGCC
CTGCAGGCTGCCAAGGACCAGTTTAACGAGGGCCGGAAGGGATTCGACATTGCCCTC
AACCTGCTCTTCGCCATGGCATTCTTGGCCAGCACGTTCTCCATCCTGGCGGTCAGC
GAGAGGGCCGTGCAGGCCAAGCATGTGCAGTTTGTGAGTGGAGTCCACGTGGCCAG
TTTCTGGCTCTCTGCTCTGCTGTGGGACCTCATCTCCTTCCTCATCCCCAGTCTGCTG
CTGCTGGTGGTGTTTAAGGCCTTCGACGTGCGTGCCTTCACGCGGGACGGCCACATG
GCTGACACCCTGCTGCTGCTCCTGCTCTACGGCTGGGCCATCATCCCCCTCATGTAC
CTGATGAACTTCTTCTTCTTGGGGGCGGCCACTGCCTACACGAGGCTGACCATCTTCA
ACATCCTGTCAGGCATCGCCACCTTCCTGATGGTCACCATCATGCGCATCCCAGCTGT
AAAACTGGAAGAACTTTCCAAAACCCTGGATCACGTGTTCCTGGTGCTGCCCAACCAC
TGTCTGGGGATGGCAGTCAGCAGTTTCTACGAGAACTACGAGACGCGGAGGTACTGC
ACCTCCTCCGAGGTCGCCGCCCACTACTGCAAGAAATATAACATCCAGTACCAGGAG
AACTTCTATGCCTGGAGCGCCCCGGGGGTCGGCCGGTTTGTGGCCTCCATGGCCGC
CTCAGGGTGCGCCTACCTCATCCTGCTCTTCCTCATCGAGACCAACCTGCTTCAGAGA
CTCAGGGGCATCCTCTGCGCCCTCCGGAGGAGGCGGACACTGACAGAATTATACACC
CGGATGCCTGTGCTTCCTGAGGACCAAGATGTAGCGGACGAGAGGACCCGCATCCTG
GCCCCCAGCCCGGACTCCCTGCTCCACACACCTCTGATTATCAAGGAGCTCTCCAAG
GTGTACGAGCAGCGGGTGCCCCTCCTGGCCGTGGACAGGCTCTCCCTCGCGGTGCA
GAAAGGGGAGTGCTTCGGCCTGCTGGGCTTCAATGGAGCCGGGAAGACCACGACTTT
CAAAATGCTGACCGGGGAGGAGAGCCTCACTTCTGGGGATGCCTTTGTCGGGGGTCA
CAGAATCAGCTCTGATGTCGGAAAGGTGCGGCAGCGGATCGGCTACTGCCCGCAGTT
TGATGCCTTGCTGGACCACATGACAGGCCGGGAGATGCTGGTCATGTACGCTCGGCT
CCGGGGCATCCCTGAGCGCCACATCGGGGCCTGCGTGGAGAACACTCTGCGGGGCC
TGCTGCTGGAGCCACATGCCAACAAGCTGGTCAGGACGTACAGTGGTGGTAACAAGC
GGAAGCTGAGCACCGGCATCGCCCTGATCGGAGAGCCTGCTGTCATCTTCCTGGACG
AGCCGTCCACTGGCATGGACCCCGTGGCCCGGCGCCTGCTTTGGGACACCGTGGCA
CGAGCCCGAGAGTCTGGCAAGGCCATCATCATCACCTCCCACAGCATGGAGGAGTGT
GAGGCCCTGTGCACCCGGCTGGCCATCATGGTGCAGGGGCAGTTCAAGTGCCTGGG
CAGCCCCCAGCACCTCAAGAGCAAGTTCGGCAGCGGCTACTCCCTGCGGGCCAAGG
TGCAGAGTGAAGGGCAACAGGAGGCGCTGGAGGAGTTCAAGGCCTTCGTGGACCTG
ACCTTTCCAGGCAGCGTCCTGGAAGATGAGCACCAAGGCATGGTCCATTACCACCTG
CCGGGCCGTGACCTCAGCTGGGCGAAGGTTTTCGGTATTCTGGAGAAAGCCAAGGAA
AAGTACGGCGTGGACGACTACTCCGTGAGCCAGATCTCGCTGGAACAGGTCTTCCTG
AGCTTCGCCCACCTGCAGCCGCCCACCGCAGAGGAGGGGCGA
**10)SEQ ID NO: 10**
   Summary: a native ABCA3 DNA sequence with a 5' CYBA UTR and a 3' CYBA UTR (SEQ ID NO: 10) 
CGCGCCTAGCAGTGTCCCAGCCGGGTTCGTGTCGCCGCCACCATGGCTGTGCTCAG
GCAGCTGGCGCTCCTCCTCTGGAAGAACTACACCCTGCAGAAGCGGAAGGTCCTGGT
GACGGTCCTGGAACTCTTCCTGCCATTGCTGTTTTCTGGGATCCTCATCTGGCTCCGC
TTGAAGATTCAGTCGGAAAATGTGCCCAACGCCACCATCTACCCGGGCCAGTCCATC
CAGGAGCTGCCTCTGTTCTTCACCTTCCCTCCGCCAGGAGACACCTGGGAGCTTGCC
TACATCCCTTCTCACAGTGACGCTGCCAAGACCGTCACTGAGACAGTGCGCAGGGCA
CTTGTGATCAACATGCGAGTGCGCGGCTTTCCCTCCGAGAAGGACTTTGAGGACTAC
ATTAGGTACGACAACTGCTCGTCCAGCGTGCTGGCCGCCGTGGTCTTCGAGCACCCC
TTCAACCACAGCAAGGAGCCCCTGCCGCTGGCGGTGAAATATCACCTACGGTTCAGT
TACACACGGAGAAATTACATGTGGACCCAAACAGGCTCCTTTTTCCTGAAAGAGACAG
AAGGCTGGCACACTACTTCCCTTTTCCCGCTTTTCCCAAACCCAGGACCAAGGGAACC
TACATCCCCTGATGGCGGAGAACCTGGGTACATCCGGAAGGCTTCCTGGCCGTGCA
GCATGCTGTGGACCGGGCCATCATGGAGTACCATGCCGATGCCGCCACACGCCAGC
TGTTCCAGAGACTGACGGTGACCATCAAGAGGTTCCCGTACCCGCCGTTCATCGCAG
ACCCCTTCCTCGTGGCCATCCAGTACCAGCTGCCCCTGCTGCTGCTGCTCAGCTTCA
CCTACACCGCGCTCACCATTGCCCGTGCTGTCGTGCAGGAGAAGGAAAGGAGGCTGA
AGGAGTACATGCGCATGATGGGGCTCAGCAGCTGGCTGCACTGGAGTGCCTGGTTCC
TCTTGTTCTTCCTCTTCCTCCTCATCGCCGCCTCCTTCATGACCCTGCTCTTCTGTGTC
AAGGTGAAGCCAAATGTAGCCGTGCTGTCCCGCAGCGACCCCTCCCTGGTGCTCGCC
TTCCTGCTGTGCTTCGCCATCTCTACCATCTCCTTCAGCTTCATGGTCAGCACCTTCTT
CAGCAAAGCCAACATGGCAGCAGCCTTCGGAGGCTTCCTCTACTTCTTCACCTACATC
CCCTACTTCTTCGTGGCCCCTCGGTACAACTGGATGACTCTGAGCCAGAAGCTCTGCT
CCTGCCTCCTGTCTAATGTCGCCATGGCAATGGGAGCCCAGCTCATTGGGAAATTTGA
GGCGAAAGGCATGGGCATCCAGTGGCGAGACCTCCTGAGTCCCGTCAACGTGGACG
ACGACTTCTGCTTCGGGCAGGTGCTGGGGATGCTGCTGCTGGACTCTGTGCTCTATG
GCCTGGTGACCTGGTACATGGAGGCCGTCTTCCCAGGGCAGTTCGGCGTGCCTCAG
CCCTGGTACTTCTTCATCATGCCCTCCTATTGGTGTGGGAAGCCAAGGGCGGTTGCA
GGGAAGGAGGAAGAAGACAGTGACCCCGAGAAAGCACTCAGAAACGAGTACTTTGAA
GCCGAGCCAGAGGACCTGGTGGCGGGGATCAAGATCAAGCACCTGTCCAAGGTGTT
CAGGGTGGGAAATAAGGACAGGGCGGCCGTCAGAGACCTGAACCTCAACCTGTACG
AGGGACAGATCACCGTCCTGCTGGGCCACAACGGTGCCGGGAAGACCACCACCCTC
TCCATGCTCACAGGTCTCTTTCCCCCCACCAGTGGACGGGCATACATCAGCGGGTAT
GAAATTTCCCAGGACATGGTTCAGATCCGGAAGAGCCTGGGCCTGTGCCCGCAGCAC
GACATCCTGTTTGACAACTTGACAGTCGCAGAGCACCTTTATTTCTACGCCCAGCTGA
AGGGCCTGTCACGTCAGAAGTGCCCTGAAGAAGTCAAGCAGATGCTGCACATCATCG
GCCTGGAGGACAAGTGGAACTCACGGAGCCGCTTCCTGAGCGGGGGCATGAGGCGC
AAGCTCTCCATCGGCATCGCCCTCATCGCAGGCTCCAAGGTGCTGATACTGGACGAG
CCCACCTCGGGCATGGACGCCATCTCCAGGAGGGCCATCTGGGATCTTCTTCAGCGG
CAGAAAAGTGACCGCACCATCGTGCTGACCACCCACTTCATGGACGAGGCTGACCTG
CTGGGAGACCGCATCGCCATCATGGCCAAGGGGGAGCTGCAGTGCTGCGGGTCCTC
GCTGTTCCTCAAGCAGAAATACGGTGCCGGCTATCACATGACGCTGGTGAAGGAGCC
GCACTGCAACCCGGAAGACATCTCCCAGCTGGTCCACCACCACGTGCCCAACGCCAC
GCTGGAGAGCAGCGCTGGGGCCGAGCTGTCTTTCATCCTTCCCAGAGAGAGCACGC
ACAGGTTTGAAGGTCTCTTTGCTAAACTGGAGAAGAAGCAGAAAGAGCTGGGCATTGC
 
CAGCTTTGGGGCATCCATCACCACCATGGAGGAAGTCTTCCTTCGGGTCGGGAAGCT
GGTGGACAGCAGTATGGACATCCAGGCCATCCAGCTCCCTGCCCTGCAGTACCAGCA
CGAGAGGCGCGCCAGCGACTGGGCTGTGGACAGCAACCTCTGTGGGGCCATGGACC
CCTCCGACGGCATTGGAGCCCTCATCGAGGAGGAGCGCACCGCTGTCAAGCTCAACA
CTGGGCTCGCCCTGCACTGCCAGCAATTCTGGGCCATGTTCCTGAAGAAGGCCGCAT
ACAGCTGGCGCGAGTGGAAAATGGTGGCGGCACAGGTCCTGGTGCCTCTGACCTGC
GTCACCCTGGCCCTCCTGGCCATCAACTACTCCTCGGAGCTCTTCGACGACCCCATG
CTGAGGCTGACCTTGGGCGAGTACGGCAGAACCGTCGTGCCCTTCTCAGTTCCCGGG
ACCTCCCAGCTGGGTCAGCAGCTGTCAGAGCATCTGAAAGACGCACTGCAGGCTGAG
GGACAGGAGCCCCGCGAGGTGCTCGGTGACCTGGAGGAGTTCTTGATCTTCAGGGC
TTCTGTGGAGGGGGGCGGCTTTAATGAGCGGTGCCTTGTGGCAGCGTCCTTCAGAGA
TGTGGGAGAGCGCACGGTCGTCAACGCCTTGTTCAACAACCAGGCGTACCACTCTCC
AGCCACTGCCCTGGCCGTCGTGGACAACCTTCTGTTCAAGCTGCTGTGCGGCCTCA
CGCCTCCATTGTGGTCTCCAACTTCCCCCAGCCCCGGAGCGCCCTGCAGGCTGCCAA
GGACCAGTTTAACGAGGGCCGGAAGGGATTCGACATTGCCCTCAACCTGCTCTTCGC
CATGGCATTCTTGGCCAGCACGTTCTCCATCCTGGCGGTCAGCGAGAGGGCCGTGCA
GGCCAAGCATGTGCAGTTTGTGAGTGGAGTCCACGTGGCCAGTTTCTGGCTCTCTGC
TCTGCTGTGGGACCTCATCTCCTTCCTCATCCCCAGTCTGCTGCTGCTGGTGGTGTTT
AAGGCCTTCGACGTGCGTGCCTTCACGCGGGACGGCCACATGGCTGACACCCTGCT
GCTGCTCCTGCTCTACGGCTGGGCCATCATCCCCCTCATGTACCTGATGAACTTCTTC
TTCTTGGGGGCGGCCACTGCCTACACGAGGCTGACCATCTTCAACATCCTGTCAGGC
ATCGCCACCTTCCTGATGGTCACCATCATGCGCATCCCAGCTGTAAAACTGGAAGAAC
TTTCCAAAACCCTGGATCACGTGTTCCTGGTGCTGCCCAACCACTGTCTGGGGATGGC
AGTCAGCAGTTTCTACGAGAACTACGAGACGCGGAGGTACTGCACCTCCTCCGAGGT
CGCCGCCCACTACTGCAAGAAATATAACATCCAGTACCAGGAGAACTTCTATGCCTGG
AGCGCCCCGGGGGTCGGCCGGTTTGTGGCCTCCATGGCCGCCTCAGGGTGCGCCTA
CCTCATCCTGCTCTTCCTCATCGAGACCAACCTGCTTCAGAGACTCAGGGGCATCCTC
TGCGCCCTCCGGAGGAGGCGGACACTGACAGAATTATACACCCGGATGCCTGTGCTT
CCTGAGGACCAAGATGTAGCGGACGAGAGGACCCGCATCCTGGCCCCCAGCCCGGA
CTCCCTGCTCCACACACCTCTGATTATCAAGGAGCTCTCCAAGGTGTACGAGCAGCG
GGTGCCCCTCCTGGCCGTGGACAGGCTCTCCCTCGCGGTGCAGAAAGGGGAGTGCT
TCGGCCTGCTGGGCTTCAATGGAGCCGGGAAGACCACGACTTTCAAAATGCTGACCG
GGGAGGAGAGCCTCACTTCTGGGGATGCCTTTGTCGGGGGTCACAGAATCAGCTCTG
ATGTCGGAAAGGTGCGGCAGCGGATCGGCTACTGCCCGCAGTTTGATGCCTTGCTGG
ACCACATGACAGGCCGGGAGATGCTGGTCATGTACGCTCGGCTCCGGGGCATCCCT
GAGCGCCACATCGGGGCCTGCGTGGAGAACACTCTGCGGGGCCTGCTGCTGGAGCC
ACATGCCAACAAGCTGGTCAGGACGTACAGTGGTGGTAACAAGCGGAAGCTGAGCAC
CGGCATCGCCCTGATCGGAGAGCCTGCTGTCATCTTCCTGGACGAGCCGTCCACTGG
CATGGACCCCGTGGCCCGGCGCCTGCTTTGGGACACCGTGGCACGAGCCCGAGAGT
CTGGCAAGGCCATCATCATCACCTCCCACAGCATGGAGGAGTGTGAGGCCCTGTGCA
CCCGGCTGGCCATCATGGTGCAGGGGCAGTTCAAGTGCCTGGGCAGCCCCCAGCAC
CTCAAGAGCAAGTTCGGCAGCGGCTACTCCCTGCGGGCCAAGGTGCAGAGTGAAGG
GCAACAGGAGGCGCTGGAGGAGTTCAAGGCCTTCGTGGACCTGACCTTTCCAGGCA
GCGTCCTGGAAGATGAGCACCAAGGCATGGTCCATTACCACCTGCCGGGCCGTGACC
TCAGCTGGGCGAAGGTTTTCGGTATTCTGGAGAAAGCCAAGGAAAAGTACGGCGTGG
ACGACTACTCCGTGAGCCAGATCTCGCTGGAACAGGTCTTCCTGAGCTTCGCCCACC
TGCAGCCGCCCACCGCAGAGGAGGGGCGAACGCGTACGCGACCGCTCGAGCAGAAA
CTCATCTCAGAAGAGGATCTGGCAGCAAATGATATCCTGGATTACAAGGATGACGACG
 
ATAAGGTTTGACCTCGCCCCGGACCTGCCCTCCCGCCAGGTGCACCCACCTGCAATA
AATGCAGCGAAGCCGGGAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAA
AAAAAAAAAAAAAAAAAAAAATT
**11)SEQ ID NO: 11**
   Summary: a native ABCA3 mRNA sequence with a 5' CYBA UTR and a 3' CYBA UTR (SEQ ID NO: 11) 
CGCGCCUAGCAGUGUCCCAGCCGGGUUCGUGUCGCCGCCACCAUGGCUGUGCUCA
GGCAGCUGGCGCUCCUCCUCUGGAAGAACUACACCCUGCAGAAGCGGAAGGUCCU
GGUGACGGUCCUGGAACUCUUCCUGCCAUUGCUGUUUCUGGGAUCCUCAUCUGG
CUCCGCUUGAAGAUUCAGUCGGAAAAUGUGCCCAACGCCACCAUCUACCCGGGCCA
GUCCAUCCAGGAGCUGCCUCUGUUCUUCACCUUCCCUCCGCCAGGAGACACCUGG
GAGCUUGCCUACAUCCCUUCUCACAGUGACGCUGCCAAGACCGUCACUGAGACAGU
GCGCAGGGCACUUGUGAUCAACAUGCGAGUGCGCGGCUUUCCCUCCGAGAAGGAC
UUUGAGGACUACAUUAGGUACGACAACUGCUCGUCCAGCGUGCUGGCCGCCGUGG
UCUUCGAGCACCCCUUCAACCACAGCAAGGAGCCCCUGCCGCUGGCGGUGAAAUAU
CACCUACGGUUCAGUUACACACGGAGAAAUUACAUGUGGACCCAAACAGGCUCCUU
UUUCCUGAAAGAGACAGAAGGCUGGCACACUACUUCCCUUUUCCCGCUUUUCCCAA
ACCCAGGACCAAGGGAACCUACAUCCCCUGAUGGCGGAGAACCUGGGUACAUCCG
GGAAGGCUUCCUGGCCGUGCAGCAUGCUGUGGACCGGGCCAUCAUGGAGUACCAU
GCCGAUGCCGCCACACGCCAGCUGUUCCAGAGACUGACGGUGACCAUCAAGAGGU
UCCCGUACCCGCCGUUCAUCGCAGACCCCUUCCUCGUGGCCAUCCAGUACCAGCU
GCCCCUGCUGCUGCUGCUCAGCUUCACCUACACCGCGCUCACCAUUGCCCGUGCU
GUCGUGCAGGAGAAGGAAAGGAGGCUGAAGGAGUACAUGCGCAUGAUGGGCUCA
GCAGCUGGCUGCACUGGAGUGCCUGGUUCCUCUUGUUCUUCCUCUUCCUCCUCAU
CGCCGCCUCCUUCAUGACCCUGCUCUUCUGUGUCAAGGUGAAGCCAAAUGUAGCC
GUGCUGUCCCGCAGCGACCCCUCCCUGGUGCUCGCCUUCCUGCUGUGCUUCGCCA
UCUCUACCAUCUCCUUCAGCUUCAUGGUCAGCACCUUCUUCAGCAAAGCCAACAUG
GCAGCAGCCUUCGGAGGCUUCCUCUACUUCUUCACCUACAUCCCCUACUUCUUCGU
GGCCCCUCGGUACAACUGGAUGACUCUGAGCCAGAAGCUCUGCUCCUGCCUCCUG
UCUAAUGUCGCCAUGGCAAUGGGAGCCCAGCUCAUUGGGAAAUUUGAGGCGAAAG
GCAUGGGCAUCCAGUGGCGAGACCUCCUGAGUCCCGUCAACGUGGACGACGACUU
CUGCUUCGGGCAGGUGCUGGGGAUGCUGCUGGACUCUGUGCUCUAUGGCCU
GGUGACCUGGUACAUGGAGGCCGUCUUCCCAGGGCAGUUCGGCGUGCCUCAGCCC
UGGUACUUCUUCAUCAUGCCCUCCUAUUGGUGUGGAAGCCAAGGGCGGUUGCAG
GGAAGGAGGAAGAAGACAGUGACCCCGAGAAAGCACUCAGAAACGAGUACUUGAA
GCCGAGCCAGAGGACCUGGUGGCGGGAUCAAGAUCAAGCACCUGUCCAAGGUGU
UCAGGGUGGGAAAUAAGGACAGGGCGGCCGUCAGAGACCUGAACCUCAACCUGUA
CGAGGGACAGAUCACCGUCCUGCUGGGCCACAACGGUGCCGGGAAGACCACCACC
CUCUCCAUGCUCACAGGUCUCUUUCCCCCCACCAGUGGACGGGCAUACAUCAGCG
GGUAUGAAAUUUCCCAGGACAUGGUUCAGAUCCGGAAGAGCCUGGGCCUGUGCCC
GCAGCACGACAUCCUGUUUGACAACUUGACAGUCGCAGAGCACCUUUAUUUCUACG
CCCAGCUGAAGGGCCUGUCACGUCAGAAGUGCCCUGAAGAAGUCAAGCAGAUGCU
GCACAUCAUCGGCCUGGAGGACAAGUGGAACUCACGGAGCCGCUUCCUGAGCGGG
GGCAUGAGGCGCAAGCUCUCCAUCGGCAUCGCCCUCAUCGCAGGCUCCAAGGUGC
UGAUACUGGACGAGCCCACCUCGGGCAUGGACGCCAUCUCCAGGAGGGCCAUCUG
 
GGAUCUUCUUCAGCGGCAGAAAAGUGACCGCACCAUCGUGCUGACCACCCACUUCA
UGGACGAGGCUGACCUGCUGGGAGACCGCAUCGCCAUCAUGGCCAAGGGGGAGCU
GCAGUGCUGCGGGUCCUCGCUGUUCCUCAAGCAGAAAUACGGUGCCGGCUAUCAC
AUGACGCUGGUGAAGGAGCCGCACUGCAACCCGGAAGACAUCUCCCAGCUGGUCC
ACCACCACGUGCCCAACGCCACGCUGGAGAGCAGCGCUGGGGCCGAGCUGUCUUU
CAUCCUUCCCAGAGAGAGCACGCACAGGUUUGAAGGUCUCUUUGCUAAACUGGAGA
AGAAGCAGAAAGAGCUGGGCAUUGCCAGCUUUGGGGCAUCCAUCACCACCAUGGA
GGAAGUCUUCCUUCGGGUCGGGAAGCUGGUGGACAGCAGUAUGGACAUCCAGGCC
AUCCAGCUCCCUGCCCUGCAGUACCAGCACGAGAGGCGCGCCAGCGACUGGGCUG
UGGACAGCAACCUCUGUGGGGCCAUGGACCCCUCCGACGGCAUUGGAGCCCUCAU
CGAGGAGGAGCGCACCGCUGUCAAGCUCAACACUGGGCUCGCCCUGCACUGCCAG
CAAUUCUGGGCCAUGUUCCUGAAGAAGGCCGCAUACAGCUGGCGCGAGUGGAAAA
UGGUGGCGGCACAGGUCCUGGUGCCUCUGACCUGCGUCACCCUGGCCCUCCUGG
CCAUCAACUACUCCUCGGAGCUCUUCGACGACCCCAUGCUGAGGCUGACCUUGGG
CGAGUACGGCAGAACCGUCGUGCCCUUCUCAGUUCCCGGGACCUCCCAGCUGGGU
CAGCAGCUGUCAGAGCAUCUGAAAGACGCACUGCAGGCUGAGGGACAGGAGCCCC
GCGAGGUGCUCGGUGACCUGGAGGAGUUCUUGAUCUUCAGGGCUUCUGUGGAGG
GGGGCGGCUUUAAUGAGCGGUGCCUUGUGGCAGCGUCCUUCAGAGAUGUGGGAG
AGCGCACGGUCGUCAACGCCUUGUUCAACAACCAGGCGUACCACUCUCCAGCCACU
GCCCUGGCCGUCGUGGACAACCUUCUGUUCAAGCUGCUGUGCGGGCCUCACGCCU
CCAUUGUGGUCUCCAACUUCCCCCAGCCCCGGAGCGCCCUGCAGGCUGCCAAGGA
CCAGUUUAACGAGGGCCGGAAGGGAUUCGACAUUGCCCUCAACCUGCUCUUCGCC
AUGGCAUUCUUGGCCAGCACGUUCUCCAUCCUGGCGGUCAGCGAGAGGGCCGUGC
AGGCCAAGCAUGUGCAGUUUGUGAGUGGAGUCCACGUGGCCAGUUUCUGGCUCUC
UGCUCUGCUGUGGGACCUCAUCUCCUUCCUCAUCCCCAGUCUGCUGCUGCUGGUG
GUGUUUAAGGCCUUCGACGUGCGUGCCUUCACGCGGGACGGCCACAUGGCUGACA
CCCUGCUGCUGCUCCUGCUCUACGGCUGGGCCAUCAUCCCCCUCAUGUACCUGAU
GAACUUCUUCUUCUUGGGGGCGGCCACUGCCUACACGAGGCUGACCAUCUUCAAC
AUCCUGUCAGGCAUCGCCACCUUCCUGAUGGUCACCAUCAUGCGCAUCCCAGCUG
UAAAACUGGAAGAACUUUCCAAAACCCUGGAUCACGUGUUCCUGGUGCUGCCCAAC
CACUGUCUGGGGAUGGCAGUCAGCAGUUUCUACGAGAACUACGAGACGCGGAGGU
ACUGCACCUCCUCCGAGGUCGCCGCCCACUACUGCAAGAAAUAUAACAUCCAGUAC
CAGGAGAACUUCUAUGCCUGGAGCGCCCCGGGGGUCGGCCGGUUUGUGGCCUCCA
UGGCCGCCUCAGGGUGCGCCUACCUCAUCCUGCUCUUCCUCAUCGAGACCAACCU
GCUUCAGAGACUCAGGGGCAUCCUCUGCGCCCUCCGGAGGAGGCGGACACUGACA
GAAUUAUACACCCGGAUGCCUGUGCUUCCUGAGGACCAAGAUGUAGCGGACGAGA
GGACCCGCAUCCUGGCCCCCAGCCCGGACUCCCUGCUCCACACACCUCUGAUUAU
CAAGGAGCUCUCCAAGGUGUACGAGCAGCGGGUGCCCCUCCUGGCCGUGGACAGG
CUCUCCCUCGCGGUGCAGAAAGGGGAGUGCUUCGGCCUGCUGGGCUUCAAUGGAG
CCGGGAAGACCACGACUUUCAAAAUGCUGACCGGGGAGGAGAGCCUCACUUCUGG
GGAUGCCUUUGUCGGGGGUCACAGAAUCAGCUCUGAUGUCGGAAAGGUGCGGCAG
CGGAUCGGCUACUGCCCGCAGUUUGAUGCCUUGCUGGACCACAUGACAGGCCGGG
AGAUGCUGGUCAUGUACGCUCGGCUCCGGGGCAUCCCUGAGCGCCACAUCGGGGC
CUGCGUGGAGAACACUCUGCGGGGCCUGCUGCUGGAGCCACAUGCCAACAAGCUG
GUCAGGACGUACAGUGGUGGUAACAAGCGGAAGCUGAGCACCGGCAUCGCCCUGA
UCGGAGAGCCUGCUGUCAUCUUCCUGGACGAGCCGUCCACUGGCAUGGACCCCGU
GGCCCGGCGCCUGCUUUGGGACACCGUGGCACGAGCCCGAGAGUCUGGCAAGGCC
AUCAUCAUCACCUCCCACAGCAUGGAGGAGUGUGAGGCCCUGUGCACCCGGCUGG
 
CCAUCAUGGUGCAGGGGCAGUUCAAGUGCCUGGGCAGCCCCCAGCACCUCAAGAG
CAAGUUCGGCAGCGGCUACUCCCUGCGGCCAAGGUGCAGAGUGAAGGCAACAG
GAGGCGCUGGAGGAGUUCAAGGCCUUCGUGGACCUGACCUUUCCAGGCAGCGUCC
UGGAAGAUGAGCACCAAGGCAUGGUCCAUUACCACCUGCCGGGCCGUGACCUCAG
CUGGGCGAAGGUUUUCGGUAUUCUGGAGAAAGCCAAGGAAAAGUACGGCGUGGAC
GACUACUCCGUGAGCCAGAUCUCGCUGGAACAGGUCUUCCUGAGCUUCGCCCACC
UGCAGCCGCCCACCGCAGAGGAGGGGCGAACGCGUACGCGACCGCUCGAGCAGAA
ACUCAUCUCAGAAGAGGAUCUGGCAGCAAAUGAUAUCCUGGAUUACAAGGAUGACG
ACGAUAAGGUUUGACCUCGCCCCGGACCUGCCCUCCCGCCAGGUGCACCCACCUG
CAAUAAAUGCAGCGAAGCCGGGAAAAAAAAAAAAAAAAAAAAAAAAAAAAAAAAA
AAAAAAAAAAAAAAAAAAAAAAAAAAAAAAAAAAAAAAAAAAAAAAAAAAAAAAAA
AAAAAAAAAAAAAAAAAAAAAAAAAAUU
**12)SEQ ID NO: 12**
   Summary: a codon optimized ABCA3 DNA sequence with a 5' CYBA UTR and a 3' CYBA UTR (SEQ ID NO: 12) 
AGACCGCGCCTAGCAGTGTCCCAGCCGGGTTCGTGTCGCCGCCACCATGGCCGTGC
TGAGACAGCTGGCTCTGCTGCTGTGGAAGAACTACACCCTGCAGAAACGGAAGGTGC
TCGTGACCGTGCTGGAACTGTTCCTGCCCCTGCTGTTCAGCGGCATCCTGATCTGGC
TGCGGCTGAAGATCCAGAGCGAGAACGTGCCCAACGCCACCATCTACCCCGGCCAG
AGCATCCAGGAACTGCCCCTGTTCTTCACCTTCCCCCCACCCGGCGATACCTGGGAG
CTGGCCTATATCCCTAGCCACAGCGACGCCGCCAAGACCGTGACAGAGACAGTGCG
GAGAGCCCTCGTGATCAACATGAGAGTGCGGGGCTTCCCCAGCGAGAAGGACTTCGA
GGACTACATCAGATACGACAACTGCAGCAGCAGCGTGCTGGCCGCCGTGGTGTTTGA
GCACCCCTTCAACCACAGCAAAGAGCCCCTGCCTCTGGCCGTGAAGTACCACCTGAG
ATTCAGCTACACCCGGCGGAACTACATGTGGACCCAGACCGGCTCATTCTTCCTGAAA
GAGACAGAGGGCTGGCACACCACCAGCCTGTTCCCTCTGTTCCCCAACCCTGGCCCC
AGAGAGCCTACATCTCCTGACGGCGGCGAGCCCGGCTATATCAGAGAAGGATTCCTG
GCCGTGCAGCACGCCGTGGACAGAGCCATCATGGAATACCACGCCGATGCCGCCAC
CCGGCAGCTGTTTCAGAGACTGACCGTGACCATCAAGCGGTTCCCTTACCCCCCCTTT
ATCGCCGACCCTTTCCTGGTGGCCATCCAGTACCAGCTGCCACTCCTCCTGCTGCTG
AGCTTTACCTACACCGCCCTGACAATCGCCAGAGCCGTGGTGCAGGAAAAAGAGCGG
CGGCTGAAAGAGTACATGCGGATGATGGGCCTGTCCAGCTGGCTGCATTGGAGCGC
CTGGTTTCTGCTGTTCTTCCTGTTCCTGCTGATCGCCGCCAGCTTCATGACACTGCTG
TTTTGCGTGAAAGTGAAGCCCAACGTGGCAGTGCTGAGCCGCAGCGATCCTAGCCTG
GTGCTGGCCTTCCTGCTGTGCTTCGCCATCAGCACCATCAGCTTCAGCTTTATGGTGT
CCACCTTCTTCAGCAAGGCCAACATGGCCGCTGCCTTCGGCGGCTTCCTGTACTTCTT
TACCTATATTCCCTACTTCTTCGTGGCCCCTCGGTACAACTGGATGACCCTGAGCCAG
AAGCTGTGCAGCTGCCTGCTGAGCAACGTGGCCATGGCTATGGGAGCCCAGCTGATC
GGCAAGTTCGAGGCCAAGGGCATGGGCATCCAGTGGCGGGATCTGCTGAGCCCCGT
GAACGTGGACGACGACTTCTGCTTCGGCCAGGTGCTGGGCATGCTGCTGCTGGACTC
CGTGCTGTATGGCCTCGTGACCTGGTATATGGAAGCCGTGTTCCCTGGCCAGTTCGG
CGTGCCCCAGCCCTGGTACTTCTTCATCATGCCTAGCTATTGGTGCGGCAAGCCCAG
GGCCGTGGCCGGCAAAGAGGAAGAGGATAGCGACCCCGAGAAGGCCCTGCGGAAC
GAGTACTTTGAGGCCGAGCCCGAGGATCTGGTGGCCGGAATCAAGATCAAGCACCTG
AGCAAGGTGTTCCGCGTGGGCAACAAGGATAGAGCCGCTGTGCGGGACCTGAACCT
GAATCTGTACGAGGGCCAGATCACCGTGCTGCTGGGCCATAATGGCGCCGGAAAGAC
 
CACCACCCTGAGCATGCTGACCGGCCTGTTTCCCCAACAAGCGGCAGGGCCTACAT
CAGCGGCTACGAGATCAGCCAGGACATGGTGCAGATCCGGAAGTCCCTGGGCCTGT
GCCCCCAGCACGACATCCTGTTCGACAACCTGACCGTGGCCGAGCACCTGTACTTTT
ACGCTCAGCTGAAGGGCCTGAGCCGGCAGAAATGCCCCGAGGAAGTGAAGCAGATG
CTGCACATCATCGGCCTGGAAGATAAGTGGAACAGCCGGTCCCGGTTCCTGTCCGGC
GGAATGAGAAGAAAGCTGAGCATCGGAATCGCCCTGATTGCCGGCAGCAAGGTGCTG
ATCCTGGACGAGCCTACCAGCGGCATGGACGCCATCTCCAGAAGGGCCATCTGGGA
CCTGCTGCAGCGGCAGAAGTCCGACAGAACCATCGTGCTGACCACCCACTTCATGGA
CGAGGCCGACCTGCTGGGCGACCGGATCGCTATTATGGCCAAGGGGGAGCTGCAGT
GCTGCGGCAGCAGCCTGTTTCTGAAGCAGAAATACGGCGCTGGCTACCACATGACCC
TCGTGAAAGAGCCTCACTGCAACCCCGAGGACATCTCCCAGCTGGTGCACCACCACG
TGCCAAATGCCACCCTGGAAAGCTCTGCCGGCGCTGAGCTGAGCTTCATCCTGCCCA
GAGAGAGCACCCACAGATTCGAGGGCCTGTTCGCCAAGCTGGAAAAGAAACAGAAAG
AGCTGGGCATTGCCAGCTTCGGCGCCAGCATCACAACAATGGAAGAGGTGTTCCTGA
GAGTGGGCAAGCTGGTGGACAGCTCCATGGACATCCAGGCTATCCAGCTGCCCGCC
CTGCAGTATCAGCACGAGAGAAGGGCTAGCGACTGGGCCGTGGACTCCAATCTGTGC
GGCGCCATGGATCCCTCCGATGGAATCGGCGCCCTGATCGAAGAGGAACGGACCGC
CGTGAAGCTGAACACAGGACTGGCCCTGCACTGCCAGCAGTTCTGGGCCATGTTCCT
GAAGAAAGCCGCCTACAGCTGGCGCGAGTGGAAAATGGTGGCCGCACAGGTGCTGG
TGCCCCTGACCTGTGTGACACTGGCACTGCTGGCCATCAACTACAGCAGCGAGCTGT
TCGACGACCCCATGCTGAGACTGACACTGGGCGAGTACGGCAGGACCGTGGTGCCT
TTTTCTGTGCCCGGCACCTCACAGCTGGGCCAGCAGCTGTCTGAACACCTGAAGGAT
GCCCTGCAGGCCGAAGGCCAGGAACCCAGAGAAGTGCTGGGCGATCTGGAAGAGTT
CCTGATCTTCCGGGCCAGCGTGGAAGGCGGCGGATTCAACGAGAGATGCCTGGTGG
CTGCCTCCTTCCGGGATGTGGGCGAGAGAACAGTCGTGAACGCCCTGTTCAACAATC
AGGCCTACCACAGCCCCGCCACCGCTCTGGCTGTGGTGGACAACCTGCTGTTTAAGC
TGCTGTGTGGCCCCCACGCCTCCATCGTGGTGTCCAATTTCCCCCAGCCCAGAAGCG
CTCTGCAGGCTGCCAAGGACCAGTTCAACGAGGGCCGGAAGGGCTTCGACATTGCTC
TGAATCTGCTGTTTGCCATGGCCTTTCTGGCCTCCACCTTCAGCATCCTGGCTGTGTC
CGAGAGAGCCGTGCAGGCCAAGCACGTGCAGTTTGTGTCTGGCGTGCACGTGGCCA
GCTTTTGGCTGTCTGCCCTGCTGTGGGACCTGATCAGCTTCCTGATCCCCAGCCTCCT
GCTGCTGGTGGTGTTCAAGGCCTTCGACGTGCGGGCCTTCACCAGGGATGGACACAT
GGCCGACACCTTGTTGTTGCTGCTGCTGTACGGCTGGGCCATCATCCCCCTGATGTA
CCTGATGAACTTCTTCTTCCTGGGCGCTGCCACCGCCTACACCAGACTGACCATCTTC
AACATCCTGAGCGGGATCGCCACCTTCCTGATGGTCACAATCATGCGGATCCCTGCC
GTGAAACTGGAAGAACTGAGCAAGACCCTGGACCATGTGTTTCTGGTGCTGCCCAAC
CACTGCCTGGGCATGGCCGTGTCTAGCTTCTACGAGAACTACGAGACACGGCGGTAC
TGCACCTCCAGCGAAGTGGCCGCCCACTACTGCAAGAAGTATAACATCCAGTATCAG
GAAAACTTCTACGCTTGGAGCGCACCCGGCGTGGGCAGATTTGTGGCCTCTATGGCC
GCCAGCGGCTGCGCCTATCTGATCCTGCTGTTCCTGATCGAGACTAACCTGCTGCAG
AGACTGAGAGGCATCCTGTGCGCCCTGCGGCGGAGAAGAACACTGACCGAGCTGTA
CACCCGGATGCCCGTGCTGCCTGAGGACCAGGATGTGGCCGACGAGCGGACAAGAA
TCCTGGCCCCTAGCCCCGATAGCCTGCTGCACACCCCCCTGATCATCAAAGAACTGT
CCAAGGTGTACGAGCAGCGGGTGCCACTGCTGGCTGTGGACAGACTGAGTCTGGCT
GTGCAGAAAGGCGAGTGCTTCGGACTGCTGGGCTTCAACGGCGCAGGCAAGACCAC
AACCTTCAAGATGCTGACAGGCGAGGAAAGCCTGACCTCCGGCGACGCCTTTGTGGG
CGGACACAGGATCTCTTCCGATGTGGGCAAAGTGCGGCAGCGGATCGGCTACTGCC
CTCAGTTCGACGCCCTGCTGGATCACATGACCGGCAGGGAAATGCTCGTGATGTACG
 
CCCGGCTGAGGGCATCCCCGAGAGACACATTGGCGCCTGCGTGGAAAACACCCTG
CGGGGCCTGCTGCTGGAACCCCACGCTAACAAACTCGTGCGGACCTACAGCGGCGG
CAACAAGAGAAAGCTGTCTACCGGCATTGCACTGATCGGCGAGCCAGCCGTGATCTT
TCTGGATGAGCCCAGCACAGGCATGGACCCCGTGGCTCGGAGACTGCTGTGGGATA
CAGTGGCCAGAGCCAGAGAGTCCGGCAAGGCCATCATTATCACCAGCCACAGCATGG
AAGAGTGCGAGGCCCTGTGTACAAGACTGGCAATTATGGTGCAGGGACAGTTCAAGT
GTCTGGGCAGCCCTCAGCACCTGAAGTCCAAGTTCGGCTCCGGCTACAGCCTGCGG
GCCAAGGTGCAGTCTGAAGGGCAGCAGGAAGCCCTGGAAGAATTCAAAGCCTTCGTG
GACCTGACCTTCCCCGGCTCTGTGCTGGAAGATGAGCACCAGGGAATGGTGCACTAC
CATCTGCCTGGCAGGGACCTGTCCTGGGCCAAAGTGTTTGGCATCCTGGAAAAGGCC
AAAGAGAAGTACGGCGTGGACGATTACAGCGTGTCCCAGATCAGCCTGGAACAGGTG
TTCCTGTCCTTTGCCCATCTGCAGCCCCCTACCGCCGAAGAGGGAAGAACGCGTACG
CGACCGCTCGAGCAGAAACTCATCTCAGAAGAGGATCTGGCAGCAAATGATATCCTG
GATTACAAGGATGACGACGATAAGGTTTGACCTCGCCCCGGACCTGCCCTCCCGCCA
GGTGCACCCACCTGCAATAAATGCAGCGAAGCCGGAAAAAAAAAAAAAAAAAAAAA
AAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAATT
**13)SEQ ID NO: 13**
   Summary: a codon optimized ABCA3 mRNA sequence with a 5' CYBA UTR and a 3' CYBA UTR (SEQ ID NO: 13). 
AGACCGCGCCUAGCAGUGUCCCAGCCGGGUUCGUGUCGCCGCCACCAUGGCCGUG
CUGAGACAGCUGGCUCUGCUGCUGUGGAAGAACUACACCCUGCAGAAACGGAAGG
UGCUCGUGACCGUGCUGGAACUGUUCCUGCCCCUGCUGUUCAGCGGCAUCCUGAU
CUGGCUGCGGCUGAAGAUCCAGAGCGAGAACGUGCCCAACGCCACCAUCUACCCC
GGCCAGAGCAUCCAGGAACUGCCCCUGUUCUUCACCUUCCCCCCACCCGGCGAUA
CCUGGGAGCUGGCCUAUAUCCCUAGCCACAGCGACGCCGCCAAGACCGUGACAGA
GACAGUGCGGAGAGCCCUCGUGAUCAACAUGAGAGUGCGGGGCUUCCCCAGCGAG
AAGGACUUCGAGGACUACAUCAGAUACGACAACUGCAGCAGCAGCGUGCUGGCCGC
CGUGGUGUUUGAGCACCCCUUCAACCACAGCAAAGAGCCCCUGCCUCUGGCCGUG
AAGUACCACCUGAGAUUCAGCUACACCCGGCGGAACUACAUGUGGACCCAGACCGG
CUCAUUCUUCCUGAAAGAGACAGAGGGCUGGCACACCACCAGCCUGUUCCCUCUGU
UCCCCAACCCUGGCCCCAGAGAGCCUACAUCUCCUGACGGCGGCGAGCCCGGCUA
UAUCAGAGAAGGAUUCCUGGCCGUGCAGCACGCCGUGGACAGAGCCAUCAUGGAA
UACCACGCCGAUGCCGCCACCCGGCAGCUGUUUCAGAGACUGACCGUGACCAUCAA
GCGGUUCCCUUACCCCCCCUUUAUCGCCGACCCUUUCCUGGUGGCCAUCCAGUAC
CAGCUGCCACUCCUCCUGCUGCUGAGCUUUACCUACACCGCCCUGACAAUCGCCAG
AGCCGUGGUGCAGGAAAAAGAGCGGCGGCUGAAAGAGUACAUGCGGAUGAUGGGC
CUGUCCAGCUGGCUGCAUUGGAGCGCCUGGUUUCUGCUGUUCUUCCUGUUCCUGC
UGAUCGCCGCCAGCUUCAUGACACUGCUGUUUGCGUGAAAGUGAAGCCCAACGU
GGCAGUGCUGAGCCGCAGCGAUCCUAGCCUGGUGCUGGCCUUCCUGCUGUGCUUC
GCCAUCAGCACCAUCAGCUUCAGCUUUAUGGUGUCCACCUUCUUCAGCAAGGCCAA
CAUGGCCGCUGCCUUCGGCGGCUUCCUGUACUUCUUUACCUAUAUUCCCUACUUC
UUCGUGGCCCCUCGGUACAACUGGAUGACCCUGAGCCAGAAGCUGUGCAGCUGCC
UGCUGAGCAACGUGGCCAUGGCUAUGGGAGCCCAGCUGAUCGGCAAGUUCGAGGC
CAAGGGCAUGGGCAUCCAGUGGCGGGAUCUGCUGAGCCCCGUGAACGUGGACGAC
GACUUCUGCUUCGGCCAGGUGCUGGGCAUGCUGCUGCUGGACUCCGUGCUGUAU
 
GGCCUCGUGACCUGGUAUGGAAGCCGUGUUCCCUGGCCAGUUCGGCGUGCCCC
AGCCCUGGUACUUCUUCAUCAUGCCUAGCUAUUGGUGCGGCAAGCCCAGGGCCGU
GGCCGGCAAAGAGGAAGAGGAUAGCGACCCCGAGAAGGCCCUGCGGAACGAGUAC
UUUGAGGCCGAGCCCGAGGAUCUGGUGGCCGGAAUCAAGAUCAAGCACCUGAGCA
AGGUGUUCCGCGUGGGCAACAAGGAUAGAGCCGCUGUGCGGGACCUGAACCUGAA
UCUGUACGAGGGCCAGAUCACCGUGCUGCUGGGCCAUAAUGGCGCCGGAAAGACC
ACCACCCUGAGCAUGCUGACCGGCCUGUUCCCCCAACAAGCGGCAGGGCCUACA
UCAGCGGCUACGAGAUCAGCCAGGACAUGGUGCAGAUCCGGAAGUCCCUGGGCCU
GUGCCCCCAGCACGACAUCCUGUUCGACAACCUGACCGUGGCCGAGCACCUGUAC
UUUUACGCUCAGCUGAAGGGCCUGAGCCGGCAGAAAUGCCCCGAGGAAGUGAAGC
AGAUGCUGCACAUCAUCGGCCUGGAAGAUAAGUGGAACAGCCGGUCCCGGUUCCU
GUCCGGCGGAAUGAGAAGAAAGCUGAGCAUCGGAAUCGCCCUGAUUGCCGGCAGC
AAGGUGCUGAUCCUGGACGAGCCUACCAGCGGCAUGGACGCCAUCUCCAGAAGGG
CCAUCUGGGACCUGCUGCAGCGGCAGAAGUCCGACAGAACCAUCGUGCUGACCAC
CCACUUCAUGGACGAGGCCGACCUGCUGGGCGACCGGAUCGCUAUUAUGGCCAAG
GGGGAGCUGCAGUGCUGCGGCAGCAGCCUGUUUCUGAAGCAGAAAUACGGCGCUG
GCUACCACAUGACCCUCGUGAAAGAGCCUCACUGCAACCCCGAGGACAUCUCCCAG
CUGGUGCACCACCACGUGCCAAAUGCCACCCUGGAAAGCUCUGCCGGCGCUGAGC
UGAGCUUCAUCCUGCCCAGAGAGAGCACCCACAGAUUCGAGGGCCUGUUCGCCAA
GCUGGAAAAGAAACAGAAAGAGCUGGGCAUUGCCAGCUUCGGCGCCAGCAUCACAA
CAAUGGAAGAGGUGUUCCUGAGAGUGGGCAAGCUGGUGGACAGCUCCAUGGACAU
CCAGGCUAUCCAGCUGCCCGCCCUGCAGUAUCAGCACGAGAGAAGGGCUAGCGAC
UGGGCCGUGGACUCCAAUCUGUGCGGCGCCAUGGAUCCCUCCGAUGGAAUCGGCG
CCCUGAUCGAAGAGGAACGGACCGCCGUGAAGCUGAACACAGGACUGGCCCUGCA
CUGCCAGCAGUUCUGGGCCAUGUUCCUGAAGAAAGCCGCCUACAGCUGGCGCGAG
UGGAAAAUGGUGGCCGCACAGGUGCUGGUGCCCCUGACCUGUGUGACACUGGCAC
UGCUGGCCAUCAACUACAGCAGCGAGCUGUUCGACGACCCCAUGCUGAGACUGACA
CUGGGCGAGUACGGCAGGACCGUGGUGCCUUUUCUGUGCCCGGCACCUCACAGC
UGGGCCAGCAGCUGUCUGAACACCUGAAGGAUGCCCUGCAGGCCGAAGGCCAGGA
ACCCAGAGAAGUGCUGGGCGAUCUGGAAGAGUUCCUGAUCUUCCGGGCCAGCGUG
GAAGGCGGCGGAUUCAACGAGAGAUGCCUGGUGGCUGCCUCCUUCCGGGAUGUGG
GCGAGAGAACAGUCGUGAACGCCCUGUUCAACAAUCAGGCCUACCACAGCCCCGCC
ACCGCUCUGGCUGUGGUGGACAACCUGCUGUUUAAGCUGCUGUGUGGCCCCCACG
CCUCCAUCGUGGUGUCCAAUUUCCCCCAGCCCAGAAGCGCUCUGCAGGCUGCCAA
GGACCAGUUCAACGAGGGCCGGAAGGGCUUCGACAUUGCUCUGAAUCUGCUGUUU
GCCAUGGCCUUUCUGGCCUCCACCUUCAGCAUCCUGGCUGUGUCCGAGAGAGCCG
UGCAGGCCAAGCACGUGCAGUUUGUGUCUGGCGUGCACGUGGCCAGCUUUUGGCU
GUCUGCCCUGCUGUGGGACCUGAUCAGCUUCCUGAUCCCCAGCCUCCUGCUGCUG
GUGGUGUUCAAGGCCUUCGACGUGCGGGCCUUCACCAGGGAUGGACACAUGGCCG
ACACCUUGUUGUUGCUGCUGCUGUACGGCUGGGCCAUCAUCCCCCUGAUGUACCU
GAUGAACUUCUUCUUCCUGGGCGCUGCCACCGCCUACACCAGACUGACCAUCUUCA
ACAUCCUGAGCGGGAUCGCCACCUUCCUGAUGGUCACAAUCAUGCGGAUCCCUGC
CGUGAAACUGGAAGAACUGAGCAAGACCCUGGACCAUGUGUUUCUGGUGCUGCCC
AACCACUGCCUGGGCAUGGCCGUGUCUAGCUUCUACGAGAACUACGAGACACGGC
GGUACUGCACCUCCAGCGAAGUGGCCGCCCACUACUGCAAGAAGUAUAACAUCCAG
UAUCAGGAAAACUUCUACGCUUGGAGCGCACCCGGCGUGGGCAGAUUUGUGGCCU
CUAUGGCCGCCAGCGGCUGCGCCUAUCUGAUCCUGCUGUUCCUGAUCGAGACUAA
CCUGCUGCAGAGACUGAGAGGCAUCCUGUGCGCCCUGCGGCGGAGAAGAACACUG
 
ACCGAGCUGUACACCCGGAUGCCCGUGCUGCCUGAGGACCAGGAUGUGGCCGACG
AGCGGACAAGAAUCCUGGCCCCUAGCCCCGAUAGCCUGCUGCACACCCCCCUGAUC
AUCAAAGAACUGUCCAAGGUGUACGAGCAGCGGGUGCCACUGCUGGCUGUGGACA
GACUGAGUCUGGCUGUGCAGAAAGGCGAGUGCUUCGGACUGCUGGGCUUCAACGG
CGCAGGCAAGACCACAACCUUCAAGAUGCUGACAGGCGAGGAAAGCCUGACCUCCG
GCGACGCCUUUGUGGGCGGACACAGGAUCUCUUCCGAUGUGGGCAAAGUGCGGCA
GCGGAUCGGCUACUGCCCUCAGUUCGACGCCCUGCUGGAUCACAUGACCGGCAGG
GAAAUGCUCGUGAUGUACGCCCGGCUGAGGGGCAUCCCCGAGAGACACAUUGGCG
CCUGCGUGGAAAACACCCUGCGGGGCCUGCUGCUGGAACCCCACGCUAACAAACU
CGUGCGGACCUACAGCGGCGGCAACAAGAGAAAGCUGUCUACCGGCAUUGCACUG
AUCGGCGAGCCAGCCGUGAUCUUUCUGGAUGAGCCCAGCACAGGCAUGGACCCCG
UGGCUCGGAGACUGCUGUGGGAUACAGUGGCCAGAGCCAGAGAGUCCGGCAAGGC
CAUCAUUAUCACCAGCCACAGCAUGGAAGAGUGCGAGGCCCUGUGUACAAGACUGG
CAAUUAUGGUGCAGGGACAGUUCAAGUGUCUGGGCAGCCCUCAGCACCUGAAGUC
CAAGUUCGGCUCCGGCUACAGCCUGCGGGCCAAGGUGCAGUCUGAAGGGCAGCAG
GAAGCCCUGGAAGAAUUCAAAGCCUUCGUGGACCUGACCUUCCCCGGCUCUGUGC
UGGAAGAUGAGCACCAGGGAAUGGUGCACUACCAUCUGCCUGGCAGGGACCUGUC
CUGGGCCAAAGUGUUUGGCAUCCUGGAAAAGGCCAAAGAGAAGUACGGCGUGGAC
GAUUACAGCGUGUCCCAGAUCAGCCUGGAACAGGUGUUCCUGUCCUUUGCCCAUC
UGCAGCCCCCUACCGCCGAAGAGGGAAGAACGCGUACGCGACCGCUCGAGCAGAA
ACUCAUCUCAGAAGAGGAUCUGGCAGCAAAUGAUAUCCUGGAUUACAAGGAUGACG
ACGAUAAGGUUUGACCUCGCCCCGGACCUGCCCUCCCGCCAGGUGCACCCACCUG
CAAUAAAUGCAGCGAAGCCGGGAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAA
AAAAAAAAAAAAAAAAAAAAAAAAAAAAAUU
**14)SEQ ID NO: 14**
   Summary: mRNA counterpart of SEQ ID NO: 1
   CGCGCCUAGCAGUGUCCCAGCCGGGUUCGUGUCGCC
**15)SEQ ID NO: 15**
   Summary: mRNA counterpart of SEQ ID NO: 2 
CCUCGCCCCGGACCUGCCCUCCCGCCAGGUGCACCCACCUGCAAUAAAUGCAGCG
AAGCCGGGA
**16) SEQ ID NO: 16**
   Summary: mRNA counterpart of SEQ ID NO: 5
   UCUUCUGGUCCCCACAGACUCAGAGAGAAC
**17)SEQ ID NO: 17**
   Summary: Transcript TTX-ABCA3-RNA-018
   Black - flanking sequences
   italic, underlined - *Kozak sequence*
   Blue - codon-optimized ABCA3
   Red - c-myc-tag
   Green - FLAG-tag
   Highlighted red - stop codon
   Orange - poly(A) tail 
gggagacccaagctggctagcgtttaaacttaagcttggcaatccggtactgttggtaaagccaccatggccgtgctgagaca
gctggctctgctgctgtggaagaactacaccctgcagaaacggaaggtgctcgtgaccgtgctggaactgttcctgcccctgct
gttcagcggcatcctgatctggctgcggctgaagatccagagcgagaacgtgcccaacgccaccatctaccccggccagag
catccaggaactgcccctgttcttcaccttcccccacccggcgatacctgggagctggcctatatccctagccacagcgacgc
cgccaagaccgtgacagagacagtgcggagagccctcgtgatcaacatgagagtgcggggcttccccagcgagaaggact
tcgaggactacatcagatacgacaactgcagcagcagcgtgctggccgccgtggtgtttgagcaccccttcaaccacagcaa
agagcccctgcctctggccgtgaagtaccacctgagattcagctacacccggcggaactacatgtggacccagaccggctca
ttcttcctgaaagagacagagggctggcacaccaccagcctgttccctctgttccccaaccctggccccagagagcctacatct
cctgacggcggcgagcccggctatatcagagaaggattcctggccgtgcagcacgccgtggacagagccatcatggaatac
cacgccgatgccgccacccggcagctgtttcagagactgaccgtgaccatcaagcggttcccttacccccctttatcgccgac
cctttcctggtggccatccagtaccagctgccactcctcctgctgctgagctttacctacaccgccctgacaatcgccagagccgt
ggtgcaggaaaaagagcggcggctgaaagagtacatgcggatgatgggcctgtccagctggctgcattggagcgcctggttt
ctgctgttcttcctgttcctgctgatcgccgccagcttcatgacactgcttttgcgtgaaagtgaagcccaacgtggcagtgctga
gccgcagcgatcctagcctggtgctggccttcctgctgtgcttcgccatcagcaccatcagcttcagctttatggtgtccaccttcttc
agcaaggccaacatggccgctgccttcggcggcttcctgtacttctttacctatattccctacttcttcgtggcccctcggtacaact
ggatgaccctgagccagaagctgtgcagctgcctgctgagcaacgtggccatggctatgggagcccagctgatcggcaagtt
cgaggccaagggcatgggcatccagtggcgggatctgctgagccccgtgaacgtggacgacgacttctgcttcggccaggtg
ctgggcatgctgctgctggactccgtgctgtatggcctcgtgacctggtatatggaagccgtgttccctggccagttcggcgtgcc
ccagccctggtacttcttcatcatgcctagctattggtgcggcaagcccagggccgtggccggcaaagaggaagaggatagc
gaccccgagaaggccctgcggaacgagtactttgaggccgagcccgaggatctggtggccggaatcaagatcaagcacct
gagcaaggtgttccgcgtgggcaacaaggatagagccgctgtgcgggacctgaacctgaatctgtacgagggccagatcac
cgtgctgctgggccataatggcgccggaaagaccaccaccctgagcatgctgaccggcctgtttccccccaacaagcggcag
ggcctacatcagcggctacgagatcagccaggacatggtgcagatccggaagtccctgggcctgtgcccccagcacgacat
cctgttcgacaacctgaccgtggccgagcacctgtacttttacgctcagctgaagggcctgagccggcagaaatgccccgagg
aagtgaagcagatgctgcacatcatcggcctggaagataagtggaacagccggtcccggttcctgtccggcggaatgagaa
gaaagctgagcatcggaatcgccctgattgccggcagcaaggtgctgatcctggacgagcctaccagcggcatggacgcca
tctccagaagggccatctgggacctgctgcagcggcagaagtccgacagaaccatcgtgctgaccacccacttcatggacga
ggccgacctgctgggcgaccggatcgctattatggccaagggggagctgcagtgctgcggcagcagcctgtttctgaagcag
aaatacggcgctggctaccacatgaccctcgtgaaagagcctcactgcaaccccgaggacatctcccagctggtgcaccac
cacgtgccaaatgccaccctggaaagctctgccggcgctgagctgagcttcatcctgcccagagagagcacccacagattcg
agggcctgttcgccaagctggaaaagaaacagaaagagctgggcattgccagcttcggcgccagcatcacaacaatggaa
gaggtgttcctgagagtgggcaagctggtggacagctccatggacatccaggctatccagctgcccgccctgcagtatcagca
cgagagaagggctagcgactggccgtggactccaatctgtgcggcgccatggatccctccgatggaatcggcgccctgatc
gaagaggaacggaccgccgtgaagctgaacacaggactggccctgcactgccagcagttctgggccatgttcctgaagaaa
gccgcctacagctggcgcgagtggaaaatggtggccgcacaggtgctggtgcccctgacctgtgtgacactggcactgctgg
ccatcaactacagcagcgagctgttcgacgaccccatgctgagactgacactgggcgagtacggcaggaccgtggtgcctttt
tctgtgcccggcacctcacagctgggccagcagctgtctgaacacctgaaggatgccctgcaggccgaaggccaggaaccc
agagaagtgctgggcgatctggaagagttcctgatcttccgggccagcgtggaaggcggcggattcaacgagagatgcctgg
tggctgcctccttccgggatgtgggcgagagaacagtcgtgaacgccctgttcaacaatcaggcctaccacagccccgccac
cgctctggctgtggtggacaacctgctgtttaagctgctgtgtggcccccacgcctccatcgtggtgtccaatttcccccagccca
gaagcgctctgcaggctgccaaggaccagttcaacgagggccggaagggcttcgacattgctctgaatctgctgtttgccatgg
cctttctggcctccaccttcagcatcctggctgtgtccgagagagccgtgcaggccaagcacgtgcagtttgtgtctggcgtgcac
gtggccagcttttggctgtctgccctgctgtgggacctgatcagcttcctgatccccagcctcctgctgctggtggtgttcaaggcctt
 
cgacgtgcgggccttcaccagggatggacacatggccgacaccttgttgctgctgctgtacggctgggccatcatcccctg
atgtacctgatgaacttcttcctgggcgctgccaccgcctacaccagactgaccatcttcaacatcctgagcggatcgcca
ccttcctgatggtcacaatcatgcggatccctgccgtgaaactggaagaactgagcaagaccctggaccatgtgtttctggtgct
gcccaaccactgcctgggcatggccgtgtctagcttctacgagaactacgagacacggcggtactgcacctccagcgaagtg
gccgcccactactgcaagaagtataacatccagtatcaggaaaacttctacgcttggagcgcacccggcgtgggcagatttgt
ggcctctatggccgccagcggctgcgcctatctgatcctgctgttcctgatcgagactaacctgctgcagagactgagaggcatc
ctgtgcgccctgcggcggagaagaacactgaccgagctgtacacccggatgcccgtgctgcctgaggaccaggatgtggcc
gacgagcggacaagaatcctggcccctagccccgatagcctgctgcacacccccctgatcatcaagaactgtccaaggtgt
acgagcagcgggtgccactgctggctgtggacagactgagtctggctgtgcagaaaggcgagtgcttcggactgctgggcttc
aacggcgcaggcaagaccacaaccttcaagatgctgacaggcgaggaaagcctgacctccggcgacgcctttgtgggcgg
acacaggatctcttccgatgtgggcaaagtgcggcagcggatcggctactgccctcagttcgacgccctgctggatcacatga
ccggcagggaaatgctcgtgatgtacgcccggctgaggggcatccccgagagacacattggcgcctgcgtggaaaacacc
ctgcggggcctgctgctggaaccccacgctaacaaactcgtgcggacctacagcggcggcaacaagagaaagctgtctacc
ggcattgcactgatcggcgagccagccgtgatctttctggatgagcccagcacaggcatggaccccgtggctcggagactgct
gtgggatacagtggccagagccagagagtccggcaaggccatcattatcaccagccacagcatggaagagtgcgaggccc
tgtgtacaagactggcaattatggtgcagggacagttcaagtgtctgggcagccctcagcacctgaagtccaagttcggctccg
gctacagcctgcgggccaaggtgcagtctgaagggcagcaggaagccctggaagagttcaaagccttcgtggacctgacctt
ccccggctctgtgctggaagatgagcaccagggaatggtgcactaccatctgcctggcagggacctgtcctggccaaagtgt
ttggcatcctggaaaaggccaaagagaagtacggcgtggacgattacagcgtgtcccagatcagcctggaacaggtgttcct
gtcctttgcccatctgcagccccctaccgccgaagagggaagaacgcgtacgcggccgctcgagcagaaactcatctcaga
agaggatctggcagcaaatgatatcctggattacaaggatgacgacgataaggtttaagaattctgcagAAAAAAAAAA
AAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAGC
**18)SEQ ID NO: 18**
   Summary: Transcript TTX-ABCA3-RNA-019
   Black - flanking sequences
   Gray - 5' UTR human alpha globin
   italic, underlined - *Kozak sequence*
   Blue - codon-optimized ABCA3
   Red - c-myc-tag
   Green - FLAG-tag
   Highlighted red - stop codon
   Orange - poly(A) tail 
GGGAGACTCTTCTGGTCCCCACAGACTCAGAGAGAACGCCACCATGGCCGTGCTGAG
ACAGCTGGCTCTGCTGCTGTGGAAGAACTACACCCTGCAGAAACGGAAGGTGCTCGT
GACCGTGCTGGAACTGTTCCTGCCCCTGCTGTTCAGCGGCATCCTGATCTGGCTGCG
GCTGAAGATCCAGAGCGAGAACGTGCCCAACGCCACCATCTACCCCGGCCAGAGCAT
CCAGGAACTGCCCCTGTTCTTCACCTTCCCCCCACCCGGCGATACCTGGGAGCTGGC
CTATATCCCTAGCCACAGCGACGCCGCCAAGACCGTGACAGAGACAGTGCGGAGAG
CCCTCGTGATCAACATGAGAGTGCGGGGCTTCCCCAGCGAGAAGGACTTCGAGGACT
ACATCAGATACGACAACTGCAGCAGCAGCGTGCTGGCCGCCGTGGTGTTTGAGCACC
CCTTCAACCACAGCAAAGAGCCCCTGCCTCTGGCCGTGAAGTACCACCTGAGATTCA
GCTACACCCGGCGGAACTACATGTGGACCCAGACCGGCTCATTCTTCCTGAAAGAGA
CAGAGGGCTGGCACACCACCAGCCTGTTCCCTCTGTTCCCCAACCCTGGCCCCAGAG
AGCCTACATCTCCTGACGGCGGCGAGCCCGGCTATATCAGAGAAGGATTCCTGGCCG
 
TGCAGCACGCCGTGGACAGAGCCATCATGGAATACCACGCCGATGCCGCCACCCGG
CAGCTGTTTCAGAGACTGACCGTGACCATCAAGCGGTTCCCTTACCCCCCCTTTATCG
CCGACCCTTTCCTGGTGGCCATCCAGTACCAGCTGCCACTCCTCCTGCTGCTGAGCTT
TACCTACACCGCCCTGACAATCGCCAGAGCCGTGGTGCAGGAAAAAGAGCGGCGGC
TGAAAGAGTACATGCGGATGATGGGCCTGTCCAGCTGGCTGCATTGGAGCGCCTGGT
TTCTGCTGTTCTTCCTGTTCCTGCTGATCGCCGCCAGCTTCATGACACTGCTGTTTTGC
GTGAAAGTGAAGCCCAACGTGGCAGTGCTGAGCCGCAGCGATCCTAGCCTGGTGCT
GGCCTTCCTGCTGTGCTTCGCCATCAGCACCATCAGCTTCAGCTTTATGGTGTCCACC
TTCTTCAGCAAGGCCAACATGGCCGCTGCCTTCGGCGGCTTCCTGTACTTCTTTACCT
ATATTCCCTACTTCTTCGTGGCCCCTCGGTACAACTGGATGACCCTGAGCCAGAAGCT
GTGCAGCTGCCTGCTGAGCAACGTGGCCATGGCTATGGGAGCCCAGCTGATCGGCA
AGTTCGAGGCCAAGGGCATGGGCATCCAGTGGCGGGATCTGCTGAGCCCCGTGAAC
GTGGACGACGACTTCTGCTTCGGCCAGGTGCTGGGCATGCTGCTGCTGGACTCCGTG
CTGTATGGCCTCGTGACCTGGTATATGGAAGCCGTGTTCCCTGGCCAGTTCGGCGTG
CCCCAGCCCTGGTACTTCTTCATCATGCCTAGCTATTGGTGCGGCAAGCCCAGGGCC
GTGGCCGGCAAAGAGGAAGAGGATAGCGACCCCGAGAAGGCCCTGCGGAACGAGTA
CTTTGAGGCCGAGCCCGAGGATCTGGTGGCCGGAATCAAGATCAAGCACCTGAGCAA
GGTGTTCCGCGTGGGCAACAAGGATAGAGCCGCTGTGCGGACCTGAACCTGAATCT
GTACGAGGGCCAGATCACCGTGCTGCTGGGCCATAATGGCGCCGGAAAGACCACCA
CCCTGAGCATGCTGACCGGCCTGTTTCCCCCAACAAGCGGCAGGGCCTACATCAGCG
GCTACGAGATCAGCCAGGACATGGTGCAGATCCGGAAGTCCCTGGGCCTGTGCCCC
CAGCACGACATCCTGTTCGACAACCTGACCGTGGCCGAGCACCTGTACTTTTACGCTC
AGCTGAAGGGCCTGAGCCGGCAGAAATGCCCCGAGGAAGTGAAGCAGATGCTGCAC
ATCATCGGCCTGGAAGATAAGTGGAACAGCCGGTCCCGGTTCCTGTCCGGCGGAATG
AGAAGAAAGCTGAGCATCGGAATCGCCCTGATTGCCGGCAGCAAGGTGCTGATCCTG
GACGAGCCTACCAGCGGCATGGACGCCATCTCCAGAAGGGCCATCTGGGACCTGCT
GCAGCGGCAGAAGTCCGACAGAACCATCGTGCTGACCACCCACTTCATGGACGAGGC
CGACCTGCTGGGCGACCGGATCGCTATTATGGCCAAGGGGGAGCTGCAGTGCTGCG
GCAGCAGCCTGTTTCTGAAGCAGAAATACGGCGCTGGCTACCACATGACCCTCGTGA
AAGAGCCTCACTGCAACCCCGAGGACATCTCCCAGCTGGTGCACCACCACGTGCCAA
ATGCCACCCTGGAAAGCTCTGCCGGCGCTGAGCTGAGCTTCATCCTGCCCAGAGAGA
GCACCCACAGATTCGAGGGCCTGTTCGCCAAGCTGGAAAAGAAACAGAAAGAGCTGG
GCATTGCCAGCTTCGGCGCCAGCATCACAACAATGGAAGAGGTGTTCCTGAGAGTGG
GCAAGCTGGTGGACAGCTCCATGGACATCCAGGCTATCCAGCTGCCCGCCCTGCAGT
ATCAGCACGAGAGAAGGGCTAGCGACTGGGCCGTGGACTCCAATCTGTGCGGCGCC
ATGGATCCCTCCGATGGAATCGGCGCCCTGATCGAAGAGGAACGGACCGCCGTGAA
GCTGAACACAGGACTGGCCCTGCACTGCCAGCAGTTCTGGGCCATGTTCCTGAAGAA
AGCCGCCTACAGCTGGCGCGAGTGGAAAATGGTGGCCGCACAGGTGCTGGTGCCCC
TGACCTGTGTGACACTGGCACTGCTGGCCATCAACTACAGCAGCGAGCTGTTCGACG
ACCCCATGCTGAGACTGACACTGGGCGAGTACGGCAGGACCGTGGTGCCTTTTTCTG
TGCCCGGCACCTCACAGCTGGGCCAGCAGCTGTCTGAACACCTGAAGGATGCCCTGC
AGGCCGAAGGCCAGGAACCCAGAGAAGTGCTGGGCGATCTGGAAGAGTTCCTGATCT
TCCGGGCCAGCGTGGAAGGCGGCGGATTCAACGAGAGATGCCTGGTGGCTGCCTCC
TTCCGGGATGTGGGCGAGAGAACAGTCGTGAACGCCCTGTTCAACAATCAGGCCTAC
CACAGCCCCGCCACCGCTCTGGCTGTGGTGGACAACCTGCTGTTTAAGCTGCTGTGT
GGCCCCCACGCCTCCATCGTGGTGTCCAATTTCCCCCAGCCCAGAAGCGCTCTGCAG
GCTGCCAAGGACCAGTTCAACGAGGGCCGGAAGGGCTTCGACATTGCTCTGAATCTG
CTGTTTGCCATGGCCTTTCTGGCCTCCACCTTCAGCATCCTGGCTGTGTCCGAGAGAG
 
CCGTGCAGGCCAAGCACGTGCAGTTTGTGTCTGGCGTGCACGTGGCCAGCTTTTGGC
TGTCTGCCCTGCTGTGGACCTGATCAGCTTCCTGATCCCCAGCCTCCTGCTGCTGG
TGGTGTTCAAGGCCTTCGACGTGCGGGCCTTCACCAGGATGGACACATGGCCGACA
CCTTGTTGTTGCTGCTGCTGTACGGCTGGGCCATCATCCCCCTGATGTACCTGATGAA
CTTCTTCTTCCTGGGCGCTGCCACCGCCTACACCAGACTGACCATCTTCAACATCCTG
AGCGGGATCGCCACCTTCCTGATGGTCACAATCATGCGGATCCCTGCCGTGAAACTG
GAAGAACTGAGCAAGACCCTGGACCATGTGTTTCTGGTGCTGCCCAACCACTGCCTG
GGCATGGCCGTGTCTAGCTTCTACGAGAACTACGAGACACGGCGGTACTGCACCTCC
AGCGAAGTGGCCGCCCACTACTGCAAGAAGTATAACATCCAGTATCAGGAAAACTTCT
ACGCTTGGAGCGCACCCGGCGTGGGCAGATTTGTGGCCTCTATGGCCGCCAGCGGC
TGCGCCTATCTGATCCTGCTGTTCCTGATCGAGACTAACCTGCTGCAGAGACTGAGAG
GCATCCTGTGCGCCCTGCGGCGGAGAAGAACACTGACCGAGCTGTACACCCGGATG
CCCGTGCTGCCTGAGGACCAGGATGTGGCCGACGAGCGGACAAGAATCCTGGCCCC
TAGCCCCGATAGCCTGCTGCACACCCCCCTGATCATCAAAGAACTGTCCAAGGTGTAC
GAGCAGCGGGTGCCACTGCTGGCTGTGGACAGACTGAGTCTGGCTGTGCAGAAAGG
CGAGTGCTTCGGACTGCTGGGCTTCAACGGCGCAGGCAAGACCACAACCTTCAAGAT
GCTGACAGGCGAGGAAAGCCTGACCTCCGGCGACGCCTTTGTGGGCGGACACAGGA
TCTCTTCCGATGTGGGCAAAGTGCGGCAGCGGATCGGCTACTGCCCTCAGTTCGACG
CCCTGCTGGATCACATGACCGGCAGGGAAATGCTCGTGATGTACGCCCGGCTGAGG
GGCATCCCCGAGAGACACATTGGCGCCTGCGTGGAAAACACCCTGCGGGGCCTGCT
GCTGGAACCCCACGCTAACAAACTCGTGCGGACCTACAGCGGCGGCAACAAGAGAAA
GCTGTCTACCGGCATTGCACTGATCGGCGAGCCAGCCGTGATCTTTCTGGATGAGCC
CAGCACAGGCATGGACCCCGTGGCTCGGAGACTGCTGTGGGATACAGTGGCCAGAG
CCAGAGAGTCCGGCAAGGCCATCATTATCACCAGCCACAGCATGGAAGAGTGCGAGG
CCCTGTGTACAAGACTGGCAATTATGGTGCAGGGACAGTTCAAGTGTCTGGGCAGCC
CTCAGCACCTGAAGTCCAAGTTCGGCTCCGGCTACAGCCTGCGGGCCAAGGTGCAGT
CTGAAGGGCAGCAGGAAGCCCTGGAAGAATTCAAAGCCTTCGTGGACCTGACCTTCC
CCGGCTCTGTGCTGGAAGATGAGCACCAGGGAATGGTGCACTACCATCTGCCTGGCA
GGGACCTGTCCTGGGCCAAAGTGTTTGGCATCCTGGAAAAGGCCAAAGAGAAGTACG
GCGTGGACGATTACAGCGTGTCCCAGATCAGCCTGGAACAGGTGTTCCTGTCCTTTG
CCCATCTGCAGCCCCCTACCGCCGAAGAGGGAAGAacgcgtacgcggccgctcgagcagaaactc
atctcagaagaggatctggcagcaaatgatatcctggattacaaggatgacgacgataaggtttaagaattctgcagAAAAA
AAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAGC
19)SEQ ID NO: 19
   Summary: Codon optimized ABCA3 sequence without any tags (-FLAG and Myc tags)

gggagacccaagctggctagcgtttaaacttaagcttggcaatccggtactgttggtaaagccaccatggccgtgctgagaca
gctggctctgctgctgtggaagaactacaccctgcagaaacggaaggtgctcgtgaccgtgctggaactgttcctgcccctgct
gttcagcggcatcctgatctggctgcggctgaagatccagagcgagaacgtgcccaacgccaccatctaccccggccagag
catccaggaactgcccctgttcttcaccttcccccccacccggcgatacctgggagctggcctatatccctagccacagcgacgc
cgccaagaccgtgacagagacagtgcggagagccctcgtgatcaacatgagagtgcggggcttccccagcgagaaggact
tcgaggactacatcagatacgacaactgcagcagcagcgtgctggccgccgtggtgtttgagcaccccttcaaccacagcaa
agagcccctgcctctggccgtgaagtaccacctgagattcagctacacccggcggaactacatgtggacccagaccggctca
ttcttcctgaaagagacagagggctggcacaccaccagcctgttccctctgttccccaaccctggccccagagagcctacatct
cctgacggcggcgagcccggctatatcagagaaggattcctggccgtgcagcacgccgtggacagagccatcatggaatac
cacgccgatgccgccacccggcagctgtttcagagactgaccgtgaccatcaagcggttcccttaccccccctttatcgccgac

cctttcctggtggccatccagtaccagctgccactcctcctgctgctgagctttacctacaccgccctgacaatcgccagagccgt
ggtgcaggaaaaagagcggcggctgaaagagtacatgcggatgatgggcctgtccagctggctgcattggagcgcctggttt
ctgctgttcttcctgttcctgctgatcgccgccagcttcatgacactgctgttttgcgtgaaagtgaagcccaacgtggcagtgctga
gccgcagcgatcctagcctggtgctggccttcctgctgtgcttcgccatcagcaccatcagcttcagctttatggtgtccaccttcttc
agcaaggccaacatggccgctgccttcggcggcttcctgtacttctttacctatattccctacttcttcgtggcccctcggtacaact
ggatgaccctgagccagaagctgtgcagctgcctgctgagcaacgtggccatggctatgggagcccagctgatcggcaagtt
cgaggccaagggcatgggcatccagtggcgggatctgctgagccccgtgaacgtggacgacgacttctgcttcggccaggtg
ctgggcatgctgctgctggactccgtgctgtatggcctcgtgacctggtatatggaagccgtgttccctggccagttcggcgtgcc
ccagccctggtacttcttcatcatgcctagctattggtgcggcaagcccagggccgtggccggcaaagaggaagaggatagc
gaccccgagaaggccctgcggaacgagtactttgaggccgagcccgaggatctggtggccggaatcaagatcaagcacct
gagcaaggtgttccgcgtgggcaacaaggatagagccgctgtgcgggacctgaacctgaatctgtacgagggccagatcac
cgtgctgctgggccataatggcgccggaaagaccaccaccctgagcatgctgaccggcctgtttcccccaacaagcggcag
ggcctacatcagcggctacgagatcagccaggacatggtgcagatccggaagtccctgggcctgtgcccccagcacgacat
cctgttcgacaacctgaccgtggccgagcacctgtacttttacgctcagctgaagggcctgagccggcagaaatgccccgagg
aagtgaagcagatgctgcacatcatcggcctggaagataagtggaacagccggtcccggttcctgtccggcggaatgagaa
gaaagctgagcatcggaatcgccctgattgccggcagcaaggtgctgatcctggacgagcctaccagcggcatggacgcca
tctccagaagggccatctgggacctgctgcagcggcagaagtccgacagaaccatcgtgctgaccacccacttcatggacga
ggccgacctgctgggcgaccggatcgctattatggccaagggggagctgcagtgctgcggcagcagcctgtttctgaagcag
aaatacggcgctggctaccacatgaccctcgtgaaagagcctcactgcaaccccgaggacatctcccagctggtgcaccac
cacgtgccaaatgccaccctggaaagctctgccggcgctgagctgagcttcatcctgcccagagagagcacccacagattcg
agggcctgttcgccaagctggaaaagaaacagaaagagctgggcattgccagcttcggcgccagcatcacaacaatggaa
gaggtgttcctgagagtgggcaagctggtggacagctccatggacatccaggctatccagctgcccgccctgcagtatcagca
cgagagaagggctagcgactgggccgtggactccaatctgtgcggcgccatggatccctccgatggaatcggcgccctgatc
gaagaggaacggaccgccgtgaagctgaacacaggactggccctgcactgccagcagttctgggccatgttcctgaagaaa
gccgcctacagctggcgcgagtggaaaatggtggccgcacaggtgctggtgcccctgacctgtgtgacactggcactgctgg
ccatcaactacagcagcgagctgttcgacgaccccatgctgagactgacactgggcgagtacggcaggaccgtggtgcctttt
tctgtgcccggcacctcacagctgggccagcagctgtctgaacacctgaaggatgccctgcaggccgaaggccaggaaccc
agagaagtgctgggcgatctggaagagttcctgatcttccgggccagcgtggaaggcggcggattcaacgagagatgcctgg
tggctgcctccttccgggatgtgggcgagagaacagtcgtgaacgccctgttcaacaatcaggcctaccacagccccgccac
cgctctggctgtggtggacaacctgctgtttaagctgctgtgtggcccccacgcctccatcgtggtgtccaatttcccccagccca
gaagcgctctgcaggctgccaaggaccagttcaacgagggccggaagggcttcgacattgctctgaatctgctgtttgccatgg
cctttctggcctccaccttcagcatcctggctgtgtccgagagagccgtgcaggccaagcacgtgcagtttgtgtctggcgtgcac
gtggccagcttttggctgtctgccctgctgtgggacctgatcagcttcctgatccccagcctcctgctgctggtggtgttcaaggcctt
cgacgtgcgggccttcaccagggatggacacatggccgacaccttgttgttgctgctgctgctgtacggctgggccatcatcccccctg
atgtacctgatgaacttcttcttcctgggcgctgccaccgcctacaccagactgaccatcttcaacatcctgagcggatcgcca
ccttcctgatggtcacaatcatgcggatccctgccgtgaaactggaagaactgagcaagaccctggaccatgtgtttctggtgct
gcccaaccactgcctgggcatggccgtgtctagcttctacgagaactacgagacacggcggtactgcacctccagcgaagtg
gccgcccactactgcaagaagtataacatccagtatcaggaaaacttctacgcttggagcgcacccggcgtgggcagatttgt
ggcctctatggccgccagcggctgcgcctatctgatcctgctgttcctgatcgagactaacctgctgcagagactgagaggcatc
ctgtgcgccctgcggcggagaagaacactgaccgagctgtacacccggatgcccgtgctgcctgaggaccaggatgtggcc
gacgagcggacaagaatcctggcccctagccccgatagcctgctgcacacccccctgatcatcaaagaactgtccaaggtgt
acgagcagcgggtgccactgctggctgtggacagactgagtctggctgtgcagaaaggcgagtgcttcggactgctgggcttc
aacggcgcaggcaagaccacaaccttcaagatgctgacaggcgaggaaagcctgacctccggcgacgcctttgtgggcgg
acacaggatctcttccgatgtgggcaaagtgcggcagcggatcggctactgccctcagttcgacgccctgctggatcacatga
ccggcagggaaatgctcgtgatgtacgcccggctgaggggcatccccgagagacacattggcgcctgcgtggaaaacacc
ctgcggggcctgctgctggaaccccacgctaacaaactcgtgcggacctacagcggcggcaacaagagaaagctgtctacc
ggcattgcactgatcggcgagccagccgtgatctttctggatgagcccagcacaggcatggaccccgtggctcggagactgct
gtgggatacagtggccagagccagagagtccggcaaggccatcattatcaccagccacagcatggaagagtgcgaggccc

tgtgtacaagactggcaattatggtgcagggacagttcaagtgtctgggcagccctcagcacctgaagtccaagttcggctccg
gctacagcctgcgggccaaggtgcagtctgaagggcagcaggaagccctggaagagttcaaagccttcgtggacctgacctt
ccccggctctgtgctggaagatgagcaccagggaatggtgcactaccatctgcctggcagggacctgtcctgggccaaagtgt
ttggcatcctggaaaaggccaaagagaagtacggcgtggacgattacagcgtgtcccagatcagcctggaacaggtgttcct
gtcctttgcccatctgcagccccctaccgccgaagagggaaga

## Claims

1. A composition comprising a modified polyribonucleotide for treating a subject having or suspected of having a disease associated with a gene of the ATP-binding cassette (ABC) family, wherein the modified polyribonucleotide encodes an ABC-binding cassette (ABC) family member and comprises an untranslated region derived from a cytochrome b-245 alpha polypeptide gene, wherein upon translation the modified polyribonucleotide yields an ATP-binding cassette (ABC) polypeptide that ameliorates a symptom of the disease, wherein the disease is selected from the group consisting of age-related macular degeneration, benign recurrent intrahepatic cholestasis, Cantú syndrome, congenital bilateral absence of the vas deferens, congenital hyperinsulinism, cystic fibrosis, Dubin-Johnson syndrome, familial dilated cardiomyopathy, familial HDL deficiency, generalized arterial calcification of infancy, harlequin ichthyosis, hereditary pancreatitis, intrahepatic cholestasis of pregnancy, lamellar ichthyosis, permanent neonatal diabetes mellitus, progressive familial intrahepatic cholestasis, pseudoxanthoma elasticum, retinitis pigmentosa, sitosterolemia, Stargardt macular degeneration, surfactant dysfunction, Tangier disease, X-linked adrenoleukodystrophy, X-linked sideroblastic anemia and ataxia, and
wherein the modified polyribonucleotide comprises a combination of unmodified and modified nucleotides or comprises analogues of uridine or analogues of cytidine.

2. The composition of claim 1, wherein the modified polyribonucleotide includes a codon sequence that is optimized for translation within cells of the subject exposed to the modified polyribonucleotide.

3. The composition of claim 1, wherein the gene in the ATP-binding cassette family is selected from the group consisting of ABCA1, ABCA3, ABCA4, ABCA12, ABCB4, ABCB7, ABCB11, ABCC2, ABCC6, ABCC8, ABCC9, ABCD1, ABCG5, ABCG8, and CFTR, preferably wherein the gene in the ATP-binding cassette family is ABCA3.

4. The composition of claim 1, wherein the gene in the ATP-binding cassette family is at least 70% homologous to the human ABCA3.

5. The composition of claim 1, wherein the composition comprises a ratio of moles of amine groups of cationic polymers to moles of phosphate groups of the modified polyribonucleotide of at least about 8.

6. The composition of claim 2, wherein the optimized codon sequence is translated at least 20% more effectively within a cell of a subject than a non-optimized codon sequence.

7. The composition of claim 1, wherein the modified polyribonucleotide provides expression of the polypeptide for a time period in the cell of the subject having the modified polyribonucleotide, wherein the time period is up to 4 weeks, wherein the expression is enhanced as compared to expression in a control cell that has been exposed to an unmodified polyribonucleotide encoding the polypeptide.

8. The composition of claim 7, wherein the time period is at least about 30 seconds, or wherein the time period is up to 5 days.

9. The composition of claim 7, wherein the modified polyribonucleotide:
comprises a 3' or 5' noncoding region flanking the codon sequence which encodes the polypeptide, wherein the noncoding region aids in enhanced expression of the polypeptide in the cells; or
is formulated in a nanoparticle or nanocapsule; or
is formulated in a cationic lipid, cationic polymer, or nanoemulsion.

10. The composition of claim 1, wherein the modified polyribonucleotide comprises 5% to 50% analogues of uridine or 5% to 50% analogues of cytidine or 15% to 30% analogues of uridine, or 15% to 30% analogues of cytidine.

11. The composition of claim 1, wherein the analogues of uridine are selected from the group consisting of pseurouridine, 2-thiouridine, 5-iodouridine, and 5-methyluridine; and/or wherein the analogues of cytidine are selected from the group consisting of 5-methylcytidine, 2'-amino-2'-deoxycytidine, 2'-fluoro-2'-deoxycytidine, and 5-iodocytidine.

12. The composition of claim 1, wherein the modified polyribonucleotide comprises 5-methylcytidine or pseudouridine, or (i) uridine and cytidine; and (ii) analogues of the uridine and cytidine.

13. The composition of claim 1, wherein the modified polyribonucleotide comprises analogues of adenosine or analogues of guanosine.

14. The composition of claim 13, wherein the modified polyribonucleotide comprises (i) adenosine or guanosine; and (ii) analogues of the adenosine or guanosine.

15. The composition of claim 1, wherein the modified polyribonucleotide comprises less than 50% analogues of adenosine or guanosine; or has a transfection efficiency greater than 80% among cells exposed to the modified polyribonucleotide; or induces substantially no change in a level of at least one inflammatory marker expressed by peripheral blood mononuclear cells exposed to the modified polyribonucleotide.

16. The composition of claim 1, wherein (i) the codon sequence is a gene or fragment whose defect or deficiency is associated with a presence of the disease, or (ii) a lack or deficiency of the polypeptide is associated with the presence of the disease.

17. The composition of claim 1, wherein the modified polyribonucleotide lowers an immune response of the subject as compared to an unmodified polyribonucleotide encoding the polypeptide, which immune response is as determined by a level of at least one inflammatory marker selected from TNF-α, IL-2 and IL-8 expressed by peripheral blood mononuclear cells exposed to the modified polyribonucleotide as compared to a level of the at least one inflammatory marker in peripheral blood mononuclear cells in a control that has been exposed to the unmodified polyribonucleotide.

## Patentansprüche

1. Zusammensetzung, umfassend ein modifiziertes Polyribonucleotid zur Behandlung eines Individuums, das eine Erkrankung hat oder von dem angenommen wird, dass es eine Erkrankung hat, die mit einem Gen der Familie der ATP-bindenden Kassette (ATP-binding cassette; ABC) in Zusammenhang steht, wobei das modifizierte Polyribonucleotid ein Mitglied der Familie der ABCbindenden Kassette (ABC-binding cassette; ABC) codiert und eine untranslatierte Region, die von einem Gen des Cytochrom-b-245-Alpha-Polypeptids abgeleitet ist, umfasst, wobei das modifizierte Polyribonucleotid bei der Translation ein Polypeptid der ATP-bindenden Kassette (ABC) hervorbringt, das ein Symptom der Erkrankung lindert,
wobei die Erkrankung ausgewählt ist aus der Gruppe bestehend aus altersbedingter Makuladegeneration, benigner rekurrierender intrahepatischer Cholestase, Cantú-Syndrom, kongenitaler bilateraler Aplasie des Vas deferens, kongenitalem Hyperinsulinismus, cystischer Fibrose, Dubin-Johnson-Syndrom, familiärer dilatativer Kardiomyopathie, familiärem HDL-Mangel, generalisierter arterieller Verkalkung im frühen Kindesalter, Harlekin-Ichthyose, hereditärer Pankreatitis, intrahepatischer Schwangerschaftscholestase, lamellärer Ichthyose, permanenter neonataler Diabetes mellitus, progressiver familiärer intrahepatischer Cholestase, Pseudoxanthoma elasticum, Retinitis pigmentosa, Sitosterolämie, Morbus Stargardt, Surfactant-Dysfunktion, Tangier-Krankheit, X-chromosomal gekoppelter Adrenoleukodystrophie, X-chromosomal gekoppelter sideroblastischer Anämie und Ataxie, und
wobei das modifizierte Polyribonucleotid eine Kombination unmodifizierter und modifizierter Nucleotide umfasst oder Analoga von Uridin oder Analoga von Cytidin umfasst.

2. Zusammensetzung nach Anspruch 1, wobei das modifizierte Polyribonucleotid eine Codonsequenz umfasst, die für die Translation in den Zellen des Individuums, das dem modifizierten Polyribonucleotid ausgesetzt ist, optimiert ist.

3. Zusammensetzung nach Anspruch 1, wobei das Gen in der Familie der ATP-bindenden Kassette ausgewählt ist aus der Gruppe bestehend aus ABCA1, ABCA3, ABCA4, ABCA12, ABCB4, ABCB7, ABCB11, ABCC2, ABCC6, ABCC8, ABCC9, ABCD1, ABCG5, ABCG8 und CFTR, bevorzugt wobei das Gen in der Familie der ATP-bindenden Kassette ABCA3 ist.

4. Zusammensetzung nach Anspruch 1, wobei das Gen in der Familie der ATP-bindenden Kassette mindestens 70% homolog zum humanen ABCA3 ist.

5. Zusammensetzung nach Anspruch 1, wobei die Zusammensetzung ein Verhältnis der Mole von Aminogruppen von kationischen Polymeren zu Molen von Phosphatgruppen der modifizierten Polyribonucleotide von mindestens etwa 8 umfasst.

6. Zusammensetzung nach Anspruch 2, wobei die optimierte Codonsequenz innerhalb einer Zelle eines Individuums mindestens 20% wirksamer translatiert wird als eine nicht-optimierte Codonsequenz.

7. Zusammensetzung nach Anspruch 1, wobei das modifizierte Polyribonucleotid die Expression des Polypeptids für einen Zeitraum in der Zelle des Individuums, die das modifizierte Polyribonucleotid aufweist, bereitstellt, wobei der Zeitraum bis zu 4 Wochen ist, wobei die Expression im Vergleich zur Expression in einer Kontrollzelle erhöht ist, die einem unmodifizierten Polyribonucleotid ausgesetzt wurde, das das Polypeptid codiert.

8. Zusammensetzung nach Anspruch 7, wobei der Zeitraum mindestens etwa 30 Sekunden beträgt oder wobei der Zeitraum bis zu 5 Tagen beträgt.

9. Zusammensetzung nach Anspruch 7, wobei das modifizierte Polyribonucleotid:
eine 3'- oder 5'-nicht-codierende Region umfasst, die die Codonsequenz flankiert, die das Polypeptid codiert, wobei die nicht-codierende Region zur erhöhten Expression des Polypeptids in den Zellen beiträgt; oder
in einem Nanopartikel oder in einer Nanokapsel formuliert ist; oder
in einem kationischen Lipid, einem kationischen Polymer oder einer Nanoemulsion formuliert ist.

10. Zusammensetzung nach Anspruch 1, wobei das modifizierte Polyribonucleotid 5% bis 50% Analoga von Uridin oder 5% bis 50% Analoga von Cytidin oder 15% bis 30% Analoga von Uridin oder 15% bis 30% Analoga von Cytidin umfasst.

11. Zusammensetzung nach Anspruch 1, wobei die Analoga von Uridin ausgewählt sind aus der Gruppe bestehend aus Pseudouridin, 2-Thiouridin, 5-Ioduridin und 5-Methyluridin; und/oder wobei die Analoga von Cytidin ausgewählt sind aus der Gruppe bestehend aus 5-Methylcytidin, 2'-Amino-2'desoxycytidin, 2'-Fluor-2'-desoxycytidin und 5-Iodcytidin.

12. Zusammensetzung nach Anspruch 1, wobei das modifizierte Polyribonucleotid 5-Methylcytidin oder Pseudouridin oder (i) Uridin und Cytidin; und (ii) Analoga von Uridin und Cytidin umfasst.

13. Zusammensetzung nach Anspruch 1, wobei das modifizierte Polyribonucleotid Analoga von Adenosin oder Analoga von Guanosin umfasst.

14. Zusammensetzung nach Anspruch 13, wobei das modifizierte Polyribonucleotid (i) Adenosin oder Guanosin; und (ii) Analoga von Adenosin oder Guanosin umfasst.

15. Zusammensetzung nach Anspruch 1, wobei das modifizierte Polyribonucleotid weniger als 50% Analoga von Adenosin oder Guanosin umfasst; oder eine Transfektionseffizienz von mehr als 80% in den Zellen aufweist, die dem modifizierten Polyribonucleotid ausgesetzt sind; oder im Wesentlichen keine Veränderung des Spiegels mindestens eines Entzündungsmarkers induziert, der von peripheren mononucleären Blutzellen exprimiert wird, die dem modifizierten Polyribonucleotid ausgesetzt sind.

16. Zusammensetzung nach Anspruch 1, wobei (i) die Codonsequenz ein Gen oder ein Fragment ist, dessen Defekt oder Mangel mit einem Vorliegen der Erkrankung in Zusammenhang steht, oder (ii) ein Fehlen oder Mangel des Polypeptids mit einem Vorliegen der Erkrankung in Zusammenhang steht.

17. Zusammensetzung nach Anspruch 1, wobei das modifizierte Polyribonucleotid eine Immunantwort des Individuums im Vergleich zu einem unmodifizierten Polyribonucleotid, das das Polypeptid codiert, verringert, wobei die Immunantwort von einem Spiegel mindestens eines Entzündungsmarkers bestimmt ist, der ausgewählt ist aus TNF-α, IL-2 und IL-8, die durch periphere mononucleäre Blutzellen, die dem modifizierten Polyribonucleotid ausgesetzt sind, exprimiert sind, im Vergleich zu einem Spiegel des mindestens einen Entzündungsmarkers in peripheren mononucleären Blutzellen in einer Kontrolle, die dem unmodifizierten Polyribonucleotid ausgesetzt wurde.

## Revendications

1. Composition comprenant un polyribonucléotide modifié pour traiter un sujet ayant ou suspecté d'avoir une maladie associée à un gène de la famille des cassettes se liant à l'ATP (ABC), dans laquelle le polyribonucléotide modifié code un membre de la famille des cassettes se liant à l'ABC (ABC) et comprend une région non traduite dérivée d'un gène de polypeptide de cytochrome b-245 alpha, dans laquelle, suite à la traduction, le polyribonucléotide modifié engendre un polypeptide de cassette se liant à l'ATP (ABC) qui améliore un symptôme de la maladie,
dans laquelle la maladie est choisie dans le groupe constitué par une dégénérescence maculaire liée à l'âge, une cholestase intra-hépatique récurrente bénigne, un syndrome de Cantu, une absence bilatérale congénitale des canaux déférents, un hyperinsulinisme congénital, une mucoviscidose, un syndrome de Dubin-Johnson, une cardiomyopathie dilatée familiale, une insuffisance en HDL familiale, une calcification artérielle généralisée de la petite enfance, une ichtyose arlequin, une pancréatite héréditaire, une cholestase intra-hépatique gestationnelle, une ichtyose lamellaire, un diabète sucré néonatal permanent, une cholestase intra-hépatique familiale progressive, un pseudoxanthome élastique, une rétinite pigmentaire, une sitostérolémie, une dégénérescence maculaire de Stargardt, un dysfonctionnement de tensioactif, une maladie de Tangier, une leucodystrophie avec insuffisance surrénale liée au chromosome X, une anémie sidéroblastique liée au chromosome X, et une ataxie, et
dans laquelle le polyribonucléotide modifié comprend une combinaison de nucléotides non modifiés et modifiés ou comprend des analogues d'uridine ou des analogues de cytidine.

2. Composition selon la revendication 1, dans laquelle le polyribonucléotide modifié contient une séquence de codon qui est optimisée pour une traduction à l'intérieur de cellules du sujet exposé au polyribonucléotide modifié.

3. Composition selon la revendication 1, dans laquelle le gène dans la famille des cassettes se liant à l'ATP est choisi dans le groupe constitué par ABCA1, ABCA3, ABCA4, ABCA12, ABCB4, ABCB7, ABCB11, ABCC2, ABCC6, ABCC8, ABCC9, ABCD1, ABCG5, ABCG8, et CFTR, de préférence dans laquelle le gène dans la famille des cassettes se liant à l'ATP est ABCA3.

4. Composition selon la revendication 1, dans laquelle le gène dans la famille des cassettes se liant à l'ATP est homologue à au moins 70 % à l'ABCA3 humain.

5. Composition selon la revendication 1, dans laquelle la composition comprend un rapport des moles de groupes amine de polymères cationiques aux moles de groupes phosphate du polyribonucléotide modifié d'au moins environ 8.

6. Composition selon la revendication 2, dans laquelle la séquence de codon optimisée est traduite au moins 20 % plus efficacement dans une cellule d'un sujet qu'une séquence de codon non optimisée.

7. Composition selon la revendication 1, dans laquelle le polyribonucléotide modifié réalise l'expression du polypeptide pendant une certaine période de temps dans la cellule du sujet ayant le polyribonucléotide modifié, dans laquelle la période de temps va jusqu'à 4 semaines, dans laquelle l'expression est amplifiée en comparaison avec l'expression dans une cellule témoin qui a été exposée à un polyribonucléotide non modifié codant le polypeptide.

8. Composition selon la revendication 7, dans laquelle la période de temps est d'au moins environ 30 secondes, ou dans laquelle la période de temps va jusqu'à 5 jours.

9. Composition selon la revendication 7, dans laquelle le polyribonucléotide modifié :
comprend une région non codante en 3' ou 5' flanquant la séquence de codon qui code le polypeptide, dans laquelle la région non codante facilite une expression amplifiée du polypeptide dans les cellules ; ou
est formulé dans une nanoparticule ou une nanocapsule ; ou
est formulé dans un lipide cationique, un polymère cationique, ou une nanoémulsion.

10. Composition selon la revendication 1, dans laquelle le polyribonucléotide modifié comprend 5 % à 50 % d'analogues d'uridine ou 5 % à 50 % d'analogues de cytidine ou 15 % à 30 % d'analogues d'uridine, ou 15 % à 30 % d'analogues de cytidine.

11. Composition selon la revendication 1, dans laquelle les analogues d'uridine sont choisis dans le groupe constitué par la pseudouridine, la 2-thiouridine, la 5-iodouridine, et la 5-méthyluridine ; et/ou dans laquelle les analogues de cytidine sont choisis dans le groupe constitué par la 5-méthylcytidine, la 2'-amino-2'-désoxycytidine, la 2'-fluoro-2'-désoxycytidine, et la 5-iodocytidine.

12. Composition selon la revendication 1, dans laquelle le polyribonucléotide modifié comprend de la 5-méthylcytidine ou de la pseudouridine, ou (i) de l'uridine et de la cytidine ; et (ii) des analogues de l'uridine et de la cytidine.

13. Composition selon la revendication 1, dans laquelle le polyribonucléotide modifié comprend des analogues d'adénosine ou des analogues de guanosine.

14. Composition selon la revendication 13, dans laquelle le polyribonucléotide modifié comprend (i) de l'adénosine ou de la guanosine ; et (ii) des analogues de l'adénosine ou de la guanosine.

15. Composition selon la revendication 1, dans laquelle le polyribonucléotide modifié comprend moins de 50 % d'analogues d'adénosine ou de guanosine ; ou a une efficacité de transfection supérieure à 80 % parmi les cellules exposées au polyribonucléotide modifié ; ou n'induit pratiquement pas de changement au niveau d'au moins un marqueur inflammatoire exprimé par des cellules mononucléaires de sang périphérique exposées au polyribonucléotide modifié.

16. Composition selon la revendication 1, dans laquelle (i) la séquence de codon est un gène ou fragment dont le défaut ou l'insuffisance est associé à la présence de la maladie, ou (ii) une absence ou une insuffisance du polypeptide est associée à la présence de la maladie.

17. Composition selon la revendication 1, dans laquelle le polyribonucléotide modifié abaisse la réponse immunitaire du sujet en comparaison avec un polyribonucléotide non modifié codant le polypeptide, laquelle réponse immunitaire est telle que déterminée par le niveau d'au moins un marqueur inflammatoire choisi parmi TNF-α, IL-2 et IL-8 exprimé par des cellules mononucléaires de sang périphérique exposées au polyribonucléotide modifié, en comparaison avec le niveau du au moins un marqueur inflammatoire dans des cellules mononucléaires de sang périphérique dans un témoin qui a été exposé au polyribonucléotide non modifié.
